# EUROPEAN PATENT APPLICATION

(11) **EP 1 175 907 A1**
(43) Date of publication of application: **30.01.2002**
(21) Application number: 00917309.7
(22) Date of filing: 14.04.2000
(51) Int. Cl.: A61K 31/737, A61K 31/70, A61K 31/7016, A61K 31/702, A61K 7/40, A61P 43/00, A23L 2/52, A23L 1/29, A23K 1/16

(54) **REMEDIES**

(30) Priority: 15.04.1999 JP 10806799; 15.04.1999 JP 10849999; 22.04.1999 JP 11454299; 10.05.1999 JP 12916399; 21.05.1999 JP 14234399; 02.06.1999 JP 15466299; 14.07.1999 JP 20098299; 28.09.1999 JP 27523199; 28.12.1999 JP 37560699; 31.03.2000 JP 2000099941
(71) Applicant: Takara Shuzo Co, Ltd., Kyoto-shi, Kyoto 612-8061 (JP)
(72) Inventor: SAGAWA, Hiroaki, Kusatsu-shi, Shiga 525-0025 (JP); SAKAI, Takeshi, Hirosaki-shi, Aomori 036-8183 (JP); KOBAYASHI, Eiji, Otsu-shi, Shiga 520-2153 (JP); LI, Tuo-Ping, Otsu-shi, Shiga 520-2133 (JP); OHNOGI, Hiromu, Muko-shi, Kyoto 617-0002 (JP); NISHIMURA, Kaori, Otsu-shi, Shiga 520-0853 (JP); NISHIYAMA, Eiji, Moriyama-shi, Shiga 524-0102 (JP); WU, Hua-Kang, Otsu-shi, Shiga 520-2133 (JP); MIZUTANI, Shigetoshi, Shiga 521-1322 (JP); KATO, Ikunoshin, Uji-shi, Kyoto 611-0028 (JP)
(74) Representative: VOSSIUS & PARTNER
(86) International application number: JP0002432
(87) International publication number: WO0062785

(57) **Abstract**

The present invention provides a therapeutic agent or prophylactic agent for a disease requiring induction of growth factor production, a food, beverage or feed for induction of growth factor production, a cosmetic for induction of growth factor production, and a controlling agent for growth factor production, characterized in that each comprises as an effective ingredient a compound selected from the group consisting of acidic polysaccharides, degradation products thereof, acidic oligosaccharides, acidic monosaccharides, acidic sugar alcohols and salts thereof, wherein the compound has induction action for growth factor production.

SEQUENCE LISTING

<110> Takara Shuzo Co., Ltd.

<120> Therapeutic Agents

<130> 00-028-PCT

<150> JP 11-108067
<151> 1999-4-15

<150> JP 11-108499
<151> 1999-4-15

<150> JP 11-114542
<151> 1999-4-22

<150> JP 11-129163
<151> 1999-5-10

<150> JP 11-142343
<151> 1999-5-21

<150> JP 11-154662
<151> 1999-6-2

<150> JP 11-200982
<151> 1999-7-14

<150> JP 11-275231
<151> 1999-9-28

<150> JP 11-375606
<151> 1999-12-28

<150> JP 2000-99941
<151> 2000-3-31

<160> 4

<210> 1
<211> 23
<212> DNA
<213> Artificial Sequence
<220>
<223> Primer
<400> 1
gtgaatactg cagaccaatg tgc 23

<210> 2
<211> 25
<212> DNA

<213> Artificial Sequence

<220>

<223> Primer

<400> 2

cacagacttc gtagcgtacc tctgg 25

<210> 3

<211> 21

<212> DNA

<213> Artificial Sequence

<220>

<223> Primer

<400> 3

caagagatgg ccacggctgc t 21

<210> 4

<211> 22

<212> DNA

<213> Artificial Sequence

<220>

<223> Primer

<400> 4

tccttctgca tcctgtcggc aa 22

## Description

### TECHNICAL FIELD

The present invention relates to applications of acidic saccharide compounds having physiological activity as pharmaceuticals, foods, beverages, feeds or cosmetics.

### BACKGROUND ART

As acidic polysaccharides derived from marine algae, sulfated polysaccharides such as rhamnan sulfate derived from Chlorophyceae, sulfated galactan derived from Rhodophyceae, and sulfated fucose-containing polysaccharides derived from Phaeophyceae have been known. For instance, the fucoidan is a sulfated fucose-containing polysaccharide contained in Phaeophyceae, Echinodermata, or the like, wherein the sulfated fucose is. contained as a constituting saccharide. Also, cartilage of *Chondrichthyes Selachii* or the like also contains a sulfated polysaccharide.

As physiological action of a sulfated polysaccharide, for instance, fucoidan, cancer proliferation-suppressing activity, cancer metastasis-suppressing activity, anticoagulation activity, antiviral activity and the like. have been known, and the development of applications for pharmaceuticals have been expected of the sulfated polysaccharides.

As a substance having induction action for hepatocyte growth factor production, heparin, heparan sulfate, and low-molecular weight heparins having an average molecular weight of 4400 to 5600 have been known (Japanese Patent Laid-Open No. Hei 6-312941). However, there have not been any reports on the induction action for growth factor production of other sulfated polysaccharides, for instance, fucoidan, synthetic sulfated polysaccharides, and the like.

### DISCLOSURE OF INVENTION

The present invention has been established based on the finding of new physiological action of various acidic saccharide compounds, for instance, acidic polysaccharides, such as fucoidan, and its object is to provide a pharmaceutical, food, beverage, feed or cosmetic, utilizing the induction action for growth factor production of various acidic saccharide compounds, for instance, acidic polysaccharides, such as fucoidan, especially utilizing induction action for hepatocyte growth factor production, induction action for insulin-like growth factor production or induction action for nerve growth factor production.

Summarizing the present invention, a first invention of the present invention relates to a therapeutic agent or prophylactic agent for a disease requiring induction of growth factor production, characterized in that the therapeutic agent or prophylactic agent comprises as an effective ingredient a compound selected from the group consisting of acidic polysaccharides, degradation products thereof, acidic oligosaccharides, acidic monosaccharides, acidic sugar alcohols and salts thereof, excluding heparin and heparan sulfate, wherein the compound has induction action for growth factor production.

A second invention of the present invention relates to a food, beverage (hereinafter referred to as "foodstuff" in some cases) or feed for induction of growth factor production, comprising a compound selected from the group consisting of acidic polysaccharides, degradation products thereof, acidic oligosaccharides, acidic monosaccharides, acidic sugar alcohols and salts thereof, wherein the compound has induction action for growth factor production.

A third invention of the present invention relates to a cosmetic for induction of growth factor production comprising a compound selected from the group consisting of acidic polysaccharides, degradation products thereof, acidic oligosaccharides, acidic monosaccharides, acidic sugar alcohols and salts thereof, wherein the compound has induction action for growth factor production.

A fourth invention of the present invention relates to a controlling agent for growth factor production comprising a compound selected from the group consisting of acidic polysaccharides, degradation products thereof, acidic oligosaccharides, acidic monosaccharides, acidic sugar alcohols and salts thereof.

In the present invention, the acidic polysaccharides having induction action for growth factor production are preferably exemplified by sulfated polysaccharides. As the sulfated polysaccharides, there can be suitably used a sulfated polysaccharide derived from an algae; a sulfated polysaccharide derived from Animalia, for instance, a sulfated polysaccharide derived from Echinodermata such as a sulfated polysaccharide derived from sea cucumbers, a sulfated polysaccharide derived from Pisces, for instance, a sulfated polysaccharide derived from cartilage of *Chondrichthyes Selachii*; a sulfated polysaccharide derived from a microorganism; a sulfated polysaccharide derived from Plantae, for instance, a sulfated polysaccharide derived from *Altemisia princeps pampan*; and a synthetic sulfated polysaccharide.

In addition, as the sulfated polysaccharide derived from an algae having induction action for growth factor production, rhamnan sulfate, sulfated galactan, or a sulfated fucose-containingpolysaccharide can be suitably used. The synthetic sulfated polysaccharide is exemplified by sodium dextran sulfate, sulfated starch, curdlan sulfate, sulfated pectin, and the like, and a highly-sulfated sulfated polysaccharide obtained by further sulfating the sulfated polysaccharide can be preferably used. Also, as the sulfated fucose-containing polysaccharide, fucoidan can be preferably used. The acidic oligosaccharide is preferably a sulfated oligosaccharide. For instance, there can be used sulfated maltoses, sulfated lactoses, sulfated sucroses, sulfated trehaloses, sulfated lactuloses, sulfated melibioses, sulfated cellobioses, sulfated isomaltoses, sulfated turanoses, sulfated palatinoses, sulfated maltotrioses, sulfated maltohexaoses, sulfated maltoheptaoses, sulfated dodecyl-maltohexaoses, a compound represented by the formula (I): 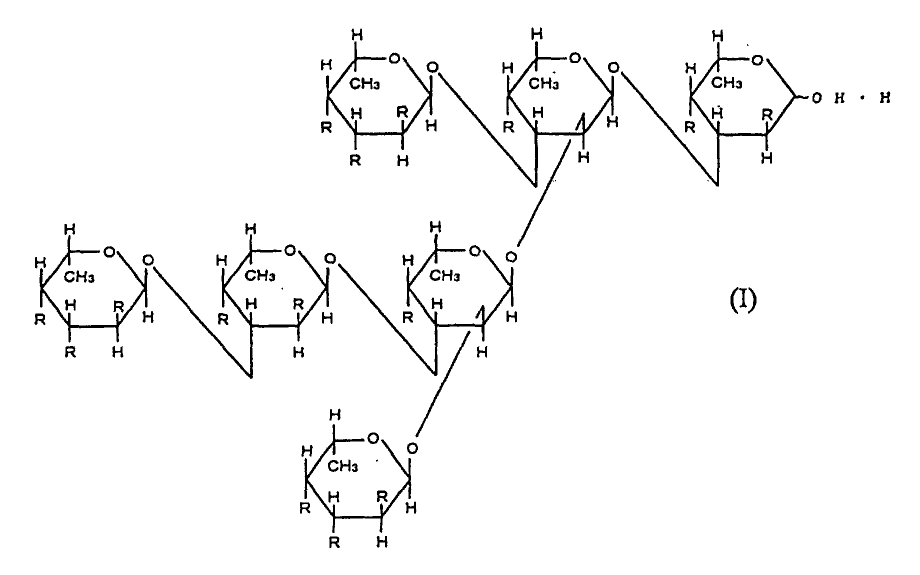 wherein R is OH or OSO₃H, and
a compound represented by the formula (II): 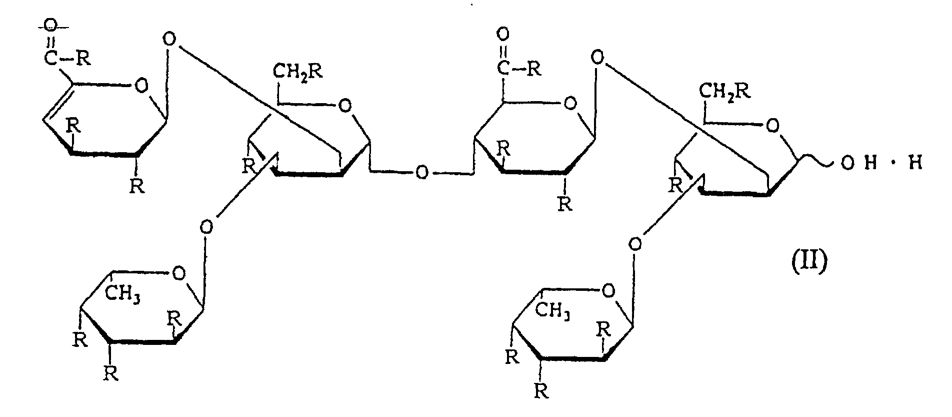 wherein R is OH or OSO₃H.

In addition, the acidic monosaccharide is preferably a sulfated monosaccharide. For instance, a sulfated glucose, a sulfated galactose, a sulfated xylose, a sulfated 2-deoxy-glucose, a sulfated talose and a sulfated mannose can be used. Also, as the acidic sugar alcohol, there can be used sulfated sugar alcohols, for instance, sulfated glucitol and the like. These sulfated oligosaccharides, sulfated monosaccharides and sulfated sugar alcohols may be prepared by a general synthesis method. The position and the number of sulfate group in these saccharide compounds are not particularly limited, as long as these sulfated oligosaccharides, sulfated monosaccharides and sulfated sugar alcohols exhibit induction action for growth factor production.

In the present invention, the degradation product of the acidic polysaccharide having induction action for growth factor production can be also used. The degradation product encompasses heparin degradation products and heparan sulfate degradation products, each having a molecular weight of 4000 or less.

The substances exemplified in the acidic polysaccharide, degradation product thereof, acidic oligosaccharide, acidic monosaccharide, or acidic sugar alcohol mentioned above can be used each alone or in admixture of two or more kinds. In addition, the salts thereof can be also preferably used.

In the present invention, the growth factor is exemplified by a hepatocyte growth factor, an insulin-like growth factor and a nerve growth factor.

In the therapeutic agent or prophylactic agent of the first invention, the food, beverage or feed of the second invention, and the cosmetic of the third invention of the present invention, there can be further contained a substance capable of synergistically increasing induction action for growth factor production of an acidic polysaccharide, degradation product thereof, acidic oligosaccharide, acidic monosaccharide, acidic sugar alcohol or salt thereof, and the substance is exemplified by a substance selected from cytokines, prostaglandins, and compounds having a cyclopentene ring, such as minoxidil and calpronium chloride.

In addition, the food, beverage or feed of the second invention of the present invention is preferably a food, beverage or feed for induction of hepatocyte growth factor production, induction of insulin-like growth factor production or induction of nerve growth factor production.

In addition, the cosmetic of the third invention of the present invention is preferably a cosmetic for induction of hepatocyte growth factor production, induction of insulin-like growth factor production or induction of nerve growth factor production.

The cosmetic of the third invention of the present invention is exemplified by a lotion, a milky lotion, cream, a facial pack, a bathing agent, a facial cleansing agent, a bathing soap, or a bathing detergent.

In the present invention, the phrase "compound selected from acidic polysaccharides, degradation products thereof, acidic oligosaccharides, acidic monosaccharides, acidic sugar alcohols and salts thereof" may be simply referred to as "effective ingredient" in the present specification.

### BRIEF DESCRIPTION OF THE DRAWINGS

Figure 1 is a diagram showing an elution pattern of the fucoidan derived from *Kjellmaniella crassifolia* on DEAE-Cellulofine A-800.
Figure 2 is a graph showing the working curve of the sulfate content using a sodium sulfate solution as a standard sample.

### BEST MODE FOR CARRYING OUT THE INVENTION

The term "acidic polysaccharide having induction action for growth factor production" in the present invention refers to any acidic polysaccharides without particular limitations, as long as the acidic polysaccharide has induction action for growth factor production. There can be preferably used acidic polysaccharides such as alginic acid, pectin, pectic acid and hyaluronic acid; sulfated polysaccharides such as chondroitin sulfate, keratan sulfate and dermatan sulfate; sulfated polysaccharides derived from Animalia, for instance, sulfated polysaccharides derived from Echinodermata, sulfated polysaccharides derived from Pisces, for instance, sulfated polysaccharides derived from cartilage of *Chondrichthyes Selachii*; sulfated polysaccharides derived from plantae, for instance, sulfated polysaccharides derived from *Altemisia princeps pampan,* sulfated polysaccharides derived from *Momordica charantia*, sulfated polysaccharides derived from Aloe, sulfated polysaccharides derived from Chrysanthemum coronarium leaf; sulfated polysaccharides derived from microorganisms, such as sulfated polysaccharides derived from *Chlorella,* sulfated polysaccharides derived from *Spirulina*, and sulfated polysaccharides derived from algae.

As the sulfated polysaccharides derived from algae, there can be used rhamnan sulfate derived from Chlorophyceae; sulfated galactan derived from Rhodophyceae, for instance, *Gelidium amansii*, *Gracilaria*, Giant kelp, *Pteroclavia capillacae*, carrageenan, agars, agarose, agaropectin, and porphyran; sulfated fucose-containing polysaccharides derived from Phaeophyceae, for instance, fucoidan, sulfated fucogalactan, sulfated fucoglucuronomannan, glucuronoxylofucane, Sargassan, glucuronomannan galactan, xylofucoglucuronan, ascorfilan, glucuronogalactofucane, sulfated glucuronofucane, and the like. Especially, fucoidan, sulfated fucogalactan, λ-carrageenan, chondroitin sulfate B, chondroitin sulfate D, alginic acid, agaropectin, and the like can be preferably used in the present invention. Also, acidic polysaccharides derived from Cyanophyceae, for instance, sulfated polysaccharides derived from *Spirulina*; and the acidic polysaccharides derived from Chlorophyceae, for instance, sulfated polysaccharides derived from *Chlorella* can be used. Especially, the sulfated polysaccharides derived from *Spirulina* are useful in amelioration of hepatic function by its induction action for hepatocyte growth factor production, being remarkably effective in amelioration of, for instance, symptoms of viral hepatitis type C. Also, phosphated polysaccharide, for instance, nucleic acid is also encompassed by the acidic polysaccharide of the present invention.

The sulfated fucose-containing polysaccharides used in the present invention are preferably exemplified by fucoidans derived from algae mentioned. above, and are not particularly limited, as long as the sulfated fucose-containing polysaccharides are a polysaccharide having sulfated fucose as a constituent and have induction action for growth factor production. There may be used fucoidans derived from Echinodermata, for instance, sea cucumber, Echnoidea, Asterozoa, and the like.

These sulfated fucose-containing polysaccharides can be used alone or in admixture of two or more kinds. In addition, the degradation products of these acidic polysaccharides exemplified and salts thereof can be used without particular limitation, as long as they exhibit induction action for growth factor production.

These acidic polysaccharides may be each prepared by a known method, and the resulting purified products, acidic polysaccharide-containing products or the like can be also used in the present invention. As the acidic polysaccharide-containing products, sulfated polysaccharide fractions can be preferably used, and as the fraction, the sulfated polysaccharide fractions derived from algae, and sulfated polysaccharide fractions derived from cartilage of *Chondrichthyes Selachii* can be preferably used. Also, as a raw material for the sulfated polysaccharide-containing product, algae, sea cucumber, cartilage of *Chondrichthyes Selachii* or the like can be used. For instance, marine algae of Laminariales, Chordariales, Fucales, and the like, such as *Kjellmaniella crassifolia, Laminaria japonica, Kjellmaniella, Fucus, Nemacystus, Cladosiphon* *okamuranus, Undaria, Ecklonia kurome, Eisenia, Ecklonia, Lessonia nigrescence* and *Ascophyllum nodosum* richly contain fucoidans especially suitable for the use in the present invention. This is why they are preferable as the raw material.

The synthetic sulfated polysaccharides used in the present invention are not particularly limited, as long as the synthetic sulfated polysaccharides have induction action for growth factor production, and the sulfated polysaccharides which have been so far used as pharmaceuticals are preferably used. The synthetic sulfated polysaccharides are exemplified by sodium dextran sulfate. This compound is a sodium salt of sulfated ester, obtained by sulfating a partially degraded product of dextran produced by fermentation of sucrose by Leuconostoc mesenteroides van Tieghem.

Also in the present invention, synthetic sulfated polysaccharides such as sulfated starch, curdlan sulfate, sulfated pectin, and the like can be used, and a highly-sulfated sulfated polysaccharide obtained by further sulfating the sulfated polysaccharide can be preferably used.

The position of sulfate group of the sulfated polysaccharide used in the present invention is not particularly limited, as long as the sulfated polysaccharide exhibits induction action for growth factor production. The sulfated polysaccharide of which constituting saccharide is sulfated at the 2-position, fucoidan, λ-carrageenan, chondroitin sulfate D, or degradation product thereof can be preferably used in the present invention. Also, the sulfate content (or number of sulfate group) of the sulfated polysaccharide is not particularly limited, as long as the sulfated polysaccharide exhibits induction action for growth factor production. Here, the degradation products of the acidic polysaccharides encompass oligosaccharides and monosaccharides, and there can be used in the present invention an oligosaccharide or monosaccharide, such as fucose-2-sulfate and glucose-2-sulfate, having sulfate group at the 2-position. These sulfated monosaccharides, sulfated oligosaccharides, and sulfated polysaccharides may be prepared by a general synthesis method, and the resulting prepared products and purified products can be also used in the present invention. In the present invention, the term "oligosaccharide" is defined as a saccharide compound in which 2 to 10 of monosaccharides are connected, and the term "polysaccharide" is defined as a saccharide compound in which 11 or more monosaccharides are connected.

For instance, a fucoidan is prepared from *Kjellmaniella crassifolia*, and the resulting fucoidan can be separated into glucuronic acid-containing fucoidan (referred to as "U-fucoidan") and glucuronic acid non-containing fucoidan (referred to as "F-fucoidan"), and each of the fucoidans can be used as an effective ingredient of the present invention. Also, sulfated fucogalactan can be prepared from *Kjellmaniella crassifolia* and used.

Further, agaropectin can be prepared from an agar and used.

After the preparation of the fucoidans from *Kjellmaniella crassifolia*, U-fucoidan and F-fucoidan are separated by using an anionic exchange resin, a surfactant or the like. The existing ratio of U-fucoidan to F-fucoidan derived from *Kjellmaniella crassifolia* is about 1:2. U-fucoidan contains fucose, mannose, galactose, glucuronic acid and the like, and its sulfate content is about 20%. F-fucoidan contains fucose and galactose, and its sulfate content is about 50%. The molecular weight for both substances is distributed, centering about 200000 (*Summary of 18th Sugar Symposium,* p. 159, 1996).

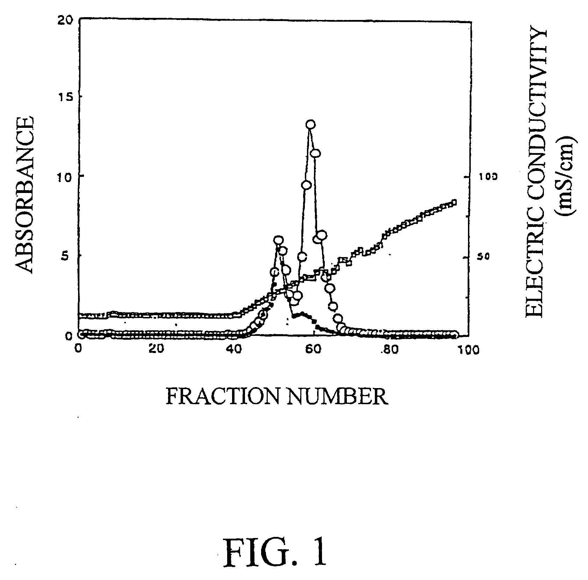

U-fucoidan and F-fucoidan can be separated, for instance, by applying a fucoidan solution prepared from *Kjellmaniella crassifolia* onto DEAE-Cellulofine A-800 column, and carrying out elution by the concentration gradient technique using NaCl-containing buffer. One of the examples is shown in Figure 1. Concretely, Figure 1 is a diagram showing the separation of U-fucoidan and F-fucoidan, wherein the former peak in the figure is U-fucoidan, and the latter peak is F-fucoidan.

In addition, for instance, each of the sulfated polysaccharide derived from *Gelidium amansii*, the sulfated polysaccharide derived from *Gracilaria*, the sulfated polysaccharide derived from *Pteroclavia,* the sulfated polysaccharide derived from other algae, the fucoidan derived from *Fucus,* the fucoidan derived from *Nemacystus*, the fucoidan derived from *Cladosiphon okamuranus*, the fucoidan derived from *Undaria*, the fucoidan derived from *Lessonia*, the fucoidan derived from *Ascophyllum*, and the fucoidan derived from other algae can be prepared by a known method, and used in the present invention.

Fucoidan-containing sea cucumber includes, for instance, sea cucumber disclosed in Japanese Patent Laid-Open No. Hei 4-91027, and the fucoidan can be prepared from sea cucumber by the method described in the publication.

In addition, the degradation products of the acidic polysaccharides, for instance, degradation products of sulfated polysaccharides and fucoidans, having induction action for growth factor production can be prepared by a known method such as an enzymological method, a chemical method, or a physical method, and a desired degradation product having induction action for growth factor production can be selected and used.

The degradation product refers to those obtained by degrading an acidic polysaccharide, wherein the degradation product has a molecular weight of preferably about 200 to about 100000, more preferably about 1000 to about 30000, depending upon the acidic polysaccharide to be degraded.

The preferable preparation method for the degradation product of the acidic polysaccharide used in the present invention is acid degradation method. By subjecting the acidic polysaccharide to an acid degradation, the degradation product having induction action for growth factor production can be prepared.

The conditions for the acid degradation of the acidic polysaccharide used in the present invention are not particularly limited, as long as the conditions enable to generate the degradation product having induction action for growth factor production (hereinafter referred to as "degradation product of the present invention").

For instance, the acidic polysaccharide is dissolved or suspended in an acidic aqueous solution or the like and subjected to the reaction, thereby generating a degradation product of the present invention. Also, the reaction mixture may be heated during the reaction, thereby shortening the time period required for the generation of the degradation product of the present invention.

The kinds of the acids for dissolving or suspending the acidic polysaccharide are not particularly limited. There can be used inorganic salts of hydrochloric acid, sulfuric acid, nitric acid and the like; organic acids such as citric acid, formic acid, acetic acid, lactic acid and ascorbic acid; and solid acids such as cationic exchange resin, cationic exchange fiber and cationic exchange membrane.

The concentration of the acid is not particularly limited, and the acid can be used at a concentration of preferably from 0.0001 to 5 N or so, more preferably from 0.01 to 1 N or so. In addition, the reaction temperature is not particularly limited, and the reaction temperature may be set at preferably from 0° to 200°C, more preferably from 20° to 130°C.

In addition, the reaction time is not particularly limited, and the reaction time may be set at preferably from several seconds to several days. The kinds and the concentration of the acids, the reaction temperature, and the reaction time may be properly selected depending upon the generated amount of the degradation product used in the present invention and the degree of polymerization of the degradation product. For instance, during the preparation of the degradation product of the fucoidan, the organic acid such as citric acid, lactic acid or malic acid is used, and the concentration of the acid is properly selected from the range of several dozens mM to several M, the heating temperature from the range of 50° to 110°C, preferably 70° to 95°C, and the heating time from the range of several minutes to 24 hours, whereby the degradation product of the present invention can be prepared. The acid degradation product of the fucoidan is exemplified by the acid degradation product of the fucoidan derived from *Kjellmaniella crassifolia*, and this degradation product can be used as dietary fiber having induction action for growth factor production, especially having new physiological function of strong induction action of hepatocyte growth factor production.

The degradation product of the present invention can be fractionated by using its induction action for growth factor production as an index. For instance, an acid degradation product can be fractionated based on a molecular weight by means of a fractionation method such as gel filtration method, molecular weight fractionation membrane, or the like.

As an example of gel filtration method, Cellulofine GCL-300 can be used to prepare any molecular weight fractions, for instance, one having a molecular weight exceeding 25000, one having a molecular weight of 25000 to exceeding 10000, one having a molecular weight of 10000 to exceeding 5000, one having a molecular weight of 5000 or less. Cellulofine GCL-25 can be used to prepare any molecular weight fractions, for instance, one having a molecular weight 5000 or less, one having a molecular weight of 5000 to exceeding 3000, one having a molecular weight of 3000 to exceeding 2000, one having a molecular weight of 2000 to exceeding 1000, one having a molecular weight of 1000 to exceeding 500, one having a molecular weight of 500 or less.

In addition, the molecular weight fractionation can be industrially carried out by using an ultrafiltration membrane. For instance, a fraction having a molecular weight of 30000 or less can be prepared by using FE10-FUSO382 manufactured by DAICEL CHEMICAL INDUSTRIES, LTD., and a fraction having a molecular weight of 6000 or less can be prepared by using FE-FUS-T653 manufactured by the same. Further, a fraction having a molecular weight of 500 or less can be obtained by using a nanofilter membrane. Any molecular weight fractions can be prepared by combining these gel filtration method and molecular weight fractionation method.

The degradation product of the acidic polysaccharide, for instance, the degradation product of the fucoidan, having the induction action for growth factor production which can be used in the present invention is exemplified by the compound represented by the formula (I) and the compound represented by the formula (II), and each of these compounds can be prepared in accordance with the method disclosed in WO 97/26896 and WO 99/41288. The sulfated polysaccharides and oligosaccharides having a repeating structure of the compound represented by the formula (I) can be also used as the sulfated polysaccharide having induction action for growth factor production of the present invention.

The compound represented by the formula (I) can be obtained by treating the previously mentioned F-fucoidan with endo-sulfated polysaccharide degradation enzyme (F-fucoidan-specific degradation enzyme) produced by *Alteromonas sp.* SN-1009 (FERM BP-5747), and purifying the degradation product. As to the content and the site of sulfate group in the compound, any ones can be purified from the degradation product. In addition, the polymer of the compound represented by the formula (I) is contained in the degradation product, and can be separated and purified depending on its purposes.

The compound represented by the formula (II) can be obtained by treating the previously mentioned U-fucoidan with endo-sulfated polysaccharide degradation enzyme (U-fucoidan-specific degradation enzyme) produced by *Flavobacterium sp.* SA-0082 (FERM BP-5402), and purifying the degradation product. As to the content and the site of sulfate group in the compound, any ones can be purified from the degradation product. In addition, the polymer having the compound represented by the formula (II) as a basic backbone structure is also contained in the degradation product, and can be separated and purified depending on its purposes.

An example of the compound represented by the formula (I) includes a compound represented by the formula (VI) given below. Also, an example of the compound represented by the formula (II) includes a compound represented by the formula (VII) given below.

In addition, the fucoidan derived from *Kjellmaniella crassifolia* is heat-treated in the presence of the organic acid, whereby a polymer of glucuronic acid and mannose can be obtained, and the resulting polymer can be also used as an acidic polysaccharide having induction action for growth factor production. Also, by adjusting the heat treatment conditions and the heating time, polymers with any polymerization degrees can be prepared.

The acidic polysaccharide having induction action for growth factor production in the present invention includes synthetic sulfated polysaccharides. There can be used sulfates of cellulose, starch, mannan, xylan, alginic acid, pectin, pectic acid, fructan, arabinan, chitin, pullulan, xyloglucan, dextran, and the like. Further, for instance, synthetic sulfated polysaccharides such as ribofuranan sulfate, xylofuranan sulfate, lentinan sulfate, curdlan sulfate and mannopyranan sulfate and synthetic sulfated alkyl polysaccharides such as a ribofuranan sulfate having palmitoyl group can be used. The highly-sulfated sulfated polysaccharide or highly sulfated degradation product can be prepared by further sulfating the sulfated polysaccharide or degradation product thereof. Each of these sulfated polysaccharides, highly-sulfated sulfated polysaccharides and highly sulfated degradation products may be prepared by a known method, and the degradation product thereof can be prepared by a known method and used in the present invention. In addition, commercially available dextran sulfate and sulfated cellulose can be used, and salts of those synthetic sulfated polysaccharides and the like may be used.

The acidic oligosaccharide preferably includes sulfated oligosaccharides, and the acidic monosaccharide preferably includes sulfated monosaccharides. Each concrete example includes the same ones as those mentioned above. The sulfated oligosaccharide or sulfated monosaccharide can be prepared each by sulfating each of the corresponding oligosaccharide or monosaccharide as a raw material by a known method. Also, salts of these sulfated oligosaccharide and sulfated monosaccharide can be preferably used. Further, the sulfated polysaccharides, the sulfated oligosaccharides and the sulfated monosaccharides of the present invention encompass fatty acid-added derivatives of the sulfated polysaccharides, the sulfated oligosaccharides and the sulfated monosaccharides and the like. Each of these can be used alone or in admixture of two or more kinds.

The growth factor desired to induce the production in the present invention is not particularly limited, as long as the growth factor has activity for accelerating the cell growth. The growth factor is exemplified by hepatocyte growth factor (HGF), nerve growth factor (NGF), neurotrophic factor, epidermal growth factor, milk-derived growth factor, fibroblast growth factor, brain-derived fibroblast growth factor, acidic fibroblast growth factor, platelet-derived growth factor, platelet basic protein, connective tissue-activated peptide, insulin-like growth factor (IGF), colony stimulating factor, erythropoietin, thrombopoietin, T cell growth factor, interleukins (for instance, interleukins 2, 3, 4, 5, 7, 9, 11 and 15), B cell growth factor, cartilage-derived factor, cartilage-derived growth factor, bone-derived growth factor, skeletal growth factor, epithelial cell growth factor, epithelial cell-derived growth factor, oculus-derived growth factor, testis-derived growth factor, Sertoli's cell-derived growth factor, mammotropic factor, spinal cord-derived growth factor, macrophage-derived growth factor, recycled mesodermal growth factor, transforming growth factor- , transforming growth factor-β, heparin-binding EGF-like growth factor, amphyllegrin, SDGF, betacellulin, epiregulin, neuregulin 1, 2 and 3, vascular endotherial growth factor, neurotrophin, BDNF, NT-3, NT-4, NT-5, NT-6, NT-7, glial cell line-derived neurotrophic factor, stem cell factor, midkine, pleiotrophin, ephrin, angiopoietin, activin, tumor necrosis factor, interferons, and the like.

Among them, it is preferable to induce the production of at least one kind selected from the group consisting of HGF, NGF, and IGF by using the effective ingredient of the present invention, from the viewpoints of prophylaxis and treatment of hepatic diseases, prophylaxis and treatment of neural diseases, and prophylaxis and treatment of diabetes.

HGF exhibits growing action for hepatocytes, accelerating action for protein synthesis, ameliorating action for cholestasia, and further preventing action for hepatic diseases caused by drugs and the like. In addition, mRNA of HGF is also synthesized in the brain, the kidney, the lungs, and the like, which is a mesoblast growth factor having growing activity for hepatocytes, renal tubule cells, epidermal cells and the like. Therefore, by inducing the production of hepatocyte growth factor, the treatment or prophylaxis of hepatitis, severe hepatitis, fulminant hepatitis, cirrhosis, and cholestasia in the liver, chronic nephritis, pneumonia or wound can be carried out.

IGF exhibits various physiological action on various cells. By inducing the IGF production, the treatment or prophylaxis of diabetes type II (insulin-independent) and growth impairment (dwarfism) can be carried out.

NGF is an endogenous growth factor for maintaining viability and functions of nerve cells, and elongating nerve cells in accordance with a concentration gradient of NGF. By inducing the NGF production, the treatment or prophylaxis of senile dementia such as Alzheimer's disease, peripheral nervous system disorder, cerebrovascular disorder, cerebral tumor, cerebral apicitis, a degenerative disease associated with head injury, diseases requiring recovery and regeneration of nerve functions, caused by intoxication with an anesthetic, and the like can be carried out. In addition, the therapeutic agent or prophylactic agent of the present invention exhibits induction action for neurotrophic factor. Owing to induction action for NGF/neurotrophic factor production, the therapeutic agent or prophylactic agent of the present invention is useful for treatment and prophylaxis of amyotrophic lateral sclerosis, drug-induced peripheral nervous system disorder, diabetic peripheral nervous system disorder, Alzheimer's disease, Parkinson's disease, sensory nerve disorder, retinitis pigmentosa, macular dystrophy, and the like.

The acidic polysaccharide, degradation product thereof, acidic oligosaccharide, acidic monosaccharide, acidic sugar alcohol or salt thereof used in the present invention has induction action for growth factor production, and a therapeutic agent or prophylactic agent for a disease requiring growth factor production comprising each of these compounds as an effective ingredient can be prepared.

The therapeutic agent or prophylactic agent for a disease requiring induction for growth factor production of the present invention comprises a compound selected from acidic polysaccharides, degradation products thereof, acidic oligosaccharides, acidic monosaccharides, acidic sugar alcohols and salts thereof used in the present invention as an effective ingredient, and the agent may be formed as a preparation by combinating the effective ingredient with a known pharmaceutical vehicle. The preparation can be generally produced by formulating a compound selected from acidic polysaccharides, degradation products thereof, acidic oligosaccharides, acidic monosaccharides, acidic sugar alcohols and salts thereof used in the present invention, with a pharmaceutically acceptable liquid or solid vehicle, and optionally adding a solvent, a dispersant, an emulsifier, a buffer, a stabilizer, an excipient, a binder, a disintegrant, a lubricant, and the like, thereby being usually made a solid agent such as a tablet, a granule, a powder, a fine powder, and a capsule, or liquid agent, suspension agent, emulsion agent, or the like. In addition, a dry product which can be made liquid by adding an appropriate vehicle before use can be also prepared.

The pharmaceutical vehicle can be selected depending upon the above-mentioned preparation form. In the case of an orally administered preparation, for instance, starch, lactose, saccharose, mannitol, carboxymethyl cellulose, cornstarch, inorganic salt and the like are available. In addition, during the preparation of the orally administered preparation, a binder, a disintegrant, a surfactant, a lubricant, a fluidity accelerator, a flavor, a colorant, a perfume, and the like can be further formulated.

On the other hand, in the case of a non-orally administered preparation, according to the conventional method, the preparation can be produced by dissolving or suspending a compound selected from acidic polysaccharides, degradation products thereof, acidic oligosaccharides, acidic monosaccharides, acidic sugar alcohols and salts thereof used in the present invention, which is an effective ingredient of the present invention, in distilled water for injection, physiological saline, aqueous glucose solution, vegetable oil for injection, sesame oil, peanut oil, soybean oil, corn oil, propylene glycol, polyethylene glycol or the like as a diluent, and optionally adding a sterilizer, a stabilizer, an osmotic regulator, a soothing agent, or the like.

The therapeutic agent or prophylactic agent of the present invention is administered via an administration route appropriate for each of the preparation form. The administration route is not limited to specific one. The agent can be administered internally or externally (or topically) or by injection. The injection can be administered, for instance, intravenously, intramuscularly, subcutaneously, intracutaneously, or the like. External preparations include a suppository.

The dosage for the therapeutic agent or prophylactic agent of the present invention is changeable and properly set depending upon its preparation form, administration method, purpose of use, age, body weight, symptom or the like of the patient to which the agent is applied, or the like. The dosage for adult per day is generally such that the amount of the compound selected from acidic polysaccharides, degradation products thereof, acidic oligosaccharides, acidic monosaccharides, acidic sugar alcohols and salts thereof used in the present invention contained in the preparation is preferably from 0.01 to 2000 mg/kg. As a matter of course, the dosage varies depending upon various conditions as mentioned above, so that an amount smaller than the dosage mentioned above may be sufficient, or an amount exceeding the dosage range may be required. In the case of the orally administered preparation, the therapeutic agent or prophylactic agent of the present invention can be directly orally administered within the desired dosage range, or the agent can be added to any foodstuffs to take it on a daily basis. Also, the compound selected from acidic polysaccharides, degradation products thereof, acidic oligosaccharides, acidic monosaccharides, acidic sugar alcohols and salts thereof used in the present invention may be used as a raw material of foodstuffs for induction of growth factor production.

A liver subjected to partial hepatectomy quickly regenerates and regains its original size. Although the substance of the factor for hepatic regeneration has been unknown for many years, HGF has been found in plasma of a patient suffering from fulminant hepatitis, and isolated and purified from the plasma of this patient (*J. Clin. Invest.,* **88** 414-419, 1988). Further, human HGF cDNA has been also cloned, and the primary structure for HGF has been also elucidated (*Biochem. Biophys. Res. Commun*., **163** 967-973, 1989). In addition, it has been elucidated that scatter factor (SF) for facilitating motility of cells, and a tumor cell disorder factor, tumor cytotoxic factor (TCF) are identical substances to HGF (*Proc. Natl. Acad. Sci. USA*, **88** 7001-7005, 1991; *Biochem. Biophys. Res. Commun.*, **180** 1151-1158, 1991).

HGF accelerates growth of many of epithelial cells, such as changioepithelial cells, renal tubule epithelial cells, and gastric mucosa cells, as well as hepatocytes. In addition, it induces morphological formations as seen in facilitation of motility of epithelial cells, vascularization or luminal formation of epithelial cells, so that HGF is a multi-functioning active substance exhibiting a wide variety of physiological activity. In other words, in various organs, HGF induces morphological formations such as proliferation acceleration and facilitation of motility of epithelial cells, or vascularization during the recovery of the disorder of the organ, and the like.

HGF exhibits growing action for hepatocytes, accelerating action for protein synthesis, ameliorating action for cholestasia, and further prophylactic action for hepatic diseases caused by drugs and the like. From these facts, HGF is expected as a therapeutic agent for severe hepatitis, cirrhosis, and cholestasia in the liver. However, HGF itself has not been used as a therapeutic agent in a practical manner. Further, although a method of introducing HGF gene in gene therapy has been also tried, its use is far from the practical level because of adverse action caused by acting at an unnecessary period and location. As described above, it would be thought that HGF is effective in the treatment and prophylaxis of diseases requiring an increase of HGF expression such as hepatitis, cirrhosis, and cholestasia in the liver, if HGF could be desirably induced without external administration. So far, the induction actions of IL-1, prostaglandins E₁ and E₂, heparin, and the like have been confirmed. Each of IL-1 and prostaglandins E₁ and E₂ induces HGF production by inducing transcription of HGF gene.

On the other hand, heparin has been known for its induction action for HGF production, and heparin induces HGF production by accelerating the steps on or after the translation of mRNA without inducing the transcription of HGF gene. In other words, there is no effect for induction of HGF production in a state where the transcription of HGF gene is not induced. Conversely, there is observed a remarkable induction for production in a state where the transcription of HGF gene is induced.

In addition, the effective ingredient in the present invention does not necessarily directly induce the transcription of a growth factor such as HGF. However, it is deduced that the effective ingredient significantly accelerates the transcription when the transcription is induced, and can further accelerate steps on or after the transcription such as translation. Consequently, the effective ingredient has action of induction of enhancement for growth factor production. In other words, the term "induction action for growth factor production" used herein means action of induction of enhancement for growth factor production, and this action is evaluated, for instance, on the basis of the enhancement of growth factor before or after the administration of the effective ingredient to a human. The phrase "when the transcription is induced" as referred to herein means that the above-mentioned effective ingredient further accelerates the transcription of HGF at an early stage when the transcription of HGF, for instance, is carried out at a necessary timing without subsequently overproduced, whereby HGF production is enhanced in the body. Thus, the induction for HGF production can be carried out very safely.

In the therapeutic agent or prophylactic agent of the present invention, there can be further contained a substance capable of synergistically increasing induction action for growth factor production of the acidic polysaccharide, degradation product thereof, acidic oligosaccharide, acidic monosaccharide, acidic sugar alcohol or salt thereof used in the present invention.

The term "substance capable of synergistically increasing (induction action)" refers to a substance by which induction for transcription is positively carried out, thereby consequently synergistically increasing induction action for growth factor production of the effective ingredient in the present invention, when the effective ingredient in the present invention, and the substance are used together.

The substance capable of synergistically increasing induction action for growth factor production of the acidic polysaccharide, degradation product thereof, acidic oligosaccharide, acidic monosaccharide, acidic sugar alcohol or salt thereof used in the present invention is not particularly limited, as long as the substance has action of synergistically increasing induction action for growth factor production of the acidic polysaccharide, degradation product thereof, acidic oligosaccharide, acidic monosaccharide, acidic sugar alcohol or salt thereof. The substance is exemplified, for instance, by a substance selected from cytokines, prostaglandins, and compounds having a cyclopentene ring, such as minoxidil and calpronium chloride. In addition, shogaol, gingerol and the like contained in ginger, curcumin and the like contained in turmeric and the like are also substances increasing the induction action for HGF production, and can be used as a substance capable of synergistically increasing induction action for HGF production of the acidic polysaccharide, degradation product thereof, acidic oligosaccharide, acidic monosaccharide, acidic sugar alcohol or salt thereof used in the present invention.

The cytokines include IL-1, and the like, and the prostaglandins include prostaglandins E₁ and E₂, and the like.

In addition, the compounds having a cyclopentene ring are exemplified by a compound represented by the following formula (III) and derivatives thereof.

Each of these substances can be used alone or in admixture of two or more kinds.

For instance, each of the compounds having a cyclopentene ring represented by the following formulas (III) to (V) can induce transcription of HGF gene in the same manner as prostaglandins E₁ and E₂, so that HGF production can be remarkably increased by synergistic action with the acidic polysaccharide, degradation product thereof, acidic oligosaccharide, acidic monosaccharide, acidic sugar alcohol or salt thereof used in the present invention. In other words, by using as a mixture of a substance selected from cytokines, prostaglandins, compounds having a cyclopentene ring, ginger-derived compounds, and turmeric-derived compounds, together with a compound selected from acidic polysaccharides, degradation products thereof, acidic oligosaccharides, acidic monosaccharides, acidic sugar alcohols and salts thereof used in the present invention, the induction action for growth factor production of the acidic polysaccharide, degradation product thereof, acidic oligosaccharide, acidic monosaccharide, acidic sugar alcohol or salt thereof used in the present invention is synergistically increased, thereby obtaining very high induction effect for HGF production.

In addition, the mixture may be used as a raw material for the foodstuff, or feed for induction of growth factor production.

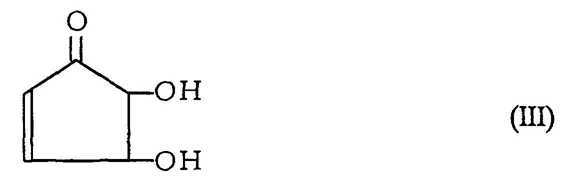

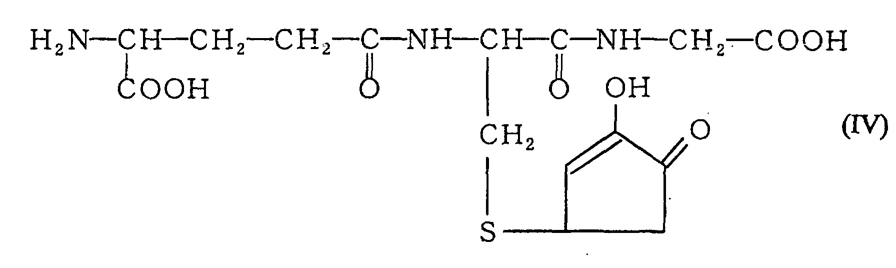

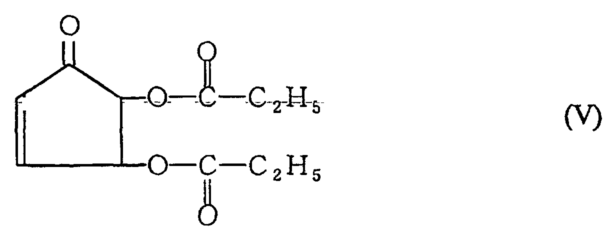

For instance, the method for preparing the compound represented by the formula (III) is described in WO 98/13328, the method for preparing the compound represented by the formula (IV) is described in WO 98/39291, and the method for preparing compound represented by the formula (V) is described in WO 98/40346, respectively, and these compounds can be prepared by the methods described therein.

The method for preparing the compound represented by the formula (III) may be any method, and the compound may be synthesized by chemical synthesis process [*Carbohydrate Res.,* **2478**, 217-222 (1993); *Helvetica Chimica Acta*, **55**, 2838-2844 (1972)]. In addition, cyclopentenone generated in a heat-treated product of at least one substance selected from uronic acid, a uronic acid derivative, a saccharide compound comprising uronic acid and/or a uronic acid derivative, a saccharide compound-containing substance comprising uronic acid and/or a uronic acid derivative, and a purified product thereof can be also used. The compound represented by the formula (IV) can be obtained by, for instance, reacting the compound represented by the formula (III) with glutathione. In addition, the compound represented by the formula (V) can be obtained by, for instance, reacting the compound represented by the formula (III) with propionic acid anhydride.

In the therapeutic agent or prophylactic agent of the present invention, the content of the substance capable of synergistically increasing induction action for growth factor production of the acidic polysaccharide, degradation product thereof, acidic oligosaccharide, acidic monosaccharide, acidic sugar alcohol or salt thereof used in the present invention is not particularly limited, as long as the content is at a level in which the induction action can be synergistically increased. Usually, the amount for adult is preferably from 0.001 to 2000 mg/kg per day. The substance capable of synergistically increasing induction action may be formed into preparations together with the compound selected from acidic polysaccharides, degradation products thereof, acidic oligosaccharides, acidic monosaccharides, acidic sugar alcohols and salts thereof used in the present invention, or formed into preparation separately. The method of forming preparations and the embodiments for administration may be carried out in accordance with the method described in the present specification, whereby obtaining the desired effects of the present invention such that the induction for growth factor production is synergistically increased.

The acidic polysaccharide, degradation product thereof, acidic oligosaccharide, acidic monosaccharide, acidic sugar alcohol, or salt thereof used in the present invention also has heparanase inhibitory activity, and has cancer-metastasis suppressing activity and vascularization suppressing activity. Therefore, there can be prepared and provided an agent for suppressing cancer-metastasis or an agent for suppressing vascularization by using a compound selected from these given above as an effective ingredient. Especially the compound represented by the formula (I) derived from fucoidan has a strong heparanase inhibitory action and cancer-metastasis suppressing action, so that a pharmaceutical composition comprising the compound as an effective ingredient is very useful as an agent for suppressing cancer-metastasis. A foodstuff comprising the compound is highly valuable as a foodstuff for suppressing cancer-metastasis or suppressing vascularization.

The food, beverage or feed for induction of growth factor production, comprising a compound selected from acidic polysaccharides, degradation products thereof, acidic oligosaccharides, acidic monosaccharides, acidic sugar alcohols and salts thereof used in the present invention, wherein the compound has induction action for growth factor production, is extremely useful in the amelioration or prophylaxis of symptoms of diseases requiring induction for growth factor production showing sensitivity to the acidic polysaccharide, degradation product thereof, acidic oligosaccharide, acidic monosaccharide, acidic sugar alcohol or salt thereof used in the present invention or improvement of physical condition of an organism as described below, owing to its induction action for growth factor production.

The term "comprise or comprising" as referred to in the food, beverage or feed, or the cosmetic (described below) of the present invention includes the meanings of containing, adding and diluting. The term "containing" refers to an embodiment of containing the effective ingredient used in the present invention in the food, beverage or feed; the term "adding" refers to an embodiment of adding the effective ingredient used in the present invention to a raw material for the food, beverage or feed; and the term "diluting" refers to an embodiment of adding a raw material for the food, beverage or feed to the effective ingredient used in the present invention.

In addition, it is preferable that the food, beverage or feed further comprises the above-mentioned substance capable of synergistically increasing induction action for growth factor production, for instance, a compound selected from cytokines, prostaglandins, and compounds having a cyclopentene ring, from the viewpoints of contributions to amelioration or prophylaxis of the above-mentioned diseases, or improvement of physical condition.

In the foodstuff of the present invention, the preferred embodiments of the effective ingredient, the growth factor, or the substance capable of synergistically increasing induction action for growth factor production are the same as those for the above-mentioned therapeutic agent or prophylactic agent. Especially as the foodstuff or feed of the present invention, the foodstuff or feed used for induction of hepatocyte growth factor production, induction of insulin-like growth factor production or induction of nerve growth factor production is preferable, from the viewpoint of contributions to amelioration in hepatic diseases, amelioration in neural diseases, or amelioration in diabetes.

The method for preparing the food or beverage of the present invention is not particularly limited, as long as the food or beverage having induction action for growth factor production is obtained. For instance, formulation, cooking, processing, and the like can be carried out in accordance with those generally employed for foods, and the food or beverage of the present invention can be prepared by the preparation methods for general food or beverage, so that the resulting food or beverage may contain the compound selected from acidic polysaccharides, degradation products thereof, acidic oligosaccharides, acidic monosaccharides, acidic sugar alcohols and salts thereof used in the present invention as an effective ingredient, wherein the compound has induction action for growth factor production.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks, and rice snacks; alcohol beverages such as *sake,* Chinese liquor, wine, whisky, Japanese distilled liquor *(shochu),* vodka, brandy, gin, ram, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like.

The food or beverage of the present invention comprises a compound selected from acidic polysaccharides, degradation products thereof, acidic oligosaccharides, acidic monosaccharides, acidic sugar alcohols and salts thereof, wherein the compound has induction action for growth factor production, and its shape is not particularly limited as long as an amount necessary for the compound to exhibit the physiological functions is contained, including products shaped into tablets, granules, capsules or the like, which can be orally taken. Here, the sulfated polysaccharide derived from an algae and a degradation product thereof, having induction action for growth factor production, for instance, fucoidan and a degradation thereof, are extremely useful as a production material for a food or beverage which is a health food material having both physiological action and dietary fiber function.

The content of the compound selected from acidic polysaccharides, degradation products thereof, acidic oligosaccharides, acidic monosaccharides, acidic sugar alcohols and salts thereof (effective ingredient) in the food or beverage of the present invention, wherein the compound has induction action for growth factor production, is not particularly limited, and the content can be appropriately selected from the viewpoints of texture and physiological activity. The content of the effective ingredient is, for instance, 10⁻⁹ parts by weight or more, preferably from 10⁻⁷ to 2 parts by weight, per 100 parts by weight of the food, or for instance, 10⁻⁹ parts by weight or more, preferably from 10⁻⁷ to 2 parts by weight, per 100 parts by weight of the beverage.

Also, the food or beverage may be taken such that the effective ingredient is from 0.01 to 2000 mg/kg per day for adult, whereby obtaining the desired effects of the present invention that the induction for growth factor production is carried out orally.

In addition, according to the present invention, there is provided a feed for an organism comprising a compound selected from acidic polysaccharides, degradation products thereof, acidic oligosaccharides, acidic monosaccharides, acidic sugar alcohols and salts thereof, wherein the compound has induction action for growth factor production.

Further, there is provided a method of feeding an organism, characterized by administering the feed to the organism.

In addition, there is provided an organism feeding agent characterized in that the organism feeding agent comprises a compound selected from acidic polysaccharides, degradation products thereof, acidic oligosaccharides, acidic monosaccharides, acidic sugar alcohols and salts thereof, wherein the compound has induction action for growth factor production.

In these inventions, the organism includes, for instance, culturing or breeding animals, pet animals, and the like. The culturing or breeding animal is exemplified by cattle, experimental animals, poultry, pisces, crustaceae or shellfish.

The feed is exemplified by a feed for physical condition improvement on the basis of the induction action for growth factor production.

The organism feeding agent includes immersion agents, feed additives, and beverage additives.

In these inventions, the compound selected from acidic polysaccharides, degradation products thereof, acidic oligosaccharides, acidic monosaccharides, acidic sugar alcohols and salts thereof, wherein the compound has induction action for growth factor production, has an effect of improving a feeding efficiency of an organism, for instance, survival rate, fattening ratio, egg production ratio, calf production ratio, weaning ratio, or the like.

Usually, the compound selected from acidic polysaccharides, degradation products thereof, acidic oligosaccharides, acidic monosaccharides, acidic sugar alcohols and salts thereof, wherein the compound has induction action for growth factor production, is administered preferably from 0.01 to 2000 mg per 1 kg of body weight of the subject organism per day. The compound can be added and mixed in a raw material for an artificially formulated feed, or can be mixed with a powder raw material for an artificially formulated feed, and thereafter the resulting mixture is further added to and mixed with other raw materials.

The content of the compound selected from acidic polysaccharides, degradation products thereof, acidic oligosaccharides, acidic monosaccharides, acidic sugar alcohols and salts thereof, wherein the compound has induction action for growth factor production, in the finally obtained feed for the subject organisms is not particularly limited. The compound may be used in accordance with its purposes, and an appropriate proportion in the feed is from 0.001 to 15% by weight. For instance, for the purpose of ameliorating hepatic function, the proportion of from 0.01 to 10% by weight is appropriate.

The artificially formulated feed includes those artificially formulated feeds using animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; and other raw materials such as vitamins, minerals, amino acids, and antioxidants; and the like. In addition, feeds for fish such as fish minced meat are also included.

The method for preparing the feed of the present invention is not particularly limited. In addition, the formulation may be in accordance with those of general feeds, as long as an effective amount of the compound selected from acidic polysaccharides, degradation products thereof, acidic oligosaccharides, acidic monosaccharides, acidic sugar alcohols and salts thereof, wherein the compound has induction action for growth factor production, is contained in the feed produced.

Also, the compound selected from acidic polysaccharides, degradation products thereof, acidic oligosaccharides, acidic monosaccharides, acidic sugar alcohols and salts thereof, wherein the compound has induction action for growth factor production, can be administered by directly adding the compound to water, seawater, or the like in a pool, a water tank, a water reservoir, or a feeding range, and immersing a subject organism into the resulting solution. The immersion method is especially effective when the amount of intake of the feed of the subject organism is lowered.

The concentration of the compound selected from acidic polysaccharides, degradation products thereof, acidic oligosaccharides, acidic monosaccharides, acidic sugar alcohols and salts thereof, wherein the compound has induction action for growth factor production, in water or seawater is not particularly limited, and the compound may be used in accordance with its purposes. It is appropriate that the concentration is preferably from 0.00001 to 1% by weight.

Also, a beverage comprising the compound selected from acidic polysaccharides, degradation products thereof, acidic oligosaccharides, acidic monosaccharides, acidic sugar alcohols and salts thereof, wherein the compound has induction action for growth factor production, may be given to a subject organism as a feeding drink.

The concentration of the compound selected from acidic polysaccharides, degradation products thereof, acidic oligosaccharides, acidic monosaccharides, acidic sugar alcohols and salts thereof, wherein the compound has induction action for growth factor production, in the beverage is not particularly limited, and the compound may be used in accordance with its purposes. It is appropriate that the concentration is preferably from 0.0001 to 1% by weight.

The organism feeding agent, for instance, an immersion agent, a feed additive, or a beverage additive comprising the compound selected from acidic polysaccharides, degradation products thereof, acidic oligosaccharides, acidic monosaccharides, acidic sugar alcohols and salts thereof, wherein the compound has induction action for growth factor production, as an effective ingredient may be prepared by known formulation and preparation method.

The organism to which the present invention can be applied is not limited. The culturing or breeding animals include cattle such as *Equus*, *Bos*, *Porcus, Ovis*, *Capra*, *Camelus*, and *Lama*; experimental animals such as mice, rats, guinea pigs, and rabbits; poultry such as *Chrysolophus*, ducks, *Meleagris,* and *Struthioniformes;* pisces such as *Pagrus, Oplegnathidae*, *Paralichthys*, plaice, *Seriola,* young *Seriola,* amberjack, *Thunna*, *Caranx delicatissimus*, *Plecoglossus, Salmo* · *Oncorhynchus, Fugu, Anguilla, Misguirus,* and *Parasilurus*; Crustaceae such as *Penaidae,* black tiger shrimp, *Penaeus roentalis,* and *Portulus trituberculatus*; and shellfish such as abalones (*awabi*), turban shells, scallops, and oysters; and the pet animals includes dogs, cats, and the like, so that the feed can be widely applied to animals on land and in water.

By allowing a subject organism to take the feed comprising the compound selected from acidic polysaccharides, degradation products thereof, acidic oligosaccharides, acidic monosaccharides, acidic sugar alcohols and salts thereof, wherein the compound has induction action for growth factor production, or immersing a subject organism into a solution containing the compound selected from acidic polysaccharides, degradation products thereof, acidic oligosaccharides, acidic monosaccharides, acidic sugar alcohols and salts thereof, wherein the compound has induction action for growth factor production, the physical conditions of the cattle, experimental animals, poultry, pisces, Caustacea, shellfish or the like are improved, so that the bacterial infections and viral infections of the subject organism is prevented or treated, whereby the symptoms in the infected organism are remarkably ameliorated. Also, the health of the subject organism is kept, so that its survival ratio, growth ratio, egg production ratio, calf production ratio, weaning ratio, nurturing ratio or the like are remarkably improved.

In addition, these breeding or culturing animals have had the problems such that (1) a disease caused by bacterial infections is frequently occurred, and if an epidemic disease is occurred, the animal is immediately infected due to culturing or breeding in a limited region, resulting in death of all animals; (2) invermination, nutritional diseases, environmental diseases, tumors and the like tend to be occurred; (3) the breeding or culturing animals in a narrow feeding region are subject to a large stress, so that scratches are generated by rubbing their body surface against a raising facility, whereby bacteria or parasites are likely to attach to individuals; (4) the amount of feed intake is lowered due to stress, so that growth is delayed; and the like. Owing to amelioration action of physical conditions, the stress of the breeding or culturing animal raised in a narrow region is dramatically reduced by the feed of the present invention, so that rubbing of the body surface to a raising facility is not generated, and the food appetite becomes flourishing, whereby the growth ratio, calf production ratio, egg production ratio, weaning ratio, disease preventive ratio or the like can be remarkably improved.

The acidic polysaccharide, degradation product thereof, acidic oligosaccharide, acidic monosaccharide, acidic sugar alcohol, or salt thereof used in the present invention, having induction action for growth factor production, is useful as an effective ingredient for a cosmetic. According to the present invention, there is provided a cosmetic for induction for growth factor production, for instance, HGF production, comprising as an effective ingredient a compound selected from acidic polysaccharides, degradation products thereof, acidic oligosaccharides, acidic monosaccharides, acidic sugar alcohols, and salts thereof used in the present invention.

In addition, it is preferable that the cosmetic comprises a substance capable of synergistically increasing induction action for growth factor production, for instance, a substance selected from cytokines, prostaglandins, and compounds having a cyclopentene ring, from the viewpoint of contributing to the desired effects.

In the cosmetic of the present invention, preferred embodiments for the effective ingredient, the growth factor, or the substance capable of synergistically increasing induction action for growth factor production mentioned above are the same as those of the above-mentioned therapeutic agent or prophylactic agent. Especially as the cosmetic of the present invention, the cosmetics used for induction of hepatocyte growth factor production, induction of insulin-like growth factor production or induction of nerve growth factor production are preferable, from the viewpoint of activation of epithelial cells.

As the effective ingredient for the cosmetic, the fucoidan and a degradation product thereof are especially preferable. For instance, there can be provided a biocosmetic having induction action for growth factor production, for instance, induction action for HGF production, comprising F-fucoidan and/or a degradation product, or the compound represented by the formula (I) as an effective ingredient. The content of the acidic polysaccharide, degradation product thereof, acidic oligosaccharide, acidic monosaccharide, acidic sugar alcohol, or salt thereof in the cosmetic for the induction for growth factor production is usually preferably from 0.0001 to 20% by weight, more preferably from 0.001 to 5% by weight.

The cosmetic for induction for growth factor production, for instance, induction for HGF production, of the present invention can be prepared in accordance with a conventional method according to a known formulation. The cosmetic for induction for growth factor production of the present invention encompasses, for instance, a lotion, a milky lotion, cream, a facial pack, a bathing agent, a facial cleansing agent, a bathing soap, a bathing detergent, or the like.

The cosmetic of the present invention can be used at a desired amount depending upon each application. For instance, in a case where the cosmetic is a lotion, if a lotion is applied onto an entire facial surface of a human, for instance, the amount of use per application is preferably from 0.01 to 5 g, more preferably 0.1 to 2 g or so, whereby epithelial cells are activated, to obtain the desired effect of the present invention such that beautifying effect is obtained.

The present invention also provides an inducing agent for growth factor production, comprising as an effective ingredient a compound selected from acidic polysaccharides, degradation products thereof, acidic oligosaccharides, acidic monosaccharides, acidic sugar alcohols and salts thereof, and the inducing agent for production is also useful for function studies of growth factor and screening of a pharmaceutical for a disease associated with growth factor.

Further, the present invention provides a controlling agent for growth factor production, characterized in that the controlling agent comprises as an effective ingredient a compound selected from acidic polysaccharides, degradation products thereof, acidic oligosaccharides, acidic monosaccharides, acidic sugar alcohols and salts thereof.

Each of the inducing agent for growth factor production and the controlling agent for growth factor production of the present invention may be formed into a preparation by a known preparation-forming method with using the above-mentioned effective ingredient. The inducing agent for growth factor production includes the above-mentioned therapeutic agents and the like as one example thereof. In addition, the controlling agent for growth factor production of the present invention means a preparation capable of accelerating transcription of growth factor at an early stage of the transcription induction of growth factor. By the use of the controlling agent for growth factor production of the present invention, there are remarkable effects such that the growth factor production is enhanced only when the growth factor production is necessary, so that the growth factor is not overproduced.

The fucoidan and/or a degradation product thereof used in the present invention has especially strong induction action for growth factor production or controlling action for growth factor production, and is extremely useful as an effective ingredient used in the preparation of the present invention.

Heparin of which induction action for HGF production has been conventionally known does not accelerate mRNA transcription of HGF, but the fucoidan and a degradation product thereof further accelerate its mRNA transcription at an early stage in which mRNA transcription of HGF is accelerated. mRNA of HGF is not always transcribed *in vivo,* but is transcribed at a necessary timing. The fucoidan and fucoidan degradation products, for instance, 7-12SFd-F described below, are very safe controlling substances for HGF production in the features such that transcription of HGF is accelerated only in an early stage where *in vivo* transcription of HGF is accelerated without subsequently overproducing HGF, and thereafter the HGF production is accelerated only when it is required in the body.

Therefore, in another embodiment of the present invention, the above-mentioned therapeutic agent or prophylactic agent, foodstuff, or the like can be directly used for the purpose of controlling induction of growth factor production. The dosage of the controlling agent for growth factor production is not particularly limited, as long as the growth factor production can be adjusted. The dosage of the controlling agent can be adjusted so that the dosage of the effective ingredient in the present invention, for instance, the dosage of the effective ingredient to human is preferably from 0.01 to 2000 mg/kg (body weight).

Here, in an oral administration of the acidic polysaccharide, for instance, the fucoidan and/or a degradation product thereof, having induction action for growth factor production used in the present invention, no case of death is found even when orally administered in a single dosage of 1 g/kg. In addition, sodium dextran sulfate is also a safe compound. In addition, another acidic polysaccharide, degradation product thereof, acidic oligosaccharide, acidic monosaccharide, acidic sugar alcohol or salt thereof used in the present invention is not found to be toxic when orally administered to a rat in its physiologically effective dosage.

In addition, as another embodiment in the present invention, there may be provided a therapeutic agent or prophylactic agent for a disease requiring induction for growth factor production, comprising as an effective ingredient an extract selected from *Altemisia princeps pampan* extracts, *Momordica charantia* extracts, Aloe extracts, *Chrysanthemum coronarium* extracts, *Chlorella* extracts, and *Spirulina* extracts, wherein the extract has induction action for growth factor production.

Also, there may be provided a foodstuff, or a feed for induction for growth factor production, comprising as an effective ingredient an extract selected from *Altemisia princeps pampan* extracts, *Momordica charantia* extracts, Aloe extracts, *Chrysanthemum coronarium* extracts, *Chlorella* extracts, and *Spirulina* extracts, wherein the extract has induction action for growth factor production.

Further, there may be provided a cosmetic for induction for growth factor production, comprising as an effective ingredient an extract selected from *Altemisia princeps pampan* extracts, *Momordica charantia* extracts, Aloe extracts, *Chrysanthemum coronarium* extracts, *Chlorella* extracts, and *Spirulina* extracts, wherein the extract has induction action for growth factor production.

The extraction and purification of the above-mentioned extracts from plants or microorganisms can be carried out by a known method as described below. A fruit, a seed, a leaf, a stem, a root, a rhizome, or the like of a plant, or a microorganism, which is a raw material, is collected at an appropriate time, and used without treatment, or usually after being subjected to a drying process of air-drying or the like as a raw material for the extract. In a case where the raw material is a squeezed juice or sap of a plant, it can be used without treatment as a raw material for the extract.

The extraction of the extract comprising the above-mentioned effective ingredient from dried plants and microorganisms mentioned above is carried out by a known method as described below. The extraction can be carried out by powdering or thinly slicing the raw material, and thereafter carrying out a extraction method in a batch process or continuous process with a solvent. As the extraction solvent, there can be used a hydrophilic or lipophilic solvent such as water; chloroform; an alcohol such as ethanol, methanol, or isopropyl alcohol; a ketone such as acetone or methyl ethyl ketone; methyl acetate; or ethyl acetate, alone or as a mixed solution. The extraction is carried out at a temperature of usually from 0° to 150°C, preferably from 5° to 120°C.

In a case where the extraction is carried out in a batch process, the extraction time period is from 10 minutes to 20 days or so, and the amount of the solvent is usually 1 to 30 times the weight, preferably 2 to 20 times the weight per dry raw material. The extraction step may be carried out by stirring or by immersing and allowing to stand, or a combination of both. The extraction step may be optionally repeated twice or three times. The continuous extraction process includes a process using a Soxhlet extractor in which a reflux condenser is combined with a siphon, and the like. The amount of the solvent, the extraction time period and the like are the same as the conditions for the extraction method in a batch process.

The extract used in the present invention encompasses those in which insoluble residues are removed from the crude extract obtained by the above-mentioned step by means of filtration or centrifugation. Also, the insoluble residue may be used as an active ingredient in some cases.

The purification of the active ingredient from the crude extract may be carried out by any process, as long as it is a known purification process for the active ingredient derived from a plant. It is preferable to use two-phase solvent separation method, column chromatography, or the like, alone or in combination.

There can be prepared a pharmaceutical agent, a foodstuff, a feed, a cosmetic or the like according to its purposes, comprising the resulting extract as an effective ingredient. The preparation of those can be carried out in accordance with the above-mentioned methods of the first to third inventions of the present invention.

The content of the extract in the manufactured product in accordance with each purpose can be determined on the basis of induction action for growth factor production. Generally, the content of the extract in the manufactured product is preferably from 0.001 to 100% by weight, more preferably from 0.01 to 30% by weight, more preferably from 0.1 to 20% by weight.

These extracts in the present invention are not found to be toxic even when an effective dosage is orally administered to a rat.

The present invention will be more concretely described by means of the examples, without by no means limiting the scope of the present invention thereto. "%" in the formulation of each component in the examples means "% by weight."

### Reference Example 1

(1) *Kjellmaniella crassifolia* was sufficiently dried, and thereafter 20 kg of the dried product was powdered with a free mill (manufactured by Nara Kikai Seisakusho).

In 900 liters of tap water was dissolved 7.3 kg of calcium chloride dihydrate (manufactured by Nippon Soda Co., Ltd.), and 20 kg of the powdered product of *Kjellmaniella crassifolia* was then mixed therewith. The resulting mixture was heated for 40 minutes until the liquid temperature was raised from 12°C to 90°C by blowing steam. Thereafter, the mixture was kept at 90° to 95°C for 1 hour under stirring, and then cooled, to give 1100 liters of a cooled product.

Subsequently, the cooled product was subjected to solid-liquid separation with a solid-liquid separator (manufactured by West Farrier Separator, Model: CNA), to give about 900 liters of supernatant of solid-liquid separation.

The amount 360 liters of the supernatant of solid-liquid separation was concentrated up to a volume of 20 liters with FE10-FC-FUS0382 (fraction molecular weight: 30000) manufactured by DAICEL CHEMICAL INDUSTRIES, LTD. Thereafter, the steps of adding 20 liters of tap water and again concentrating the resulting liquid mixture up to a volume of 20 liters were repeated 5 times, and the concentrate was subjected to a desalting treatment, to give 25 liters of an extract derived from *Kjellmaniella crassifolia.*

One liter of the extract was lyophilized, to give 13 g of a dried product of fucoidan derived from *Kjellmaniella crassifolia.*

A dried product of fucoidan derived from *Laminaria japonica* was prepared from a lyophilized, powdered product of *Laminaria japonica* according to the method described above. Similarly, a dried product of fucoidan derived from *Lessonia nigrescence* was prepared from a dry powder of *Lessonia nigrescence* (trade name: Seaweed Powder, sold by Andesu Boeki K.K.).

(2) Seven grams of the dried product of fucoidan described in item (1) of Reference Example 1 was dissolved in 700 ml of a 20 mM imidazole buffer (pH 8.0) containing 50 mM sodium chloride and 10% ethanol, and an insoluble matters were removed by centrifugation. The supernatant after centrifugation was applied onto a DEAE-Cellulofine A-800 column (φ 11.4 cm x 48 cm) equilibrated with the same buffer, and then washed with the same buffer. The elution was carried out with a concentration gradient of from 50 mM to 1.95 M sodium chloride (250 ml per fraction). A total sugar content and an uronic acid content were determined by the phenol-sulfuric acid method and the carbazole-sulfuric acid method, to give Fractions 43 to 49, Fractions 50 to 55, and Fractions 56 to 67, in the order of elution. Next, these fractions were desalted by electrodialysis, and thereafter lyophilized, to give each of Fraction I (340 mg) from Fractions 43 to 49, Fraction II (870 mg) from Fractions 50 to 55, and Fraction III (2.64 g) from Fractions 56 to 67.

Figure 1 shows an elution pattern of the fucoidan derived from *Kjellmaniella crassifolia* on the DEAE-Cellulofine A-800 column. In Figure 1, the axis of ordinates is the absorbance at 530 nm as determined by the carbazole-sulfuric acid method (solid circles in the figure), the absorbance at 480 nm as determined by the phenol-sulfuric acid method (open circles in the figure), and the electric conductivity (mS/cm: open squares in the figure), and the axis of abscissas is the fraction number.

### Reference Example 2

(1) A 2-liter Erlenmeyer flask was charged with 60 ml of a culture medium comprising an artificial sea water (manufactured by Jamarin Laboratory), pH 8.2, containing 0.25% glucose, 1.0% peptone, and 0.05% yeast extract, and then sterilized (at 120°C for 20 minutes). *Alteromonas* sp. SN-1009 (FERM BP-5747) was inoculated into the culture medium, and cultured at 25°C for 26 hours, to give a seed culture medium. A 30-liter jar fermentor was charged with 20 liters of a culture medium comprising an artificial sea water, pH 8.0, containing 1.0% peptone, 0.02% yeast extract, 0.2% sulfated polysaccharide described in item (2) of Reference Example 2 described below, and 0.01% defoaming agent (manufactured by Shin-Etsu Chemical Co., Ltd., KM70), and sterilized at 120°C for 20 minutes. After cooling, 600 ml of the above-mentioned seed culture medium was inoculated, and cultured at 24°C for 24 hours under the conditions of 10 liters of aeration per minute and a stirring rate of 250 rpm. After termination of the culture, the culture medium was centrifuged, to give cells and culture supernatant. The culture supernatant obtained was concentrated with an ultrafilter equipped with holofiber having an excluding molecular weight of 10000, and the concentrate was then subjected to salting out with an 85% saturated ammonium sulfate. Precipitates formed were harvested by centrifugation, and sufficiently dialyzed against a 20 mM Tris-HCl buffer (pH 8.2) containing an artificial sea water at a one-tenth concentration, to give 600 ml of a solution of an endo-sulfated polysaccharide-degrading enzyme, selectively acting on the sulfated polysaccharide.

(2) Two kilograms of dried *Kjellmaniella crassifolia* was powdered with a cutter mill (manufactured by Masuko Sangyo) fitted with a screen having a diameter of 1 mm, and the resulting sea tangle chips were suspended in 20 liters of 80% ethanol. The suspension was stirred at 25°C for 3 hours and filtered with a filter paper, and thereafter the residue was sufficiently washed. The residue obtained was suspended in 40 liters of a 20 mM sodium phosphate buffer, pH 6.5, which was heated to 95°C, the buffer containing 50 mM sodium chloride. The suspension was treated at 95°C for 2 hours with occasional stirring, to extract a sulfated polysaccharide.

A suspension in the extract was filtered, to give a filtrate. Thereafter, the filtration residue was washed with 3.5 liters of 100 mM sodium chloride, to give an additional filtrate.

Both filtrates were combined, and then the temperature was lowered to 30°C. After 3000 U of alginic acid lyase (manufactured by Nagase Seikagaku Kogyo) was added to the resulting mixture, 4 liters of ethanol was added thereto. The resulting mixture was stirred at 25°C for 24 hours. Next, the mixture was centrifuged, and the resulting supernatant was concentrated with an ultrafilter equipped with holofiber having an excluding molecular weight of 100000. Further, the ultrafiltration was continued with 100 mM sodium chloride containing 10% ethanol until a colored substance was no longer filtered.

Precipitates formed in a non-filtrate solution were removed by centrifugation, and the temperature of the resulting supernatant was lowered to 5°C. The pH was adjusted to 2.0 with 0.5 N hydrochloric acid, and thereafter the formed precipitates such as a protein were removed by centrifugation. The pH of the resulting supernatant was rapidly adjusted to 8.0 with 1 N sodium hydroxide.

Next, an ultrafiltration was carried out with an ultrafilter equipped with holofiber having an excluding molecular weight of 100000, and the solvent was completely substituted with 20 mM sodium chloride, pH 8.0. Thereafter, the pH was again adjusted to 8.0, and the resulting mixture was centrifuged and then lyophilized, to give about 95 g of a sulfated polysaccharide.

(3) Two kilograms of dried *Kjellmaniella crassifolia* was powdered with a cutter mill fitted with a screen having a diameter of 1 mm, and the resulting sea tangle chips were suspended in 20 liters of 80% ethanol. The resulting suspension was stirred at 25°C for 3 hours, and filtered with a filter paper, and thereafter the residue was sufficiently washed. The residue obtained was suspended in 20 liters of a buffer (pH 8.2) containing 30 ml of a solution of the endo-sulfated polysaccharide-degrading enzyme prepared in item (1) of the above-mentioned Reference Example 2, 10% ethanol, 100 mM sodium chloride, 50 mM calcium chloride and 50 mM imidazole, and the resulting mixture was stirred at 25°C for 48 hours. This suspension was filtered with a stainless screen having a screen-opening diameter of 32 µm, and the residue was washed with 10% ethanol containing 50 mM sodium chloride. Further, the residue was suspended in 10 liters of 10% ethanol containing 50 mM calcium chloride, and the suspension was stirred for 3 hours, and thereafter filtered with the stainless screen, and the residue was washed. Further, the residue was suspended under the same conditions, and the suspension was then stirred for 16 hours. The suspension was filtered with the stainless screen having a diameter of 32 µm, and the residue was washed.

The filtrate and the washings thus obtained were collected, and the combined mixture was subjected to ultrafiltration with an ultrafilter equipped with holofiber having an excluding molecular weight of 3000, thereby separating a filtered solution from a non-filtered solution.

This filtered solution was concentrated to a volume of about 3 liters with a rotary evaporator, and thereafter the concentrate was centrifuged, to give supernatant. The supernatant obtained was desalted with an electric dialyzer equipped with a membrane having an excluding molecular weight of 300. To the resulting solution was added calcium acetate so as to give a concentration of 0.1 M, and precipitates formed were removed by centrifugation. The resulting supernatant was applied onto a DEAE-Cellulofine column (amount of resin: 4 liters) previously equilibrated with 50 mM calcium acetate, and sufficiently washed with 50 mM calcium acetate and 50 mM sodium chloride. Thereafter, the elution was carried out with a gradient of from 50 mM to 800 mM sodium chloride. The amount collected at this time was 500 ml per fraction. The collected fraction was analyzed by cellulose acetate membrane electrophoresis [*Analytical Biochemistry,* **37,** 197-202 (1970)]. As a result, a sulfated saccharide which was eluted on a concentration of about 0.4 M sodium chloride (Proximity of Fraction No. 63) was homogeneous.

Then, a solution of Fraction No. 63 was first concentrated to a volume of 150 ml, and thereafter sodium chloride was added so as to give a concentration of 4 M. The resulting solution was applied onto a Phenyl-Cellulofine column (amount of resin: 200 ml) previously equilibrated with 4 M sodium chloride, and sufficiently washed with 4 M sodium chloride. Non-adsorbent sulfated saccharide fractions were collected, and desalted with an electrodialyzer equipped with a membrane having an excluding molecular weight of 300, to give 505 ml of a desalted solution.

Forty milliliters of the desalted solution obtained was applied onto a Cellulofine GCL-90 column (4.1 cm x 87 cm) equilibrated with 0.2 M sodium chloride containing 10% ethanol, to perform gel filtration. The collection was performed at 9.2 ml per fraction.

All of the fractions were analyzed for a total sugar content by the phenol-sulfuric acid method [*Analytical Chemistry,* **28,** 350 (1956)].

As a result, since the sulfated saccharide formed a single peak, Fraction Nos. 63 to 70, which were fractions corresponding to a central part of the peak were collected. The combined fraction was desalted with an electrodialyzer equipped with a membrane having an excluding molecular weight of 300, and thereafter lyophilized, to give 112 mg of a dried product of the compound represented by the following formula (VI). The compound is hereinafter referred to as 7-12SFd-F.

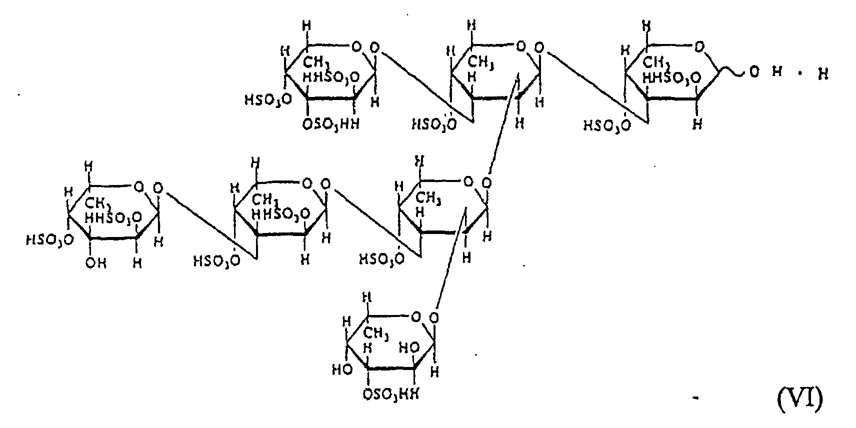

(4) To 80 ml of a 2.5% aqueous solution of Fraction III (F-fucoidan) prepared in item (2) of Reference Example 1 were added 16 ml of 1 M Tris-HCl buffer (pH 7.6), 16 ml of a 1 M aqueous CaCl₂ solution, 24 ml of a 4 M aqueous Sodium chloride solution, 8 ml of the solution of the endo-sulfated polysaccharide-degrading enzyme obtained in item (1) of Reference Example 2 and 176 ml of distilled water, and the resulting mixture was heated at 30°C for 3 hours. The resulting enzyrnatically treated F-fucoidan solution was concentrated with a rotary evaporator so as to give a final concentration of the enzymatically treated F-fucoidan of 2%, and thereafter the concentrate was dialyzed in distilled water, to give a 2% aqueous solution of the enzymatically treated F-fucoidan. This sample was analyzed by HPLC (column: SB802.5; column temperature: 35°C; mobile phase: 50 mM NaCl; flow rate: 0.5 ml/min; detection: RI ATT=8). As a result, it was revealed that about 40% of the sample was 7-12SFd-F.

### Reference Example 3

(1) Two kilograms of dried *Kjellmaniella crassifolia* was powdered with a cutter mill (manufactured by Masuko Sangyo) fitted with a screen having a hole diameter of 1 mm. After the powdered product was stirred in 20 liters of 80% ethanol at 25°C for 3 hours, the mixture was filtered, and the residue was washed. The resulting residue was suspended in 20 liters of a 30 mM imidazole buffer (pH 8.2) containing 50 mM calcium chloride, 100 mM sodium chloride, 10% ethanol, and 1 U of *Alteromonas* sp. SN-1009 (FERM BP-5747) endo-sulfated polysaccharide-degrading enzyme prepared in item (1) of Reference Example 2. The resulting suspension was stirred at 25°C for 2 days, and thereafter filtered with a stainless screen having a hole diameter of 32 µm, and the residue was washed. The resulting residue was suspended in 40 liters of a sodium phosphate buffer (pH 6.6) containing 100 mM sodium chloride, 10% ethanol and 4 g of an alginic acid lyase (manufactured by Nagase Seikagaku Kogyo). The resulting suspension was stirred at 25°C for 4 days, and thereafter centrifuged, to give supernatant. In order to remove low-molecular weight products of alginic acid contained in the supernatant obtained, the supernatant was concentrated to a volume of 2 liters with an ultrafilter equipped with holofiber having an excluding molecular weight of 100000, and thereafter the solvent was exchanged for 100 mM sodium chloride containing 10% ethanol. To the resulting solution was added an equivolume of 400 mM calcium acetate and stirred, and thereafter the mixture was centrifuged. The pH of the resulting supernatant was adjusted to 2 with 1 N hydrochloric acid, with cooling on ice. Precipitates formed were removed by centrifugation, and the pH of the resulting supernatant was adjusted to 8.0 with 1 N sodium hydroxide. This solution was concentrated to a volume of 1 liter by ultrafiltration, and thereafter the solvent was exchanged for 100 mM sodium chloride. Precipitates formed at this time were removed by centrifugation. In order to remove hydrophobic substances in the resulting supernatant, sodium chloride was added to the supernatant so as to give a concentration of 1 M, and the resulting mixture was applied onto a column containing 3 liters of Phenyl-Cellulofine (manufactured by Seikagaku Corporation) equilibrated with 1 M sodium chloride, to collect an effluent fraction. The fraction was concentrated with an ultrafilter, and the solvent was exchanged for 20 mM sodium chloride. The resulting solution was lyophilized, and the weight of the lyophilized product was 29.3 g.

(2) Fifteen grams of the above-mentioned lyophilized product was dissolved in 1.5 liters of 50 mM Tris-HCl buffer containing 400 mM sodium chloride and 9 U of an endo-sulfated polysaccharide-degrading enzyme obtained from a culture prepared by culturing *Flavobacterium* sp. SA-0082 (FERM BP-5402) disclosed in WO97/26896. After the resulting solution was subjected to the reaction at 25°C for 6 days, the reaction mixture was concentrated to a volume of about 300 ml with an evaporator. The concentrate was placed in a dialyzing tube having an excluding molecular weight of 3500 and thoroughly dialyzed. The solution remaining in the dialysis tube was applied onto a column containing 4 liters of DEAE-Cellulofine A-800 equilibrated with 50 mM sodium chloride, and sufficiently washed with 50 mM sodium chloride. Thereafter, the elution was carried out on a concentration gradient of from 50 to 650 mM sodium chloride. Further, the elution was sufficiently carried out in the same column with 650 mM sodium chloride. Among the eluted fractions, the fractions eluted with 650 mM sodium chloride were collected as a sulfated fucogalactan fraction, and concentrated with an ultrafilter having an excluding molecular weight of 100000. Thereafter, the solvent was substituted with 10 mM sodium chloride, and the resulting solution was lyophilized, to give 0.85 g of a lyophilized product of sulfated fucogalactan. The sulfated fucogalactan obtained was found to contain galactose and fucose as constituting saccharides in a molar ratio of about 2:1.

### Reference Example 4

One-hundred and twenty grams of the sulfated polysaccharide prepared in item (2) of Reference Example 2 was suspended in 8 liters of a 20 mM imidazole buffer (pH 7.5) containing 20 mM calcium chloride, 300 mM sodium chloride, 10% ethanol and 10 U of the endo-sulfated polysaccharide-degrading enzyme prepared in item (1) of Reference Example 2. The resulting suspension was stirred at 25°C for 3 days. The mixture was ultrafiltered with an ultrafilter equipped with holofiber having an excluding molecular weight of 100000, with the above-mentioned buffer added.

To the liquid inside the ultrafilter was added 34 U of the endo-sulfated polysaccharide-degrading enzyme prepared in item (2) of Reference Example 3. The mixture was stirred at 25°C for 2 days, and ultrafiltered with an ultrafilter equipped with holofiber having an excluding molecular weight of 100000, with water added.

The filtrate was collected and concentrated to a volume of 1.5 liters with an evaporator. Thereafter, the concentrate was completely desalted with a desalting device, and applied onto a column containing 3 liters of DEAE-Cellulofine A-800 previously equilibrated with a 5 mM imidazole-hydrochloric acid buffer (pH 6.5) containing 30 mM sodium chloride. After washing the column with 6 liters of the same buffer, the elution was carried out on a concentration gradient of from 30 mM to 500 mM sodium chloride. The amount of the solution required for the elution was 48 liters. The eluate was collected 180 ml each, and the sugar content thereof was determined by the phenol-sulfuric acid method. In addition, the absorbance at 232 nm was also determined. Eluted fractions on 130 mM to 170 mM sodium chloride formed a single peak. Therefore, these fractions were collected, desalted with a desalting device, and thereafter lyophilized, to give 5.85 g of an oligosaccharide. It was confirmed that the oligosaccharide had a molecular weight of 1128 by mass spectrometry, and was the compound represented by the following formula (VII) by NMR analysis. The compound is hereinafter referred to as 6-2S.

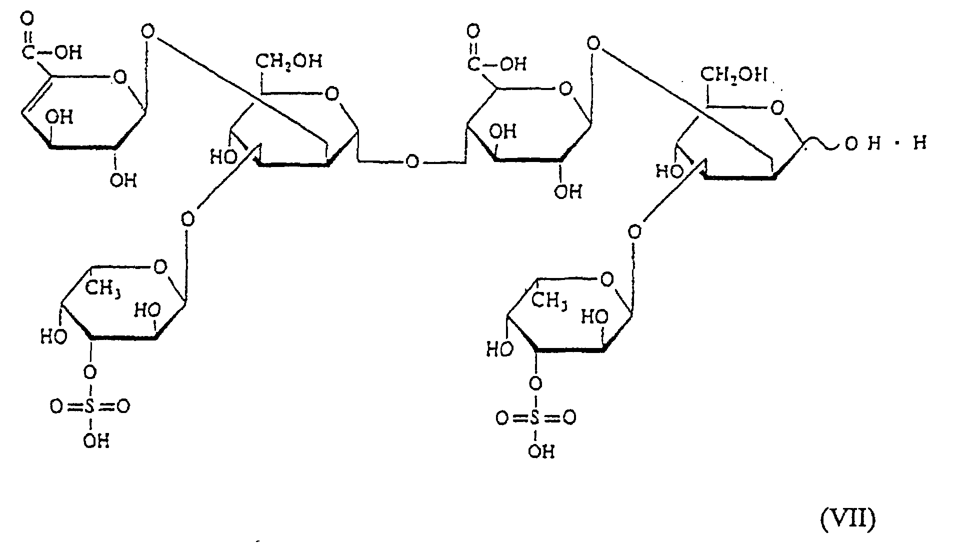

### Reference Example 6

One kilogram of a dried product of a commercially available sporophyll of *Undaria pinnatifida* (Wakame Mekabu) was powdered with a cutter mill fitted with a screen having a hole diameter of 1 mm. Thereafter, the powdered sporophyll was suspended in 10 liters of 80% ethanol, and the suspension was stirred for 3 hours, and thereafter filtered with a filter paper, to give a residue. The residue was suspended in 20 liters of a 40 mM sodium phosphate buffer (pH 6.5) containing 50 mM sodium chloride, and treated at 95°C for 2 hours. The treated solution was cooled to 37°C, and thereafter ethanol was added thereto so as to give a concentration of 10%. 12000 U of a commercially available alginic acid lyase K (manufactured by Nagase Seikagaku Kogyo) was added thereto, and thereafter the mixture was stirred at room temperature for 24 hours. The resulting treated solution was centrifuged, and the resulting supernatant was concentrated to a volume of 2 liters with an ultrafilter equipped with holofiber having an excluding molecular weight of 100000. Thereafter, precipitates formed were removed by centrifugation. The resulting supernatant was cooled to 5°C, and thereafter 0.5 N hydrochloric acid was added thereto to adjust the pH to 2.0. Subsequently, the resulting mixture was stirred for 30 minutes, and precipitates formed were removed by centrifugation. The pH of the resulting supernatant was adjusted to 8.0 with 0.5 N sodium hydroxide, and the solvent was substituted with 20 mM sodium chloride by ultrafiltration. The pH of the resulting solution was adjusted to 8.0, and thereafter the supernatant obtained by centrifuging it was lyophilized, to give 90.5 g of fucoidan derived from sporophyll of *Undaria pinnatifida.*

### Reference Example 7

One kilogram of a dried product of powdered *Fucus vesiculosus* was suspended in 10 liters of 80% ethanol, and the suspension was stirred for 3 hours, and thereafter filtered with a filter paper, to give a residue. The residue was suspended in 30 liters of a 30 mM sodium phosphate buffer (pH 6.0) containing 100 mM sodium chloride, and treated at 95°C for 2 hours. After the treated solution was cooled to 37°C, 100 g of activated carbon was added, and the mixture was stirred for 30 minutes. After 3000 U of a commercially available alginic acid lyase K was added, ethanol was added so as to give a concentration of 10%, and the resulting mixture was stirred at room temperature for 24 hours. The resulting treated solution was centrifuged, and the supernatant was concentrated to a volume of 2 liters with an ultrafilter equipped with holofiber having an excluding molecular weight of 100000. Thereafter, precipitates formed were removed by centrifugation, and the resulting supernatant was ultrafiltered with an extract added, to remove a pigment. The non-filtered solution obtained was cooled to 5°C, and thereafter 0.5 N hydrochloric acid was added thereto to adjust the pH to 2.0. Thereafter, the resulting solution was stirred for 30 minutes, and precipitates formed were removed by centrifugation. The pH of the supernatant was adjusted to 8.0 with 0.5 N sodium hydroxide, and the solvent was substituted with 20 mM sodium chloride by ultrafiltration. The pH of the resulting solution was adjusted to 8.0, and thereafter the supernatant obtained by centrifuging it was lyophilized, to give 71 g of fucoidan derived from *Fucus vesiculosus.*

Fucoidan derived from *Ascophyllum nodosum* was prepared from a dry powder of *Ascophyllum nodosum* (trade name: Algin Gold, sold by Andesu Boeki K.K) according to the method described above.

### Reference Example 8

Two grams of fucoidan derived from *Kjellmaniella crassifolia* prepared by the method described in item (1) of Reference Example 1 was dissolved in 100 ml of water, and the pH of the solution was adjusted to pH 3 with citric acid. Thereafter, the resulting mixture was treated at 100°C for 3 hours, to give a product decomposed with the acid of the fucoidan. This hydrolysate was subjected to molecular weight fractionation by gel filtration on Cellulofine GCL-300 or Cellulofine GCL-25, to fractionate the hydrolysate into molecular fractions exceeding 25000 (Fraction A); exceeding 10000 to 25000 (Fraction B); exceeding 5000 to 10000 (Fraction C); exceeding 2000 to 5000 (Fraction D); exceeding 500 to 2000 (Fraction E); and 500 or less (Fraction F). Further, each of these fractions and the product decomposed with the acid were desalted, and then lyophilized, to give the product decomposed with the acid and each fraction of the product decomposed with the acid.

### Reference Example 9

Five kilograms of a commercially available, salt-preserved *Nemacystus decipiens* was mixed with 20 liters of ethanol, and cut into thin pieces with scissors. The resulting mixture was allowed to stand overnight, and then filtered with a filter paper. The resulting residue was suspended in 12.5 liters of water, and treated at 95°C for 2 hours. After the treated solution was filtered with a filter paper, 2600 ml of a 2.5% cetyl pyridinium chloride solution containing 350 mM sodium chloride was added thereto, and the resulting mixture was allowed to stand for 3 days. The supernatant portion was discarded, the precipitate portion was centrifuged, and the resulting supernatant was also discarded. To the precipitates obtained was added 2.5 liters of 350 mM sodium chloride, and thereafter the mixture was homogenized with a homogenizer and centrifuged. The washing steps were repeated 3 times. Four-hundred milliliters of 400 mM sodium chloride was added to the precipitates obtained. Thereafter, the mixture was homogenized with a homogenizer, and ethanol was added thereto so as to give a concentration of 80%. The mixture was stirred for 30 minutes, and then filtered with a filter paper. Five hundred milliliters of 80% ethanol saturated with sodium chloride was added to the residue obtained, and thereafter the mixture was homogenized with a homogenizer. Ethanol saturated with sodium chloride was added to make the total amount 1 liter, and the mixture was stirred for 30 minutes and then filtered with a filter paper. The washing steps were repeated until the absorbance at 260 nm of the filtrate became 0 (zero) (usually 5 times). The residue obtained was dissolved in 1.5 liters of 2 M sodium chloride, and thereafter insoluble matters were removed by centrifugation. The resulting solution was allowed to flow through a column containing 100 ml of a DEAE-Cellulofine A-800 previously equilibrated with 2 M sodium chloride. Effluent fractions were concentrated to a volume of 2 liters with an ultrafilter equipped with holofiber having an excluding molecular weight of 100000, and thereafter the solvent was substituted with 2 mM sodium chloride by an ultrafilter. The resulting solution was centrifuged, and the resulting supernatant was lyophilized, to give 22.9 g of fucoidan derived from *Nemacystus decipiens.*

### Reference Example 10

(1) Fifty grams of a dried *Gelidium amansii* was cut into thin pieces with scissors, and suspended in 500 ml of 80% ethanol. Thereafter, the resulting suspension was stirred at 25°C for 3 hours, and filtered with a filter paper. The resulting residue was suspended in 1 liter of a 30 mM sodium phosphate buffer (pH 6.5) containing 100 mM sodium chloride, treated at 95°C for 2 hours, and thereafter filtered with a stainless screen having a hole diameter of 106 µm. The above-mentioned sodium phosphate buffer was added to the filtrate obtained to make the total amount 3 liters. Five grams of activated carbon was added thereto, and the resulting mixture was stirred at 25°C overnight, and then centrifuged. The resulting supernatant was concentrated to a volume of 200 ml with an ultrafilter equipped with holofiber having an excluding molecular weight of 100000, and thereafter subjected to solvent-exchange with an ultrafilter to give a 10 mM sodium chloride solution. Insoluble matters in the solution were removed by centrifugation, and thereafter the resulting solution was lyophilized, to give 2.3 g of a dried product of a sulfated polysaccharide fraction derived from *Gelidium amansii.*

(2) According to the method described in item (1) of Reference Example 10, 4.4 g of a sulfated polysaccharide derived from *Gracilaria verrucosa* was prepared from 50 g of dried *Gracilaria verrucosa.* Similarly, 1.0 g of a sulfated polysaccharide derived from *Pterocladiella* was also prepared from a dried *Pterocladiella Capillacea.*

(3) - [1] One kilogram of a commercially available powder of dried *Lessonia nigrescence* was suspended in 10 liters of 80% ethanol, and thereafter the resulting suspension was stirred at 25°C for 3 hours and filtered with a filter paper. The resulting residue was suspended in 20 liters of a 30 mM sodium phosphate buffer (pH 6.5) containing 100 mM sodium chloride, and the resulting suspension was treated at 95°C for 2 hours, and thereafter filtered with a stainless screen having a hole diameter of 106 µm. To the resulting filtrate were added 100 g of activated carbon, 2.4 liters of ethanol and 6000 U of alginic acid lyase K, and the resulting mixture was stirred at 25°C for 22 hours and then centrifuged. The resulting supernatant was concentrated to a volume of 1.2 liters with an ultrafilter equipped with holofiber having an excluding molecular weight of 100000, and thereafter insoluble matters were removed by centrifugation. The resulting solution was allowed to stand at 5°C for 24 hours. Formed precipitates were removed by centrifugation, and the resulting supernatant was subjected to solvent-exchange with an ultrafilter to give a 100 mM sodium chloride solution. After the solution was cooled to 4°C or lower, the pH was adjusted to 2.0 with hydrochloric acid, and the formed precipitates were removed by centrifugation. The pH of the resulting supernatant was adjusted to 8.0 with sodium hydroxide, and the resulting solution was concentrated to a volume of 2 liters. Thereafter, the solvent was exchanged for 20 mM sodium chloride by using an ultrafilter. Insoluble matters in the resulting solution were removed by centrifugation, and thereafter the resulting product was lyophilized, to give 41 g of a dried product of fucoidan derived from *Lessonia.*

(3) - [2] Six grams of the above-mentioned lyophilized product was dissolved in 600 ml of a 20 mM imidazole-hydrochloric acid buffer (pH 6) containing 100 mM sodium chloride, and the resulting solution was applied onto a column containing 5 liters of DEAB-Cellulofine A-800 previously equilibrated with the same buffer. After washing was carried out with 10 liters of the same buffer, the elution was carried out on a concentration gradient of from 100 to 1600 mM sodium chloride. The amount of the solution used for the elution was 13 liters, and the fraction was collected at 500 ml per fraction. Of the eluted fractions, 500 ml each of the eluted fractions obtained at around 250 mM, around 530 mM and around 700 mM sodium chloride concentration was dialyzed against purified water, and lyophilized. The lyophilized products were named DEAE Fraction 33, DEAE Fraction 37 and DEAE Fraction 40, respectively, and obtained in the amounts of 57 mg, 24 mg and 62 mg, respectively.

### Reference Example 11

Five kilograms of sea cucumbers were dissected, and the organs were removed to collect somatic layers. Five-hundred milliliters of acetone was added per 200 g of the wet weight of the somatic layers, and the mixture was treated with a homogenizer. Thereafter, the homogenate was filtered, and the residue was washed with acetone until no more colored substances remained. This residue was dried with suction, to give 140 g of a dried product. To this dried product was added 2.8 liters of a 0.4 M saline, and the mixture was treated at 100°C for 1 hour. Thereafter, the mixture was filtered, and the resulting residue was sufficiently washed with a 0.4 M saline, to give 3.7 liters of an extract. To this extract was added 5% cetyl pyridinium chloride until no more precipitates were formed, and the formed precipitates were harvested by centrifugation. The precipitates were suspended in a 0.4 M saline, and again centrifuged. One liter of a 4 M saline was added to the resulting precipitates, and the mixture was treated with a homogenizer. Thereafter, 4 liters of ethanol was added thereto with stirring, and the resulting mixture was stirred for 1 hour, and thereafter filtered, to give precipitates. The steps of suspending the precipitates in 80% ethanol and thereafter filtering the suspension were repeated until the absorbance at 260 nm of the supernatant became 0. The precipitates obtained were suspended in 2 liters of a 2M saline, and insoluble matters were removed by centrifugation. The supernatant was ultrafiltered with an ultrafilter equipped with a membrane having an excluding molecular weight of 30000, and completely desalted. Thereafter, the resulting product was lyophilized, to give 3.7 g of fucoidan derived from sea cucumbers.

### Reference Example 12

Five hundred milligrams of agar powder (manufactured by nakalaitesque) was suspended in 100 ml of distilled water, and then heated to dissolve the agar. Thereafter, the resulting solution was cooled to 45°C, and kept at 45°C.

To this agar solution was added 2 ml of an X50 β-Agarase buffer (manufactured by FMC, packed with β-Agarase), and 100 µm of 1 U/µl β-Agarase (manufactured by FMC) was added. This solution was kept at 45°C for 24 hours, and thereafter a 2.5-fold amount of ethanol was added thereto. The resulting mixture was cooled, and then centrifuged to collect precipitates. The precipitates were dried, and dissolved in 20 ml of distilled water. This solution was lyophilized, to give a powdery agaropectin fraction.

### Reference Example 13

(1) The steps of suspending 10 g of dried cells of *Spirulina platensis* in 100 ml of chloroform and filtering the suspension to collect an insoluble fraction were repeated 5 times. Thereafter, the steps of suspending the insoluble fraction in 100 ml of ethanol and filtering the suspension to collect an insoluble fraction were repeated 3 times. Ethanol was completely removed from the insoluble fraction obtained by the steps, and the resulting product was suspended in 100 ml of distilled water. This suspension was kept at 60°C for 1 hour and then centrifuged, to give supernatant. This supernatant was further filtered, and a 2.5-fold amount of ethanol was added to the filtrate. The resulting solution was cooled to -20°C and then centrifuged at a low temperature, to give precipitates. The precipitates were dissolved in distilled water and lyophilized, to give a powdery fraction containing a sulfated polysaccharide derived from *Spirulina.*

(2) Twenty grams of a dried powder of *Spirulina* (commercially available form K.K. Spirulina Kenkyusho) was placed in a homogenizer (manufactured by NIPPON SEIKI CO., LTD.), and 400 ml of acetone was added thereto. The resulting mixture was homogenized at 8000 rpm for 10 minutes, and the homogenate was filtered with a filter paper, to give a residue. Washing the residue with acetone was repeated 3 times in the same manner as that in the above-mentioned steps, to give an acetone-washed residue. The acetone-washed residue was washed 4 times with 90% ethanol, and 4 times with 80% ethanol in the same manner as that in the acetone-washing, to give an ethanol-washed residue.

Six-hundred milliliters of a 30 mM phosphate buffer (pH 7.0) containing 100 mM sodium chloride and 10% ethanol was added to the ethanol-washed residue, and the mixture was stirred at room temperature for 18 hours. This mixture was centrifuged at 10000 rpm for 40 minutes, to give supernatant. Insoluble matters admixed in the supernatant were filtered out with a filter paper, to give a crude extract (filtrate). The crude extract obtained was concentrated to a volume of 300 ml with an ultrafilter equipped with holofiber having an excluding molecular weight of 10000, and thereafter the concentrate was ultrafiltered with 2 liters of 100 mM sodium chloride containing 10% ethanol added. Thereafter, the solvent was substituted with a 10 mM imidazole-hydrochloric acid buffer (pH 7.0) containing 10% ethanol and 50 mM sodium chloride, to give 240 ml of a *Spirulina* macromolecular fraction.

The *Spirulina* macromolecular fraction was applied onto a DEAE-Cellulofine A-800 column (Φ 3 x 14.2 cm) equilibrated with a 10 mM imidazole-hydrochloric acid buffer (pH 7.0) containing 10% ethanol and *50* mM sodium chloride, and the column was washed with 360 ml of the same buffer. Thereafter, the elution was carried out on a gradient of from 0.05 M (200 ml) to 2 M (200 ml) sodium chloride. The eluate was fractionated at 10 ml per fraction. Of the eluted fractions, Fraction Nos. 14 to 30 were named *Spirulina* Sulfated Polysaccharide Fraction - I (SSP-I), Fraction Nos. 69 to 77 *Spirulina* Sulfated Polysaccharide Fraction - II (SSP-II), Fraction Nos. 78 to 83 *Spirulina* Sulfated Polysaccharide Fraction - III (SSP-III), and Fraction Nos. 84 to 99 *Spirulina* Sulfated Polysaccharide Fraction - IV (SSP-IV), respectively. SSP-I, SSP-II, SSP-III and SSP-IV were sufficiently dialyzed against distilled water and lyophilized, to give amounts of 200 mg, 260 mg, 100 mg and 60 mg, respectively.

(3) The steps of suspending 10 g of dried cells of *Chlorella vulgaris* in 100 ml of chloroform and filtering the suspension to collect an insoluble fraction were repeated 3 times. Thereafter, the steps of suspending the insoluble fraction in 100 ml of ethanol and filtering the suspension to collect an insoluble fraction were repeated 3 times. Ethanol was completely removed from the insoluble fraction obtained by the steps, and the resulting product was suspended in 100 ml of distilled water. This suspension was kept at 60°C for 1 hour and then filtered. A 2.5-fold amount of ethanol was added to the filtrate, and cooled to -20°C. Thereafter, the solution was centrifuged at a low temperature, to give precipitates. The precipitates were dissolved in distilled water and lyophilized, to give a powdery fraction containing a sulfated polysaccharide derived from *Chlorella.*

(4) Twenty grams of dried powder of *Chlorella* (sold by K.K. Chlorella Center) was placed in a homogenizer (manufactured by NIPPON SEIKI CO., LTD.), and 400 ml of acetone was added thereto. The resulting mixture was homogenized at 8000 rpm for 10 minutes, and the homogenate was filtered with a filter paper, to give a residue. Washing the residue with acetone was repeated 3 times in the same manner as that in the above-mentioned steps, to give an acetone-washed residue. The acetone-washed residue was washed 4 times with 90% ethanol, and 4 times with 80% ethanol, in the same manner as that in the acetone-washing, to give an ethanol-washed residue.

Six-hundred milliliters of a 30 mM phosphate buffer (pH 7.0) containing 100 mM sodium chloride and 10% ethanol was added to the ethanol-washed residue, and the resulting mixture was stirred at room temperature for 18 hours. This mixture was centrifuged at 10000 rpm for 40 minutes, to give supernatant. Insoluble matters admixed in the supernatant were filtered out with a filter paper, to give a crude extract (filtrate). The crude extract obtained was concentrated to a volume of 310 ml with an ultrafilter equipped with holofiber having an excluding molecular weight of 10000. Thereafter, the concentrate was ultrafiltered with 3 liters of 100 mM sodium chloride containing 10% ethanol added. Thereafter, the solvent was substituted with a 10 mM imidazole-hydrochloric acid buffer (pH 7.0) containing 10% ethanol and 50 mM sodium chloride, to give 203 ml of a *Chlorella* macromolecular fraction.

The *Chlorella* macromolecular fraction was applied onto a DEAE-Cellulofine A-800 column (Φ 3 x 14.2 cm) equilibrated with a 10 mM imidazole-hydrochloric acid buffer (pH 7.0) containing 10% ethanol and 50 mM sodium chloride, and the column was washed with 297 ml of the same buffer. Thereafter, the elution was carried out on a gradient of from 0.05 M (200 ml) to 2 M (200 ml) sodium chloride. The eluate was fractionated at 10 ml per fraction. Of the eluted fractions, Fraction Nos. 63 to 68 were named *Chlorella* Sulfated Polysaccharide Fraction I (CSP-I), and Fraction Nos. 69 to 75 were named *Chlorella* Sulfated Polysaccharide Fraction II (CSP-II). CSP-I and CSP-II were sufficiently dialyzed against distilled water and lyophilized, to give amounts of 140 mg and 200 mg, respectively.

(5) The steps of suspending 10 g of a powder of *Altemisia princeps pampan*, which was prepared by pulverizing commercially available *Altemisia princeps pampan* (manufactured by Sakamoto Kanpodo), in 100 ml of chloroform and filtering the suspension to collect an insoluble fraction were repeated 3 times. Thereafter, the steps of suspending the insoluble fraction in 100 ml of ethanol and filtering the suspension to collect an insoluble fraction were repeated 5 times. Ethanol was completely removed from the insoluble fraction obtained by the steps, and the resulting product was suspended in 100 ml of distilled water. This suspension was kept at 60°C for 1 hour and then filtered. A 2.5-fold amount of ethanol was added to the filtrate, and the resulting solution was cooled to -20°C. Thereafter, the mixture was centrifuged at a low temperature, to give precipitates and a supernatant fraction of *Altemisia princeps* *pampan.* The precipitates were dissolved in distilled water and lyophilized, to give a powedery fraction containing a sulfated polysaccharide derived from *Altemisia princeps pampan.*

(6) Fifty grams of dried leaves of *Altemisia princeps pampan* (sold by Sakamoto Kanpodo) were placed in a homogenizer (manufactured by NIPPON SEIKI CO., LTD.), and 500 ml of acetone was added thereto. The resulting mixture was homogenized at 8000 rpm for 10 minutes. The homogenate was filtered with a filter paper, to give a residue. The above-described steps were carried out twice, and 100 g of the resulting residue of the leaves of *Altemisia princeps pampan* was placed in a homogenizer, and 500 ml of acetone was added thereto. The resulting mixture was homogenized at 8000 rpm for 10 minutes. The homogenate was filtered with a filter paper, to give a residue. The steps were repeated 4 times, to give an acetone-washed residue. The acetone-washed residue was washed 4 times with 90% ethanol, and 4 times with 80% ethanol, in the same manner as that in the acetone-washing, to give an ethanol-washed residue.

Five liters of a 30 mM phosphate buffer (pH 8.0) containing 100 mM sodium chloride and 10% ethanol was added to the ethanol-washed residue, and the mixture was stirred at room temperature for 19 hours. This mixture was filtered with a filter paper, to give a crude extract (filtrate). The crude extract obtained was concentrated to a volume of 2 liters with an ultrafilter equipped with holofiber having an excluding molecular weight of 10000. Thereafter, the concentrate was ultrafiltered with 10 liters of 100 mM sodium chloride containing 10% ethanol added. Subsequently, the filtered solution was concentrated to a volume of 500 ml, and the solvent was substituted with a 10 mM imidazole-hydrochloric acid buffer (pH 7.0) containing 10% ethanol and 50 mM sodium chloride. This solution was transferred to a beaker, and 1 g of activated carbon was placed therein. The resulting mixture was stirred at room temperature for 40 minutes, and then centrifuged at 10000 rpm for 40 minutes. The activated carbon admixed in the supernatant was filtered out with a filter paper. As described above, 560 ml of a macromolecular fraction of leaves of *Altemisia princeps pampan* was obtained.

The macromolecular fraction of leaves of *Altemisia princeps pampan* was applied onto a DEAE-Cellulofine A-800 column (φ 3.5 x 31 cm) equilibrated with a 10 mM imidazole-hydrochloric acid buffer (pH 7.0) containing 10% ethanol and 50 mM sodium chloride, and the column was washed with 940 ml of the same buffer. Thereafter, the elution was carried out on a gradient of from 0.05 M (600 ml) to 2 M (600 ml) sodium chloride. The eluate was fractionated at 10 ml per fraction. Of the eluted fractions, Fraction Nos. 180 to 202 were named *Altemisia princeps pampan* leaf Acidic Polysaccharide Fraction (YAP), and Fraction Nos. 203 to 270 were named *Altemisia princeps pampan* leaf Sulfated Polysaccharide Fraction (YSP). YAP was sufficiently dialyzed against distilled water and lyophilized, to give an amount of 250 mg.

In order to further fractionate the *Altemisia princeps pampan* sulfated polysaccharide fraction, the *Altemisia princeps pampan* leaf sulfated polysaccharide fraction was dialyzed against 3 liters of a 10 mM imidazole-hydrochloric acid buffer (pH 7.0) containing 10% ethanol and 100 mM sodium chloride. The dialyzed sulfated polysaccharide fraction (327 ml) was applied onto a DEAE-Cellulofine A-800 column (Φ 3 cm x 14.2 cm) equilibrated with the same buffer. The column was washed with 273 ml of the buffer, and thereafter the elution was carried out on a gradient of from 0.1 M (200 ml) to 2 M (200 ml) sodium chloride. The eluate was fractionated at 5 ml per fraction. Of the eluted fractions, Fraction Nos. 140 to 154 were named *Altemisia princeps pampan* leaf Sulfated Polysaccharide Fraction - I (YSP-I), and Fraction Nos. 155 to 200 were named *Altemisia princeps pampan* leaf Sulfated Polysaccharide Fraction - II (YSP-II). YSP-I and YSP-II were sufficiently dialyzed against distilled water and lyophilized, to give amounts of 20 mg and 130 mg, respectively.

A 10 mM imidazole-hydrochloric acid buffer (pH 7.0) containing 59.7 ml of 10% ethanol and 0.2 M sodium chloride was added to YSP-II (119.4 mg) and stirred at room temperature overnight to dissolve YSP-II. The dissolved YSP-II was applied onto a DEAE-Cellulofine A-800 column (φ 2.5 x 10.2 cm) equilibrated with the same buffer, and the column was washed with 200 ml of the buffer. Thereafter, the elution was carried out on a gradient of from 0.2 M (100 ml) to 1 M (100 ml) sodium chloride. The eluate was fractionated at 5 ml per fraction. Of the eluted fractions, Fraction Nos. 54 to 70 were named *Altemisia princeps pampan* leaf Sulfated Polysaccharide Fraction - II-2 (YSP-II-2), Fraction Nos. 71 to 90 were named *Altemisia princeps pampan* leaf Sulfated Polysaccharide Fraction - II-3 (YSP-II-3), and Fraction Nos. 91 to 120 were named *Altemisia princeps pampan* leaf Sulfated Polysaccharide Fraction - II-4 (YSP-II-4). YSP-II-2, YSP-II-3, and YSP-II-4 were sufficiently dialyzed against distilled water and lyophilized, to give amounts of 39.5 mg, 61 mg and 57.3 mg, respectively.

(7) A powdered product prepared by pulverizing commercially available, edible *Momordica charantia* with a mixer was lyophilized, to give a dried product of *Momordica charantia*. The steps of suspending 10 g of the dried product of *Momordica charantia* in 100 ml of chloroform and filtering the suspension to collect an insoluble fraction were repeated 5 times. Thereafter, the steps of suspending the insoluble fraction in 100 ml of ethanol and filtering the suspension to collect an insoluble fraction were repeated 3 times. Ethanol was completely removed from the insoluble fraction obtained by the steps, and the resulting product was suspended in 100 ml of distilled water. This suspension was kept at 60°C for 1 hour and then filtered. A 2.5-fold amount of ethanol was added to the filtrate, and the resulting solution was cooled to -20°C. Thereafter, the solution was centrifuged at a low temperature, to give precipitates. The precipitates were dissolved in distilled water and lyophilized, to give a powdery fraction containing a sulfated polysaccharide.

(8) Transparent mesophyll portions were collected from 5 leaves of commercially available *Aloe arborescens Mill. var. natalensis,* and lyophilized. The amount 0.481 g of this lyophilized product of mesophyll of *Aloe arborescens Mill. var. natalensis* was suspended in 100 ml of distilled water. This suspension was kept at 60°C for 1 hour and then filtered. A 2.5-fold amount of ethanol was added to the filtrate, and the resulting solution was cooled to -20°C. Thereafter, the solution was centrifuged at a low temperature, to give precipitates. The precipitates were dissolved in distilled water and lyophilized, to give a powdery fraction containing a sulfated polysaccharide from the mesophyll of *Aloe arborescens Mill. var. natalensis.*

On the other hand, the remaining green surface portions of the leaves from which the transparent mesophyll portions were collected in the manner as described above were powdered, and lyophilized. The steps of suspending 3.43 g of the lyophilized product in 100 ml of chloroform and filtering the suspension to collect an insoluble fraction were repeated 3 times. Thereafter, the steps of suspending the insoluble fraction in 100 ml of ethanol and filtering the suspension to collect an insoluble fraction were repeated 3 times. Ethanol was completely removed from the insoluble fractions obtained by the steps, and the resulting product was suspended in 100 ml of distilled water. This suspension was kept at 60°C for 1 hour and then filtered. A 2.5-fold amount of ethanol was added to the filtrate, and the resulting solution was cooled to -20°C. Thereafter, the solution was centrifuged at a low temperature, to give precipitates. The precipitates were dissolved in distilled water and lyophilized, to give a powdery fraction containing a sulfated polysaccharide from a product of the green surface portions of the leaves of *Aloe arborescens Mill*. *var. natalensis.*

### Reference Example 14

(1) Two-hundred milligrams (1.1 mmol) of D-(+)-glucose was dissolved in 10 ml of pyridine, and 1.05 g (6.6 mmol) of Pyridine Sulfur Trioxide Complex (Pyr•SO₃, Tokyo Kasei) was added thereto at room temperature. Thereafter, the resulting mixture was stirred at room temperature for several minutes and at 60°C for 1 hour, and the reaction solution was diluted with water. The pH of the solution was adjusted near neutral with an aqueous saturated barium hydroxide solution, and the resulting solution was then dried under reduced pressure. Water was again added to the resulting concentrate, and the resulting solution was again dried under reduced pressure. These steps were repeated one more time. A small amount of water was added to the resulting concentrate, and precipitates of barium hydroxide were removed by centrifugation. The resulting supernatant was applied onto a cation exchange column [Amberlite IRA-120 (Na⁺)(Organo)]. The resulting column-effluent fractions were concentrated under reduced pressure, to give 700 mg of sodium salt of sulfated D-(+)-glucose.
(2) Two-hundred and forty milligrams (1.3 mmol) of D-(+)-galactose was dissolved in 10 ml of pyridine, and 1.05 g (6.6 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 406 mg of sodium salt of sulfated D-(+)-galactose.
(3) Two-hundred milligrams (1.3 mmol) of D-(+)-mannose was dissolved in 10 ml of pyridine, and 1.05 g (6.6 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 700 mg of sodium salt of sulfated D-(+)- mannose.
(4) Two-hundred and five milligrams (0.57 mmol) of maltose was dissolved in 10 ml of pyridine, and 816 mg (5.2 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 520 mg of sodium salt of sulfated maltose.
(5) Two-hundred milligrams (0.4 mmol) of maltotriose was dissolved in 10 ml of pyridine, and 700 mg (4.4 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 420 mg of sodium salt of sulfated maltotriose.
(6) Two-hundred and fifty milligrams (0.73 mmol) of trehalose was dissolved in 10 ml of pyridine, and 1.1 g (7 mmol) of Pyr·SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 750 mg of sodium salt of sulfated trehalose.
(7) Two-hundred and twenty two milligrams (0.62 mmol) of lactose was dissolved in 10 ml of pyridine, and 785 mg (4.9 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 476 mg of sodium salt of sulfated lactose.
(8) Two-hundred and twenty milligrams (0.62 mmol) of sucrose was dissolved in 10 ml of pyridine, and 785 mg (4.9 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 481 mg of sodium salt of sulfated sucrose.
(9) Three-hundred and seventy milligrams (1.08 mmol) of lactulose was dissolved in 10 ml of pyridine, and 1.38 g (8.8 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 1 g of sodium salt of sulfated lactulose.
(10) Three-hundred and seventy nine milligrams (0.9 mmol) of melibiose was dissolved in 10 ml of pyridine, and 1.43 g (9.0 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 950 mg of sodium salt of sulfated melibiose.
(11) One-hundred and fifty milligrams (1.0 mmol) of D-(+)-xylose was dissolved in 10 ml of pyridine, and 770 mg (4.8 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 350 mg of sodium salt of sulfated D-(+)- xylose.
(12) Two-hundred milligrams (1.2 mmol) of 2-deoxy-glucose was dissolved in 10 ml of pyridine, and 920 mg (5.8 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 500 mg of sodium salt of sulfated 2-deoxy-glucose.
(13) One-hundred and fifty milligrams (0.83 mmol) of D-glucitol was dissolved in 10 ml of pyridine, and 955 mg (6 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 570 mg of sodium salt of sulfated D-glucitol.
(14) One-hundred and forty-seven milligrams (0.43 mmol) of cellobiose was dissolved in 5 ml of dimethyl sulfoxide, and 657 mg (4.13 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 230 mg of sodium salt of sulfated cellobiose.
(15) Sixty-two milligrams (0.18 mmol) of isomaltose was dissolved in 5 ml of pyridine, and 275 mg (1.73 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 162 mg of sodium salt of sulfated isomaltose.
(16) Two-hundred and ninety-three milligrams (0.86 mmol) of turanose was dissolved in 5 ml of pyridine, and 1310 mg (8.22 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 835 mg of sodium salt of sulfated turanose.
(17) Three-hundred and fifteen milligrams (0.875 mmol) of palatinose was dissolved in 5 ml of pyridine, and 1.34 mg (8.4 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 845 mg of sodium salt of sulfated palatinose.
(18) Fifty-six milligrams (0.31 mmol) of α-D-talose was dissolved in 5 ml of pyridine, and 300 mg (1.9 mmol) of Pyr•SO₃ was added thereto at room temperature. Thereafter, the resulting mixture was stirred at room temperature for several minutes and at 60°C for 1 hour. The subsequent steps were carried out in the same manner as those of item (1) of Reference Example 14, to give 150 mg of sodium salt of sulfated D-talose.
(19) A completely acetylated product of 7 g of α-cyclodextrin was treated with a mixed solution of acetic acid anhydride and sulfuric acid (49:1), to give completely acetylated maltohexaose, which was then deacetylated with sodium methoxide (NaOMe) in methanol, to give 1.5 g of maltohexaose. Seventy-nine milligrams (0.83 mmol) of maltohexaose and 1.33 g of piperidinesulfuric acid were dissolved in 5 ml of dimethyl sulfoxide (DMSO), and the resulting mixture was stirred at 80°C for 2 hours. The reaction solution was cooled, and thereafter dialyzed for 2 days using a dialysis membrane having an excluding molecular weight of 1000. The resulting solution inside the dialysis membrane was applied onto a cation exchange column [Amberlite IRA-120 (Na⁺) (Organo)]. The resulting column-effluent fractions were concentrated under reduced pressure, to give 167 mg of sodium salt of sulfated maltohexaose.
(20) A completely acetylated product of 2.2 g of β-cyclodextrin was treated with a mixed solution of acetic acid anhydride and sulfuric acid (49:1), to give a completely acetylated maltoheptaose, which was deacetylated with NaOMe in methanol, to give 0.5 g of maltoheptaose. Twenty milligrams (0.83 mmol) of maltoheptaose and 325 mg of piperidinesulfuric acid were dissolved in 5 ml of DMSO, and the mixture was stirred at 80°C for 2 hours. Thereafter, the subsequent steps were carried out in the same manner as those of item (19) of Reference Example 14, to give 45.6 mg of sodium salt of sulfated maltoheptaose.
(21) Completely acetylated maltohexaose was stirred in dichloromethane in the presence of trichloroacetonitrile and potassium carbonate, to give an imidate of acetylated maltohexaose. The imidate of acetylated maltohexaose was reacted with dodecanol in dichloromethane using trimethylsilyl trifluoromethanesulfonate as a catalyst, and the reaction product obtained was deacetylated, to give dodecyl-maltohexaose. Three-hundred and seventy milligrams (0.32 mmol) of dodecyl-maltohexaose was dissolved in 10 ml of DMSO, and the mixture was stirred at 80°C for 2 hours. The subsequent steps were carried out in the same manner as those of item (19) of Reference Example 14, to give 700 mg of sodium salt of sulfated dodecyl-maltohexaose.
(22) Two-hundred and seventy-six milligrams of starch was dissolved in 10 ml of DMSO, and 2.76 g of Pyr•SO₃ was added thereto at room temperature. Thereafter, the mixture was stirred at 80°C for 2 hours. The reaction solution was cooled, and thereafter acetone was added thereto. The formed insoluble fraction was washed several times with methanol, and then diluted with water. The dilution was applied onto a cation exchange column [Amberlite IRA-120 (Na⁺) (Organo)]. The resulting column-effluent fractions were concentrated under reduced pressure, to give 350 mg of sodium salt of sulfated starch.
(23) One-hundred and eleven milligrams of cardran was dissolved in 5 ml of DMSO, and 1.11 g of Pyr•SO₃ was added thereto at room temperature. Thereafter, the mixture was stirred at 80°C for 2 hours. The reaction solution was cooled, and thereafter acetone was added thereto. The formed insoluble fraction was diluted with water, and the pH was adjusted to near neutrality with an aqueous saturated sodium bicarbonate. Thereafter, the resulting solution was dialyzed for 1 day using a dialysis membrane having an excluding molecular weight of 1000. The resulting solution inside the dialysis membrane was applied onto a cation exchange column [Amberlite IRA-120 (Na⁺)(Organo)]. The resulting fractions were dried under reduced pressure, to give 180 mg of sodium salt of sulfated cardran.
(24) Two-hundred and sixty-seven milligrams of pectin was dissolved in 5 ml of DMSO, and 2.67 g of Pyr•SO₃ was added thereto at room temperature. Thereafter, the resulting mixture was stirred at 80°C for 2 hours, and the reaction solution was cooled. The subsequent steps were carried out in the same manner as those of item (23) of Reference Example 14, to give 384 mg of sodium salt of sulfated pectin.

### Example 1

(1) Five-hundred microliters each of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, the fucoidan derived from *Kjellmaniella crassifolia* described in item (1) of Reference Example 1 was added as a sample so as to have a final concentration of 1, 10 or 100 µg/ml, and the cells were further cultured for 24 hours. The medium was then collected, and by using Quantikine Human Hepatocyte Growth Factor (HGF) ELISA Kit (manufactured by Funakoshi, Code. RS-0641-00), the amount of HGF in the medium was determined.

As a control, distilled water was added in the same volume as that of the sample. The amount of HGF of the control was 7.2 ng/ml. The amount of HGF produced in each sample-added group is shown in Table 1, assuming that the value of the control is 100%. Each experiment was carried out twice, and its average value was taken.

**Table 1**

| Fucoidan Derived from *Kjellmaniella crassifolia* (µg/ml) | Amount of HGF Produced (%) |
|---|---|
| 0 | 100 |
| 1 | 214 |
| 10 | 339 |
| 100 | 339 |

In the group where the fucoidan derived from *Kjellmaniella crassifolia* was added, the amount of HGF produced was significantly increased as compared with that of control with addition of distilled water. In addition, since the amount of HGF produced is remarkably increased, as compared to the case where heparin or low-molecular heparin is added, it was shown that the fucoidan derived from *Kjellmaniella crassifolia* possesses higher activity for promoting HGF production as compared to that of heparin or a low-molecular heparin having an average molecular weight of about 5000 in which induction of HGF production has been confirmed.

(2) Each of induction action for HGF production of Fraction I, Fraction II and Fraction III prepared by the method described in item (2) of Reference Example 1; 7-12SFd-F prepared by the method of Reference Example 2; 6-2S prepared by the method of Reference Example 4; the fucoidan derived from sporophyll of *Undaria pinnatifida* prepared by the method of Reference Example 6; and the fucoidan derived from *Fucus vesiculosus* prepared by the method of Reference Example 7 was determined under the same conditions as those in item (1) of Example 1. The results are shown in Tables 2 to 4.

**Table 2**

| Sample | Concentration (µg/ml) | Amount of HGF Produced (%) |
|---|---|---|
| Fraction I | 1 | 167 |
| | 100 | 234 |
| Fraction II | 1 | 208 |
| | 100 | 359 |
| Fraction III | 1 | 146 |
| | 100 | 291 |
| (The amount of HGF produced of control was 8.3 ng/ml.) | | |

**Table 3**

| Sample | Concentration (µg/ml) | Amount of HGF Produced (%) |
|---|---|---|
| Fucoidan Derived from | 1 | 148 |
| *Fucus vesiculosus* | 10 | 246 |
| | 100 | 335 |
| Fucoidan Derived from | 1 | 179 |
| sporophyll of *Undaria* | 10 | 250 |
| *pinnatifida* | 100 | 291 |
| 7-12S-Fd-F | 1 | 149 |
| | 10 | 276 |
| | 100 | 339 |
| (The amount of HGF produced of control was 8.6 ng/ml.) | | |

**Table 4**

| Sample | Concentration (µg/ml) | Amount of HGF Produced (%) |
|---|---|---|
| 6-2S | 10 | 112 |
| | 100 | 246 |
| (The amount of HGF produced of control was 9.9 ng/ml.) | | |

Each of fractions of the fucoidan derived from *Kjellmaniella crassifolia*, namely U-fucoidan, F-fucoidan, the fucoidan derived from *Fucus vesiculosus*, fucoidan derived from sporophyll of *Undaria pinnatifida*, 7-12SFd-F derived from F-fucoidan, and 6-2S derived from U-fucoidan was found to have strong induction action for HGF production. In addition, each of the fucoidan derived from *Laminaria japonica* and the fucoidan derived from *Lessonia nigrescence* described in item (1) of Reference Example 1, the fucoidan derived from *Ascophyllum nodosum* described in Reference Example 7, the product decomposed with the acid described in Reference Example 8, and Fractions A to F was also found to have strong induction action for HGF production.

(3) - [1] A 2% solution of the fucoidan derived from *Kjellmaniella crassifolia* prepared by the method described in item (1) of Reference Example 1 was adjusted to pH 3 with citric acid or sulfuric acid, and each solution was heated at 100°C for 30 minutes, 1 hour, 2 hours or 4 hours, to prepare a hydrolyzed solution thereof. Its induction action for HGF production was determined under the same conditions as those in item (1) of Example 1. As the sample, a 10-fold dilution of the solution decomposed with the acid was used.

**Table 5**

| Sample | Heating Time | Amount of HGF Produced (%) |
|---|---|---|
| Non-decomposed solution | | 448 |
| Solution decomposed | 30 minutes | 364 |
| with sulfuric acid | 1 hour | 385 |
| | 2 hours | 368 |
| | 4 hours | 345 |
| Non-decomposed solution | | 480 |
| Solution decomposed | 30 minutes | 402 |
| with citric acid | 1 hour | 429 |
| | 2 hours | 397 |
| | 4 hours | 341 |
| (The amount of HGF produced of control was 8.6 ng/ml.) | | |

(3) - [2] The 4-hour heat-treated product of the fucoidan derived from *Kjellmaniella crassifolia* prepared in (3) - [1] of Example 1 in the presence of citric acid was fractionated by gel filtration.

Specifically, a column filled with 1.5 liters of TOYOPEARL HW40C was equilibrated with water, and 10 ml of the heat-treated product of the fucoidan derived from *Kjellmaniella crassifolia* was applied onto this column, and thereafter eluted with water at a flow rate of 1 ml/minute. The initial 680 ml of the eluate was directly drained, and thereafter the eluate was fractionated into a volume of 14 ml each, to give gel filtration fractions of the heat-treated product.

The fractions were analyzed by TLC (solvent: butyl acetate : acetic acid : water = 3:4:3; a detecting agent: orcinol sulfate). Fractions 12-13, 16-17, 26-40, and the like were collected as gel filtration fractions on the basis of the spot patterns, and the fractions were lyophilized. The resulting lyophilized product of each fraction was re-dissolved in water so as to have a concentration of 100 mg/ml, and its induction action for HGF production was determined under the same conditions as those in item (1) of Example 1.

As a result, each of fractions of Fractions 12-13 and Fractions 16-17 was found to have induction activity for HGF production.

The fractions of Fractions 12-13 was subjected to structural determination. Their analytic values were identical with that of the compound represented by the following formula (VIII) described in WO 97/26896, and found to have induction activity for HGF production in a polymer obtained from glucuronic acid and mannose.

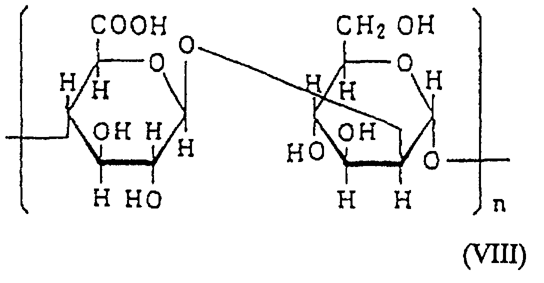

(4) A solution of commercially available sodium dextran sulfate (manufactured by Sigma) was prepared, and its induction action for HGF production was determined in accordance with the method described in item (1) of Example 1. As shown in Table 6, sodium dextran sulfate showed induction action for HGF production.

**Table 6**

| Sodium Dextran Sulfate (Average Molecular Weight) | Concentration (µg/ml) | Amount of HGF Produced (%) |
|---|---|---|
| 10000 | 1 | 588 |
| | 10 | 655 |
| | 100 | 787 |
| 8000 | 1 | 395 |
| | 10 | 573 |
| | 100 | 695 |
| 5000 | 1 | 398 |
| | 10 | 421 |
| | 100 | 565 |
| (The amount of HGF produced of control was 6.7 ng/ml.) | | |

(5) A solution of commercially available λ-carrageenan (manufactured by nacalaitesque) was prepared, and its induction action for HGF production was determined in accordance with the method described in item (1) of Example 1. As shown in Table 7, λ-carrageenan showed induction action for HGF production.

**Table 7**

| λ-Carrageenan (µg/ml) | Amount of HGF Produced (%) |
|---|---|
| 1 | 152 |
| 10 | 140 |
| (The amount of HGF produced of control was 13.4 ng/ml.) | |

(6) - [1] A solution of commercially available alginic acid (manufactured by Wako Pure Chemical Industries, Ltd.; swellable) was prepared, and its induction action for HGF production was determined in accordance with the method described in item (1) of Example 1. As shown in Table 8, alginic acid showed induction action for HGF production.

**Table 8**

| Alginic Acid (µg/ml) | Amount of HGF Produced (%) |
|---|---|
| 1 | 125 |
| 10 | 154 |
| 100 | 327 |
| (The amount of HGF produced of control was 7.3 ng/ml.) | |

(6) - [2] Similarly, the induction activity for HGF production for alginic acid (swellable, manufactured by Wako Pure Chemical Industries, Ltd.: Sample (1)), alginic acid (non-swellable, manufactured by Wako Pure Chemical Industries, Ltd.: Sample (2)), alginic acid (100 to 150 cp, manufactured by Wako Pure Chemical Industries, Ltd.: Sample (3)), alginic acid (300 to 400 cp, manufactured by Wako Pure Chemical Industries, Ltd.: Sample (4)), and alginic acid (500 to 600 cp, manufactured by Wako Pure Chemical Industries, Ltd.: Sample (5)) was studied. As shown in Table 9, all of Samples (1) to (5) induced the production of HGF. It was clarified from above that alginic acid, an acidic polysaccharide, also possesses induction activity for HGF production.

**Table 9**

| Concentration (µg/ml) | Amount of HGF Produced (%) | | | | |
|---|---|---|---|---|---|
| | Sample (1) | Sample (2) | Sample (3) | Sample (4) | Sample (5) |
| 10 | 154 | 138 | 115 | 127 | 104 |
| 100 | 327 | 158 | 184 | 152 | 187 |
| (The amount of HGF produced of control for Sample (1), (2) was 7.3 ng/ml; and the amount of HGF produced of control for Sample (3), (4), (5) was 6.7 ng/ml. | | | | | |

(6) - [3] Similarly, the induction activity for HGF production for pectic acid (manufactured by nacalaitesque) was studied. As shown in Table 10, pectic acid induced the production of HGF.

It was clarified from above that pectic acid, an acidic polysaccharide, also has induction activity for HGF production.

**Table 10**

| Sample | Concentration (µg/ml) | Amount of HGF Produced (%) |
|---|---|---|
| Pectic acid | 1 | 145 |
| | 10 | 218 |
| | 100 | 684 |
| (The amount of HGF produced of control was 5.91 ng/ml.) | | |

(7) The induction activity for HGF production for salmon sperm DNA (manufactured by K.K. Nichiro) was studied. The DNA was added so as to have a final concentration of 1, 10 or 100 µg/ml. As shown in Table 11, salmon sperm DNA showed induction activity for HGF production.

**Table 11**

| Sample | Concentration (µg/ml) | Amount of HGF Produced (%) |
|---|---|---|
| | 1 | 110 |
| Salmon Sperm DNA | 10 | 182 |
| | 100 | 285 |
| (The amount of HGF produced of control was 9.9 ng/ml.) | | |

(8) A solution of each of the fucoidan derived from *Nemacystus decipiens* and the fucoidan derived from sea cucumber prepared in Reference Examples 9 and 11 was prepared, and its induction action for HGF production was determined in accordance with the method described in item (1) of Example 1. As shown in Table 12, each fucoidan showed induction action for HGF production.

**Table 12**

| Sample | Concentration (µg/ml) | Amount of HGF Produced (%) |
|---|---|---|
| Fucoidan Derived from | 1 | 282 |
| Sea Cucumber | 10 | 356 |
| | 100 | 495 |
| Fucoidan Derived from | 1 | 218 |
| *Nemacystus decipiens* | 10 | 279 |
| | 100 | 307 |
| (The amount of HGF produced of control was 7.77 ng/ml.) | | |

(9) Each of a solution of the sulfated polysaccharide fraction derived from *Gelidium amansii* (Sample (1)), the sulfated polysaccharide derived from *Gracilaria verrucosa* (Sample (2)), and the sulfated polysaccharide derived from *Pterocladiella* (Sample (3)) each prepared in Reference Example 10 was prepared, and its induction activity for HGF production was studied in the same manner as that in item (1) of Example 1. Samples (1) and (3) were added so as to have a final concentration of 1, 10 or 100 µg/ml, and Sample (2) was added so as to have a final concentration of 10 or 100 µg/ml. As shown in Table 13, all of Samples (1) to (3) induced the production of HGF.

**Table 13**

| Sample | Concentration (µg/ml) | Amount of HGF Produced (%) |
|---|---|---|
| Sample (1) | 1 | 100 |
| | 10 | 177 |
| | 100 | 169 |
| Sample (2) | 10 | 117 |
| | 100 | 198 |
| Sample (3) | 1 | 116 |
| | 10 | 207 |
| | 100 | 228 |
| (The amount of HGF produced of control was 7.3 ng/ml.) | | |

(10) The induction activity for HGF production for the fucoidan derived from *Lessonia* (Sample (1)), DEAE Fraction 33 (Sample (2)), DEAE Fraction 37 (Sample (3)), and DEAE Fraction 40 (Sample (4)) each prepared in item (3) of Reference Example 10 was studied in the same manner as that in item (1) of Example 1. Each of the samples was added so as to have a final concentration of 1, 10 or 100 µg/ml. As shown in Table 14, all of Samples (1) to (4) induced the production of HGF.

**Table 14**

| Concentration (µg/ml) | Amount of HGF Produced (%) | | | |
|---|---|---|---|---|
| | Sample (1) | Sample (2) | Sample (3) | Sample (4) |
| 1 | 138 | 105 | 235 | 121 |
| 10 | 167 | 221 | 253 | 254 |
| 100 | 331 | 265 | 261 | 295 |
| (The amount of HGF produced of control was 11.5 ng/ml.) | | | | |

(11) The induction activity for HGF production for the sulfated fucogalactan described in item (2) of Reference Example 3, the agaropectin described in Reference Example 12, chondroitin sulfate B (manufactured by SEIKAGAKU CORPORATION), and chondroitin sulfate D (manufactured by SEIKAGAKU CORPORATION) was studied under the same conditions as those in item (1) of Example 1. Each of the samples was added so as to have a final concentration of 1, 10 or 100 µg/ml. As shown in Tables 15 to 17, the sulfated fucogalactan, the agaropectin, and the chondroitin sulfates induced the production of HGF.

**Table 15**

| Sample | Concentration (µg/ml) | Amount of HGF Produced (%) |
|---|---|---|
| Sulfated Fucogalactan | 1 | 194 |
| | 10 | 355 |
| | 100 | 429 |
| (The amount of HGF produced of control was 4.3 ng/ml.) | | |

**Table 16**

| Sample | Concentration (µg/ml) | Amount of HGF Produced (%) |
|---|---|---|
| Agalopectin | 1 | 117 |
| | 10 | 121 |
| | 100 | 256 |
| (The amount of HGF produced of control was 6.7 ng/ml.) | | |

**Table 17**

| Sample | Concentration (µg/ml) | Amount of HGF Produced (%) |
|---|---|---|
| Chondroitin Sulfate B | 1 | 117 |
| | 10 | 121 |
| | 100 | 256 |
| Chondroitin Sulfate D | 10 | 119 |
| | 100 | 144 |
| (The amount of HGF produced of control was 11.9 ng/ml.) | | |

(12) The induction activity for HGF production for the sulfated polysaccharide derived from *Spirulina,* the sulfated polysaccharide derived from *Chlorella*, the sulfated polysaccharide derived from *Altemisia princeps pampan*, the sulfated polysaccharide derived from *Momordica charantia,* the sulfated polysaccharide derived from the mesophyll of *Aloe arborescens Mill. var. natalensis,* and the sulfated polysaccharide derived from a product from the surface portions of leaves of *Aloe arborescens Mill. var*. *natalensis* each prepared in item (1) of Reference Example 13, item (3) of Reference Example 13, item (5) of Reference Example 13, item (7) of Reference Example 13, and item (8) of Reference Example 13, was studied in the same manner as that in item (1) of Example 1. Each of the sulfated polysaccharide derived from *Spirulina* and the sulfated polysaccharide derived from *Altemisia princeps pampan* was added so as to have a final concentration of 1, 10 or 100 µg/ml. Each of the sulfated polysaccharide derived from *Chlorella*, the sulfated polysaccharide derived from *Momordica charantia*, the sulfated polysaccharide from the mesophyll of *Aloe arborescens Mill. var*. *natalensis*, and the sulfated polysaccharide derived from a product from the surface portions of leaves of *Aloe arborescens Mill. var. natalensis* was added so as to have a final concentration of 1, 10, 100 or 1000 µg/ml. As shown in Tables 18 to 20, each of the sulfated polysaccharide derived from *Spirulina*, the sulfated polysaccharide derived from *Chlorella,* the sulfated polysaccharide derived from *Altemisia princeps pampan*, the sulfated polysaccharide derived from *Momordica charantia*, the sulfated polysaccharide derived from the mesophyll of *Aloe arborescens Mill. var. natalensis,* and the sulfated polysaccharide derived from a product from the surface portions of leaves of *Aloe arborescens Mill*. *var. natalensis* induced the production of HGF.

**Table 18**

| Sample | Concentration (µg/ml) | Amount of HGF Produced (%) |
|---|---|---|
| Sulfated Polysaccharide | 1 | 149 |
| Derived from *Spirulina* | 10 | 293 |
| | 100 | 398 |
| Sulfated Polysaccharide | 1 | 108 |
| Derived from *Chlorella* | 10 | 149 |
| | 100 | 175 |
| | 1000 | 396 |
| (The amount of HGF produced of control was 7.9 ng/ml.) | | |

**Table 19**

| Sample | Concentration (µg/ml) | Amount of HGF Produced (%) |
|---|---|---|
| Sulfated Polysaccharide | 1 | 137 |
| Derived from *Altemisia* | 10 | 284 |
| *princeps pampan* | 100 | 265 |
| (The amount of HGF produced of control was 12.7 ng/ml.) | | |

**Table 20**

| Sample | Concentration (µg/ml) | Amount of HGF Produced (%) |
|---|---|---|
| Sulfated Polysaccharide | 1 | 111 |
| Derived from *Momordica* | 10 | 104 |
| *charantia* | 100 | 133 |
| | 1000 | 190 |
| Sulfated Polysaccharide | 1 | 111 |
| Derived from Mesophyll | 10 | 125 |
| of *Aloe arborescens Mill.* | 100 | 150 |
| *var*. *natalensis* | 1000 | 401 |
| Sulfated Polysaccharide | 1 | 106 |
| Derived from Product | 10 | 125 |
| from Surface Portions of | 100 | 120 |
| Leaf *of Aloe arborescens Mill. var. natalensis* | 1000 | 328 |
| (The amount of HGF produced of control was 8.7 ng/ml.) | | |

(13) The induction activity for HGF production for the *Spirulina* Fractions SSP-I (Sample (1)), SSP-II (Sample (2)), SSP-III (Sample (3)) and SSP-IV (Sample (4)) each prepared in item (2) of Reference Example 13 was studied in the same manner as that in item (1) of Example 1. Each of the samples was added so as to have a final concentration of 1, 10 or 100 µg/ml. As shown in Table 21, all of Samples (1) to (4) induced the production of HGF.

**Table 21**

| Concentration (µg/ml) | Amount of HGF Produced (%) | | | |
|---|---|---|---|---|
| | Sample (1) | Sample (2) | Sample (3) | Sample (4) |
| 1 | 240 | 165 | 141 | 218 |
| 10 | 243 | 152 | 270 | 282 |
| 100 | 302 | 212 | 280 | 351 |
| (The amount of HGF produced of control was 7.3 ng/ml.) | | | | |

(14) The induction activity for HGF production for the *Chlorella* Extract Fractions CSP-I (Sample (1)) and CSP-II (Sample (2) each prepared in item (4) of Reference Example 13 was studied in the same manner as that in item (1) of Example 1. Sample (1) was added so as to have a final concentration of 10 or 100 µg/ml, and Sample (2) was added so as to have a final concentration of 100 µg/ml. As shown in Table 22, Samples (1) and (2) induced the production of HGF.

**Table 22**

| Sample | Concentration (µg/ml) | Amount of HGF Produced (%) |
|---|---|---|
| Sample (1) | 10 | 111 |
| | 100 | 173 |
| Sample (2) | 100 | 175 |
| (The amount of HGF produced of control was 11.4 ng/ml.) | | |

(15) The induction activity for HGF production for the *Altemisia princeps pampan* Extract Fractions YAP (Sample (1)), YSP-I (Sample (2), YSP-II (Sample (3)), YSP-II-2 (Sample (4)), YSP-II-3 (Sample (5)), YSP-II-4 (Sample (6)), each prepared in item (6) of Reference Example 13 was studied in the same manner as that in item (1) of Example 1. Each of the samples was added so as to have a final concentration of 1, 10 or 100 µg/ml. As shown in Tables 23 and 24, all of Samples (1) to (6) induced the production of HGF. Especially, YSP-II (Sample (3)), YSP-II-3 (Sample (4)), and YSP-II-4 (Sample (5)) were confirmed to have strong induction activity for HGF production.

**Table 23**

| Sample | Concentration (µg/ml) | Amount of HGF Produced (%) |
|---|---|---|
| Sample (1) | 10 | 121 |
| | 100 | 266 |
| Sample (2) | 1 | 104 |
| | 10 | 148 |
| | 100 | 381 |
| Sample (3) | 1 | 328 |
| | 10 | 386 |
| | 100 | 390 |
| (The amount of HGF produced of control was 11.5 ng/ml.) | | |

**Table 24**

| Concentration (µg/ml) | Amount of HGF Produced (%) | | |
|---|---|---|---|
| | Sample (4) | Sample (5) | Sample (6) |
| 1 | 152 | 290 | 226 |
| 10 | 462 | 383 | 322 |
| 100 | 475 | 321 | 314 |
| (The amount of HGF produced of control was 7.7 ng/ml.) | | | |

(16) The induction activity for HGF production for the sodium salt of sulfated maltose, the sodium salt of sulfated maltotriose, the sodium salt of sulfated lactose, the sodium salt of sulfated sucrose, the sodium salt of sulfated trehalose, the sodium salt of sulfated glucose, the sodium salt of sulfated lactulose, the sodium salt of sulfated melibiose, the sodium salt of sulfated galactose, the sodium salt of sulfated mannose, the sodium salt of sulfated xylose, the sodium salt of sulfated 2-deoxy-glucose, the sodium salt of sulfated glucitol, the sodium salt of sulfated cellobiose, the sodium salt of sulfated isomaltose, the sodium salt of sulfated turanose, the sodium salt of sulfated palatinose, the sodium salt of sulfated talose, the sodium salt of sulfated maltohexaose, the sodium salt of sulfated maltoheptaose, the sodium salt of sulfated dodecyl-maltohexaose, the sodium salt of sulfated starch, the sodium salt of sulfated cardran, and the sodium salt of sulfated pectin, each prepared in Reference Example 14 was studied in the same manner as that in item (1) of Example 1. Each of the samples was added so as to have a final concentration of 1, 10 or 100 µg/ml; or 10, 100 or 1000 µg/ml; or 100 µg/ml. As a control, distilled water was added in the same volume as that of the sample. In addition, the induction activity for HGF production for each non-sulfated saccharide was also determined at the same concentration as that of the sulfated saccharide.

As shown in Tables 25 to 34, the sulfated oligosaccharides and the sulfated monosaccharides induced the production of HGF. However, each non-sulfated saccharide did not induce HGF.

Further, from the results of the sodium salt of sulfated dodecyl-maltohexaose, it was clarified that saccharide keeps the induction activity for HGF production even if the saccharide is subjected to a modification by a lipid.

**Table 25**

| Sample | Concentration (µg/ml) | Amount of HGF Produced (%) |
|---|---|---|
| Sodium Salt of | 1 | 142 |
| Sulfated Maltose | 10 | 273 |
| | 100 | 439 |
| Sodium Salt of | 1 | 151 |
| Sulfated | 10 | 286 |
| Maltotriose | 100 | 387 |
| (The amount of HGF produced of control was 8.7 ng/ml.) | | |

**Table 26**

| Sample | Concentration (µg/ml) | Amount of HGF Produced (%) |
|---|---|---|
| Sodium Salt of | 1 | 118 |
| Sulfated Lactose | 10 | 185 |
| | 100 | 410 |
| Sodium Salt of | 1 | 173 |
| Sulfated Sucrose | 10 | 355 |
| | 100 | 501 |
| Sodium Salt of | 1 | 178 |
| Sulfated Glucose | 10 | 377 |
| | 100 | 864 |
| (The amount of HGF produced of control was 5.5 ng/ml.) | | |

**Table 27**

| Sample | Concentration (µg/ml) | Amount of HGF Produced (%) |
|---|---|---|
| Sodium Salt of | 1 | 277 |
| Sulfated Trehalose | 10 | 447 |
| | 100 | 421 |
| (The amount of HGF produced of control was 4.3 ng/ml.) | | |

**Table 28**

| Sample | Concentration (µg/ml) | Amount of HGF Produced (%) |
|---|---|---|
| Sodium Salt of Sulfated Galactose | 100 | 166 |
| (The amount of HGF produced of control was 12.7 ng/ml.) | | |

**Table 29**

| Sample | Concentration (µg/ml) | Amount of HGF Produced (%) |
|---|---|---|
| Sodium Salt of | 1 | 112 |
| Sulfated Mannose | 10 | 175 |
| | 100 | 456 |
| (The amount of HGF produced of control was 6.2 ng/ml.) | | |

**Table 30**

| Sample | Concentration (µg/ml) | Amount of HGF Produced (%) |
|---|---|---|
| Sodium Salt of | 10 | 139 |
| Sulfated | 100 | 376 |
| Lactulose | 1000 | 583 |
| Sodium Salt of | 10 | 240 |
| Sulfated | 100 | 403 |
| Melibiose | 1000 | 667 |
| Sodium Salt of | 10 | 110 |
| Sulfated Xylose | 100 | 112 |
| | 1000 | 284 |
| Sodium Salt of | 10 | 127 |
| Sulfated | 100 | 102 |
| 2-Deoxy-Glucose | 1000 | 239 |
| Sodium Salt of | 10 | 112 |
| Sulfated Glucitol | 100 | 203 |
| | 1000 | 335 |
| (The amount of HGF produced of control was 5.9 ng/ml.) | | |

**Table 31**

| Sample | Concentration (µg/ml) | Amount of HGF Produced (%) |
|---|---|---|
| Sodium Salt of | 10 | 100 |
| Sulfated | 100 | 143 |
| Cellobiose | 1000 | 546 |
| Sodium Salt of | 10 | 134 |
| Sulfated | 100 | 301 |
| Isomaltose | 1000 | 477 |
| Sodium Salt of | 10 | 127 |
| Sulfated Turanose | 100 | 203 |
| | 1000 | 379 |
| Sodium Salt of | 10 | 132 |
| Sulfated | 100 | 278 |
| Palatinose | 1000 | 519 |
| (The amount of HGF produced of control for the sodium salt of sulfated cellobiose was 11.1 ng/ml, that for the sodium salt of sulfated isomaltose was 11.3 ng/ml, that for the sodium salt of sulfated turanose and the sodium salt of sulfated palatinose was 8.6 ng/ml.) | | |

**Table 32**

| Sample | Concentration (µg/ml) | Amount of HGF Produced (%) |
|---|---|---|
| Sodium Salt of | 10 | 112 |
| Sulfated Talose | 100 | 263 |
| | 1000 | 494 |
| (The amount of HGF produced of control was 9.5 ng/ml.) | | |

**Table 33**

| Sample | Concentration (µg/ml) | Amount of HGF Produced (%) |
|---|---|---|
| Sodium Salt of | 10 | 407 |
| Sulfated | 100 | 571 |
| Maltohexaose | 1000 | 761 |
| Sodium Salt of | 10 | 341 |
| Sulfated | 100 | 486 |
| Maltoheptaose | 1000 | 706 |
| Sodium Salt of | 10 | 289 |
| Sulfated Dodecyl- | 100 | 371 |
| Maltohexaose | 1000 | 359 |
| (The amount of HGF produced of control was 8.15 ng/ml.) | | |

**Table 34**

| Sample | Concentration (µg/ml) | Amount of HGF Produced (%) |
|---|---|---|
| Sodium Salt of | 10 | 781 |
| Sulfated Starch | 100 | 864 |
| | 1000 | 804 |
| Sodium Salt of | 10 | 359 |
| Sulfated Cardran | 100 | 503 |
| | 1000 | 617 |
| Sodium Salt of | 10 | 721 |
| Sulfated Pectin | 100 | 780 |
| | 1000 | 648 |
| (The amount of HGF produced of control was 8.15 ng/ml.) | | |

### Example 2

(1) Synergistic effect for the induction action for HGF production of the fucoidan derived from *Kjellmaniella crassifolia* described in item (1) of Reference Example 1 with prostaglandin or IL-1 was studied in the same manner as that in item (1) of Example 1.

Specifically, the synergistic effect for the induction activity for HGF production was studied by simultaneously adding the fucoidan and PGE₁ (manufactured by Wako Pure Chemicals Industries, Ltd.) or IL-1α (manufactured by Genzyme).

The fucoidan sample was added so as to have a final concentration of 1, 10 or 100 µg/ml. PGE₁ was added so as to have a concentration of 0.1 or 1 µM, the IL-1α was added so as to have a concentration of 1 ng/ml. As a control, distilled water was added in same volume as that of the sample.

The synergistic effect was studied in comparison to the amount produced of a case where the fucoidan sample or PGE₁ or IL-1α was added alone.

The results are shown in Tables 35 and 36. In Tables 35 and 36, the amount of HGF produced of the control was regarded as 100%. Each experiment was carried out twice, and an average value was taken. As shown in Tables 35 and 36, the synergistic effect for induction of HGF production was found in a case where PGE₁ or IL-1α was simultaneously added with the fucoidan.

**Table 35**

| Amount of Fucoidan Added (µg/ml) | Amount of PGE₁ Added (µM) | | |
|---|---|---|---|
| | 0 | 0.1 | 1 |
| | Amount of HGF Produced (%) | | |
| 0 | 100 | 119 | 157 |
| 1 | 195 | 334 | 415 |
| 10 | 331 | 517 | 561 |
| 100 | 382 | 731 | 682 |

**Table 36**

| Amount of Fucoidan Added (µg/ml) | Amount of IL-1α Added (ng/ml) | |
|---|---|---|
| | 0 | 1 |
| | Amount of HGF Produced (%) | |
| 0 | 100 | 163 |
| 1 | 206 | 421 |
| 10 | 350 | 672 |
| 100 | 403 | 780 |
| (The amount of HGF produced of control was 9.1 ng/ml.) | | |

(2) Synergistic effect for the induction action for HGF production of 7-12SFd-F of Reference Example 2 with prostaglandin or IL-1 was studied in the same manner as that in item (1) of Example 2. The synergistic effect for the induction activity for HGF production was studied by simultaneously adding 7-12SFd-F and PGE₁ or IL-1α. In each case, 7-12SFd-F was added so as to have a final concentration of 1,10 or 100 µg/ml. PGE₁ or IL-1α was further simultaneously added to 7-12SFd-F-added cells. PGE₁ was added so as to have a concentration of 0.1 or 1 µM, and IL-1α was added so as to have a concentration of 0.1 or 1 ng/ml. As a negative control, distilled water was added in the same volume as that of the sample. The synergistic effect was studied in comparison to the amount produced of a case where 7-12SFd-F or PGE₁ or IL-1α was added alone. The amount of HGF produced of the negative control was regarded as 100%. The results are shown in Tables 37 and 38. Each experiment was carried out three times, and an average value was taken.

**Table 37**

| Amount of 7-12SFd-F Added (µg/ml) | Amount of PGE₁ Added (µM) | | |
|---|---|---|---|
| | 0 | 0.1 | 1 |
| | Amount of HGF Produced (%) | | |
| 0 | 100 | 123 | 170 |
| 1 | 120 | 158 | 216 |
| 10 | 218 | 309 | 317 |
| 100 | 219 | 365 | 382 |
| (The amount of HGF produced of control was 5.1 ng/ml.) | | | |

**Table 38**

| Amount of 7-12SFd-F Added | Amount of IL-1α Added (ng/ml) | | |
|---|---|---|---|
| | 0 | 0.1 | 1 |
| (µg/ml) | Amount | of HGF Produced (%) | |
| 0 | 100 | 130 | 151 |
| 1 | 140 | 168 | 159 |
| 10 | 191 | 154 | 208 |
| 100 | 203 | 255 | 222 |
| (The amount of HGF produced of control was 5.1 ng/ml.) | | | |

### Example 3

(1) Five-hundred microliters each of KG-1-C cells (Glyoma: manufactured by Human Science Shinko Zaidan) suspended in a DMEM medium containing 10% fetal bovine serum so as to have a concentration of 1 × 10⁵ cells/ml were added into each well of a 48-well cell culture plate. The cells were cultured at 37°C overnight in the presence of 5% CO₂, and then the medium was exchanged for a DMEM medium containing 1% fetal bovine serum. Thereafter, a sample to be analyzed was added thereto, and the cells were further cultured for 20 hours. The medium was then collected, and the amount of HGF in the medium was determined by using the HGF ELISA Kit described in Example 1.

The sample to be analyzed was added so as to have a final concentration of 1, 10 or 100 µg/ml in the case of the fucoidan derived from *Kjellmaniella crassifolia* described in item (1) of Reference Example 1, or to have a final concentration of 1 or 10 µg/ml in the case of heparin (manufactured by Wako Pure Chemical Industries, Ltd.). In addition, as a control, distilled water was added in the same volume as that of the sample. Each experiment was carried out three times, and an average value was taken. The results are shown in Table 39. In Table 39, the amount of HGF produced of the control was regarded as 100%.

All of the cells to which the fucoidan sample was added showed significant increase in the amount of HGF produced, as compared to that of the distilled water-added control, and showed remarkable increase in the amount of HGF produced as compared to that of the heparin-added case. It was shown from the above that the fucoidan possesses higher activity for promoting the production of HGF as compared to heparin in which induction of HGF has been confirmed.

**Table 39**

| Sample | Concentration (µg/ml) | Amount of HGF Produced (%) |
|---|---|---|
| Fucoidan Derived from | 1 | 194 |
| *Kjellmaniella* | 10 | 285 |
| *crassifolia* | 100 | 351 |
| Sodium Salt of Heparin | 1 | 176 |
| | 10 | 215 |
| (The amount of HGF produced of control was 4.4 ng/ml.) | | |

(2) HL-60 cells (promyelocytic leukemia cell line: ATCC CCL-240) cultured in an RPMI1640 medium containing 10% fetal bovine serum were suspended in an RPMI1640 medium containing 1% fetal bovine serum so as to have a concentration of 1 × 10⁵ cells/ml, and 500 µl each of the suspension was added into each well of a 48-well cell culture plate. Thereafter, 10 nM 12-O-tetradecanoylphorbol 13-acetate (TPA: manufactured by Gibco BRL) was added thereto, and a sample to be analyzed was further simultaneously added. After the addition, the cells were cultured for 20 hours. The medium was then collected, and the amount of HGF in the medium was determined by using the HGF ELISA Kit.

The sample to be analyzed was added so as to have a final concentration of 1, 10 or 100 µg/ml in the case of the fucoidan derived from *Kjellmaniella crassifolia* described in item (1) of Reference Example 1. Heparin was added so as to have a final concentration of 1 or 10 µg/ml. As a control, distilled water was added in the same volume as that of the sample. Each experiment was carried out three times, and an average value was taken. The results are shown in Table 40. In Table 40, the amount of HGF produced of the control was regarded as 100%.

All of the cells to which the fucoidan sample was added showed significant increase in the amount of HGF produced, as compared to that of the distilled water-added control, and showed remarkable increase in the amount of HGF produced as compared to that of the heparin-added cells. It was shown from the above that the fucoidan possesses higher activity for promoting the production of HGF as compared to heparin in which induction of HGF has been confirmed.

**Table 40**

| Sample | Concentration (µg/ml) | Amount of HGF Produced (%) |
|---|---|---|
| Fucoidan Derived from | 1 | 191 |
| *Kjellmaniella crassifolia* | 10 | 342 |
| | 100 | 490 |
| Sodium Salt of Heparin | 1 | 140 |
| | 10 | 189 |
| (The amount of HGF produced of control was 0.4 ng/ml.) | | |

### Example 4

Five-hundred microliters of a solution containing MRC-5 cells suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, a sample was added thereto, and the cells were further cultured for 24 hours. The medium was then collected, and the amount of HGF in the medium was determined by using the HGF ELISA Kit. Further, the cells were washed with PBS, and thereafter dissolved in 500 µl of a cytolytic buffer (50 mM HEPES, pH 7.4; 10 mM EDTA; 0.1% Triton x 100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium.

The fucoidan derived from *Kjellmaniella crassifolia* described in item (1) of Reference Example 1, the sample to be analyzed, was added so as to have a final concentration of 1,10 or 100 µg/ml. As a control, distilled water was added in the same volume as that of the sample. Each experiment was carried out twice, and an average value was taken. The results are shown in Table 41. As shown in Table 41, as to HGF in the medium of the fucoidan-added group, the amount of HGF increased more significantly in a fucoidan concentration-dependent manner, as compared to that of the distilled water-added control. On the other hand, the amount of HGF in the cells decreased in a fucoidan concentration-dependent manner. Further, the total amount of HGF in and outside the cells increased in a concentration-dependent manner. It was shown from the above finding that the fucoidan possesses activity of promoting the production of HGF and action of promoting the freeing of HGF from the cells.

**Table 41**

| Fucoidan Derived from *Kjellmaniella crassifolia* (µg/ml) | Amount of HGF Produced (ng/well) | | |
|---|---|---|---|
| | in Medium | in Cell | Total Amount |
| Control | 4.2 | 5.7 | 9.8 |
| 1 | 9.3 | 3.2 | 12.5 |
| 10 | 15.9 | 0.9 | 16.8 |
| 100 | 16.9 | 0.4 | 17.3 |

### Example 5

(1) Five-hundred microliters of a solution containing MRC-5 cells suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, a sample was added thereto, and the cells were further cultured for 0, 0.5, 1, 2, 4, 8, 12, or 24 hours. The medium was then collected, and the amount of HGF in the medium was determined by using the HGF ELISA Kit. The fucoidan derived from *Kjellmaniella crassifolia* described in item (1) of Reference Example 1 was added so as to have a final concentration of 10 µg/ml. As a control, distilled water was added in the same volume as that of the sample. The results are shown in Table 42. As shown in Table 42, in the fucoidan-added group, the amount of HGF produced increased more significantly in a time-dependent manner, as compared to that of the control.

It was shown from the above that the fucoidan possesses high activity for promoting HGF production, and the amount of HGF produced increases with the passage of time.

**Table 42**

| | Culture Time (hour) | | | | | | | |
|---|---|---|---|---|---|---|---|---|
| | 0 | 0.5 | 1 | 2 | 4 | 8 | 12 | 24 |
| | Amount of HGF Produced (ng/ml) | | | | | | | |
| Control | 0.02 | 0.03 | 0.81 | 0.90 | 2.31 | 3.90 | 6.94 | 9.39 |
| Fucoidan Added | 0.19 | 1.90 | 5.75 | 6.87 | 9.04 | 15.9 | 20.1 | 31.3 |

(2) Five-hundred microliters of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, a sample was added thereto, and the cells were further cultured for 0, 0.5, 1, 2, 4, 8, 12, 24, 48 or 72 hours. The medium was then collected, and the amount of HGF in the medium was determined by using Quantikine Human Hepatocyte Growth Factor (HGF) ELISA Kit (manufactured by Funakoshi, Code. RS-0641-00). Further, the medium was collected, and then the cells were washed with PBS, and thereafter dissolved in 500 µl of a cytolytic buffer (50 mM HEPES, pH 7.4; 10 mM EDTA; 0.1% Triton x 100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium. The fucoidan derived from *Kjellmaniella crassifolia* described in item (1) of Reference Example 1 was added so as to have a final concentration of 10 µg/ml. As a negative control, distilled water was added in the same volume as that of the sample. The concentration of HGF in the medium of the fucoidan-added group more significantly increased in a time-dependent manner, as compared to that of the distilled water-added negative control. On the other hand, the amount of HGF in the cells of the fucoidan-added group decreased up to 4 hours after the addition, but showed a given low value thereafter. In the distilled water-added negative control, such a change was not seen, constantly showing the tendency of increase. It was shown from the above finding that the fucoidan possesses effect of freeing HGF from the cells and activity of promoting the production of HGF, so that the amount of HGF produced increases with the passage of time. The results are shown in Tables 43 to 45.

**Table 43**

| Change in Amount of HGF in Medium with Passage of Time | | | | | | | | | | |
|---|---|---|---|---|---|---|---|---|---|---|
| | 0 | 0.5 | 1 | 2 | 4 | 8 | 12 | 24 | 48 | 72 |
| | Amount of HGF Produced (ng/well) | | | | | | | | | |
| Control | 0 | 0.11 | 0.19 | 0.29 | 1.07 | 1.56 | 2.34 | 3.46 | 7.45 | 10.5 |
| Fucoidan Added | 0.51 | 0.46 | 1.23 | 2.86 | 4.00 | 6.70 | 9.15 | 12.4 | 22.8 | 29.3 |

**Table 44**

| Change in Amount of HGF in Cell with Passage of Time | | | | | | | | | | |
|---|---|---|---|---|---|---|---|---|---|---|
| | Culture Time (hour) | | | | | | | | | |
| | 0 | 0.5 | 1 | 2 | 4 | 8 | 12 | 24 | 48 | 72 |
| | Amount of HGF Produced (ng/well) | | | | | | | | | |
| Control | 0.51 | 0.52 | 0.66 | 0.56 | 0.71 | 0.63 | 0.86 | 0.73 | 1.02 | 1.20 |
| Fucoidan Added | 0.88 | 0.66 | 0.45 | 0.46 | 0.25 | 0.29 | 0.20 | 0.20 | 0.18 | 0.23 |

**Table 45**

| Change in Total Amount of HGF in Medium and in Cell with Passage of Time | | | | | | | | | | |
|---|---|---|---|---|---|---|---|---|---|---|
| | Culture Time (hour) | | | | | | | | | |
| | 0 | 0.5 | 1 | 2 | 4 | 8 | 12 | 24 | 48 | 72 |
| | Amount of HGF Produced (ng/well) | | | | | | | | | |
| Control | 0.51 | 0.63 | 0.85 | 0.84 | 1.78 | 2.19 | 3.19 | 4.19 | 8.47 | 11.6 |
| Fucoidan Added | 1.39 | 1.12 | 1.68 | 3.31 | 4.25 | 7.00 | 9.35 | 12.6 | 22.9 | 29.5 |

(3) Five-hundred microliters of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, a sample was added thereto, and the cells were further cultured for 0, 0.5, 1, 2, 4, 8, 12, 24, 48 or 72 hours. The medium was then collected, and the amount of HGF in the medium was determined by using Quantikine Human Hepatocyte Growth Factor (HGF) ELISA Kit (manufactured by Funakoshi, Code. RS-0641-00). Further, the medium was collected, and thereafter the cells were washed with PBS, and then dissolved in 500 µl of a cytolytic buffer (50 mM HEPES, pH 7.4; 10 mM EDTA; 0.1% Triton x 100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium. 7-12SFd-F was added so as to have a final concentration of 10 µg/ml. As a negative control, distilled water was added in the same volume as that of the sample. The concentration of HGF in the medium of the 7-12SFd-F-added group more significantly increased in a time-dependent manner, as compared to that of the distilled water-added negative control. On the other hand, the amount of HGF in the cell of the 7-12SFd-F-added group decreased for some period of time after the addition, but thereafter rather increased. In the distilled water-added negative control, such a change was not seen, constantly showing a given value. It was shown from the above finding that the fucoidan possesses effect of freeing HGF from the cells and high activity of promoting the production of HGF, so that the amount of HGF produced increased with the passage of time. Thereafter, it was kept a given low value. The results are shown in Tables 46 to 48.

**Table 46**

| Change in Amount of HGF in Medium with Passage of Time | | | | | | | | | | |
|---|---|---|---|---|---|---|---|---|---|---|
| | Culture Time (hour) | | | | | | | | | |
| | 0 | 0.5 | 1 | 2 | 4 | 8 | 12 | 24 | 48 | 72 |
| | Amount of HGF Produced (ng/well) | | | | | | | | | |
| Control | 0.14 | 0.33 | 0.36 | 0.63 | 1.29 | 1.60 | 2.54 | 3.89 | 7.99 | 13.3 |
| 7-12SFd-F Added | 0.48 | 1.82 | 2.16 | 2.53 | 3.12 | 4.01 | 5.84 | 9.82 | 17.0 | 23.5 |

**Table 47**

| Change in Amount of HGF in Cell with Passage of Time | | | | | | | | | | |
|---|---|---|---|---|---|---|---|---|---|---|
| | Culture Time (hour) | | | | | | | | | |
| | 0 | 0.5 | 1 | 2 | 4 | 8 | 12 | 24 | 48 | 72 |
| | Amount of HGF Produced (ng/well) | | | | | | | | | |
| Control | 2.65 | 3.11 | 2.73 | 2.77 | 2.31 | 2.82 | 2.80 | 4.61 | 5.36 | 6.74 |
| 7-12SFd-F Added | 2.79 | 1.78 | 1.65 | 1.31 | 1.06 | 1.31 | 1.07 | 1.56 | 2.66 | 3.09 |

**Table 48**

| Change in Total Amount of HGF in Medium and in Cell with Passage of Time | | | | | | | | | | |
|---|---|---|---|---|---|---|---|---|---|---|
| | Culture Time (hour) | | | | | | | | | |
| | 0 | 0.5 | 1 | 2 | 4 | 8 | 12 | 24 | 48 | 72 |
| | Amount of HGF Produced (ng/well) | | | | | | | | | |
| Control | 2.79 | 3.43 | 3.09 | 3.39 | 3.60 | 4.41 | 5.34 | 8.50 | 13.3 | 20.2 |
| 7-12SFd-F Added | 3.26 | 3.60 | 3.81 | 3.84 | 4.18 | 5.31 | 6.91 | 11.4 | 19.7 | 26.6 |

(4) Five-hundred microliters of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, a sample was added thereto, and the cells were further cultured for 24 hours. The medium was then collected, and the amount of HGF in the medium was determined by using Quantikine Human Hepatocyte Growth Factor (HGF) ELISA Kit (manufactured by Funakoshi, Code. RS-0641-00). Further, the cells were washed with PBS, and thereafter dissolved in 500 µl of a cytolytic buffer (50 mM HEPES, pH 7.4; 10 mM EDTA; 0.1% Triton x100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium. 7-12SFd-F was added so as to have a final concentration of 1, 10 or 100 µg/ml. As a negative control, distilled water was added in the same volume as that of the sample. Each experiment was carried out three times, and an average value was taken. As a result, as shown in Table 49, as to HGF in the medium of the 7-12SFd-F-added group, the amount of HGF produced more significantly increased in a 7-12SFd-F concentration-dependent manner, as compared to that of the distilled water-added negative control. On the other hand, the amount of HGF in the cells of the 7-12SFd-F-added group decreased in a 7-12SFd-F concentration-dependent manner. Further, the total amount of HGF in and outside the cells increased in a concentration-dependent manner. It was shown from the above finding that 7-12SFd-F possesses effect of freeing the HGF from the cells and activity of promoting the production of HGF. The results are shown in Table 49.

**Table 49**

| Amount of HGF Produced During Addition of 7-12SFd-F | | | | |
|---|---|---|---|---|
| | Amount of 7-12SFd-F added (Final Concentration, µg/ml) | | | |
| | 0 | 1 | 10 | 100 |
| | Amount of HGF Produced (ng/well) | | | |
| in Medium | 5.66 | 8.46 | 17.1 | 22.0 |
| in Cell | 6.28 | 6.04 | 4.15 | 1.59 |
| Total Amount | 11.9 | 14.5 | 21.3 | 23.6 |

### Example 6

(1) Five-hundred microliters of a solution containing MRC-5 cells suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, cycloheximide (protein synthesis inhibitor: manufactured by nacalaitesque) was added thereto so as to have a final concentration of 0, 1, or 10 µg/ml, and a sample to be analyzed was further added thereto. Thereafter, the cells were cultured for 24 hours. This medium was collected, and the amount of HGF in the medium was determined by using the HGF ELISA Kit. The fucoidan derived from *Kjellmaniella crassifolia* described in item (1) of Reference Example 1 was added so as to have a final concentration of 1, 10 or 100 µg/ml. As a control, distilled water was added in the same volume as that of the sample. Each experiment was carried out twice, and an average value was taken. The results are shown in Table 50. In Table 50, the amount of HGF produced of the control was regarded as 100%.

As shown in Table 50, as a result of the addition of cycloheximide, the concentration of HGF in the medium of the fucoidan-added group decreased in a cycloheximide concentration-dependent manner. As to its inhibitory ratio, it was inhibited in a cycloheximide concentration-dependent manner in the same level as that of the inhibition by cycloheximide in the fucoidan non-added control group. It was clarified from these findings that protein synthesis was involved in the induction of HGF production by the fucoidan.

**Table 50**

| Fucoidan Derived from *Kjellmaniella crassifolia* (µg/ml) | Amount of Cycloheximide Added (µg/ml) | | |
|---|---|---|---|
| | 0 | 1 | 10 |
| | Ratio of Amount of HGF Produced to Control (%) | | |
| Control | 100 | 35 | 27 |
| 1 | 226 | 110 | 72 |
| 10 | 317 | 130 | 110 |
| 100 | 456 | 206 | 127 |
| (The amount of HGF produced of control was 7.3 ng/ml.) | | | |

(2) Five-hundred microliters of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, cycloheximide (protein synthesis inhibitor: manufactured by nacalaitesque) was added thereto so as to have a final concentration of 0, 1, or 10 µg/ml, and a sample to be analyzed was further added thereto. The cells were then cultured for 24 hours. This medium was collected, and the amount of HGF in the medium was determined by using Quantikine Human Hepatocyte Growth Factor (HGF) ELISA Kit (manufactured by Funakoshi, Code. RS-0641-00). In addition, the cells were washed with PBS, and thereafter dissolved in 500 µl of a cytolytic buffer (50 mM HEPES, pH 7.4; 10 mM EDTA, 0.1% Triton x 100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium. As to the amount of HGF produced, negative control was regarded as 100%. As to the inhibitory ratio, the inhibitory ratio (%) in the cycloheximide-added fraction was calculated on the basis of the amount of HGF produced when 7-12SFd-F was added alone at each concentration. 7-12SFd-F was added so as to have a final concentration of 1, 10 or 100 µg/ml. As the negative control, distilled water was added in the same volume as that of the sample. Each experiment was carried out three times, and an average value was taken. As a result, as shown in Tables 51 and 52, as a result of the addition of cycloheximide, both the amount of HGF in the medium and the total amount of HGF in the cells and the medium of the 7-12SFd-F-added group decreased in a cycloheximide concentration-dependent manner. It was clarified from these findings that protein synthesis was involved in the induction of HGF production by 7-12SFd-F, not simply freeing of HGF from the cells.

**Table 51**

| Inhibition of Induction of HGF Production (in Medium) | | | |
|---|---|---|---|
| Amount of 7-12SFd-F Added (µg/ml) | Amount of Cycloheximide Added (µg/ml) | | |
| | 0 | 1 | 10 |
| | Amount of HGF Produced: % / | | |
| | Inhibitory Ratio Where Cycloheximide Non-Added Group Was Regarded as 100%: % | | |
| 0 | 100/0 | 40/60 | 40/60 |
| 1 | 124/0 | 61/51 | 39/69 |
| 10 | 216/0 | 88/59 | 53/75 |
| 100 | 265/0 | 117/56 | 74/72 |

**Table 52**

| Inhibition of Induction of HGF Production (Total in Cells and in Medium) | | | |
|---|---|---|---|
| Amount of 7-12SFd-F Added (µg/ml) | Amount of Cycloheximide Added (µg/ml) | | |
| | 0 | 1 | 10 |
| | Amount of HGF Produced: % / | | |
| | Inhibitory Ratio Where Cycloheximide Non-Added Group Was Regarded as 100%: % | | |
| 0 | 100/0 | 31/69 | 27/73 |
| 1 | 104/0 | 31/70 | 25/76 |
| 10 | 128/0 | 38/70 | 28/78 |
| 100 | 137/0 | 47/66 | 37/73 |

(3) Five-hundred microliters of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, actinomycin D (RNA synthesis inhibitor: manufactured by Sigma) was added thereto so as to have a final concentration of 0, 0.1, or 1 µg/ml, and a sample to be analyzed was further added thereto. The cells were then cultured for 24 hours. This medium was collected, and the amount of HGF in the medium was determined by using Quantikine Human Hepatocyte Growth Factor (HGF) ELISA Kit (manufactured by Funakoshi, Code. RS-0641-00). As to the amount of HGF produced, negative control was regarded as 100%. As to the inhibitory ratio, the inhibitory ratio (%) of the actinomycin D-added fraction was calculated on the basis of the amount of HGF produced when the fucoidan was added alone at each concentration. The fucoidan derived from *Kjellmaniella crassifolia* described in item (1) of Reference Example 1 was added so as to have a final concentration of 1, 10 or 100 µg/ml. As the negative control, distilled water was added in the same volume as that of the sample. Each experiment was carried out twice, and an average value was taken. As a result, as shown in Table 53, due to the addition of actinomycin D, the concentration of HGF in the medium of the fucoidan-added group was inhibited in an actinomycin D concentration-dependent manner. It was clarified from these findings that RNA synthesis was probably involved in the induction of HGF production by the fucoidan, not simply freeing of HGF from the cells.

**Table 53**

| Fucoidan Derived from *Kjellmaniella crassifolia* (µg/ml) | Amount of Actinomycin D Added (µg/ml) | | |
|---|---|---|---|
| | 0 0 | 0.1 0.1 | 1 1 |
| | Amount of HGF Produced: % / | | |
| | Inhibitory Ratio Where Actinomycin D Non-Added Group Was Regarded as 100%: % | | |
| 0 | 100/0 | 79/21 | 83/17 |
| 1 | 244/0 | 138/43 | 119/51 |
| 10 | 343/0 | 224/35 | 239/30 |
| 100 | 492/0 | 341/31 | 285/42 |

(4) Five-hundred microliters of MRC-5 cells (CCL171: manufactured by DAINIPPON PHARMACEUTICAL CO., LTD., code. 02-021) suspended in a DME medium containing 10% fetal bovine serum so as to have a concentration of 1 × 10⁵ cells/ml were added into each well of a 48-well cell culture plate. The cells were cultured at 37°C for 24 hours in the presence of 5% CO₂, and then the medium was exchanged for a DME medium containing 1% fetal bovine serum. Thereafter, actinomycin D (RNA synthesis inhibitor: manufactured by Sigma) was added thereto so as to have a final concentration of 0, 0.1, or 1 µg/ml, and a sample was further added thereto. The cells were then cultured for 24 hours. This medium was collected, and the amount of HGF in the medium was determined by using Quantikine Human Hepatocyte Growth Factor (HGF) ELISA Kit (manufactured by Funakoshi, Code. RS-0641-00). In addition, the cells were washed with PBS, and thereafter dissolved in 500 µl of a cytolytic buffer (50 mM HEPES, pH 7.4; 10 mM EDTA, 0.1% Triton x 100; 1 mM PMSF; 1 µg/ml pepstatin A and 1 µg/ml, leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium. As to the amount of HGF produced, negative control was regarded as 100%. As to the inhibitory ratio, the inhibitory ratio (%) of the actinomycin D-added fraction was calculated on the basis of the amount of HGF produced when 7-12SFd-F was added alone at each concentration. 7-12SFd-F was added so as to have a final concentration of 1,10 or 100 µg/ml. As the negative control, distilled water was added in the same volume as that of the sample. Each experiment was carried out three times, and an average value was taken. As a result, as shown in Tables 54 and 55, due to the addition of actinomycin D, both the amount of HGF in the medium and the total amount of HGF in the cells and the medium of the 7-12SFd-F-added group were inhibited in an actinomycin D concentration-dependent manner. It was clarified from these findings that RNA synthesis was probably involved in the induction of HGF production by 7-12SFd-F, not simply freeing of HGF from the cells.

**Table 54**

| Inhibition of Induction of HGF Production (in Medium) | | | |
|---|---|---|---|
| 7-12SFd-F (µg/ml) | Amount of Actinomycin D Added (µg/ml) | | |
| | 0 | 0.1 | 1 |
| | Amount of HGF Produced: % / | | |
| | Inhibitory Ratio Where Actinomycin D Non-Added Group Was Regarded as 100%: % | | |
| 0 | 100/0 | 76/24 | 80/20 |
| 1 | 130/0 | 95/27 | 96/26 |
| 10 | 225/0 | 182/19 | 152/32 |
| 100 | 295/0 | 212/28 | 187/48 |

**Table 55**

| Inhibition of Induction of HGF Production (Total in Cells and in Medium) | | | |
|---|---|---|---|
| 7-12SFd-F (µg/ml) | Amount of Actinomycin D Added (µg/ml) | | |
| | 0 | 0.1 | 1 |
| | Amount of HGF Produced: % / | | |
| | Inhibitory Ratio Where Actinomycin D Non-Added Group Was Regarded as 100%: % | | |
| 0 | 100/0 | 61/39 | 59/41 |
| 1 | 98/0 | 64/35 | 61/38 |
| 10 | 113/0 | 83/27 | 71/37 |
| 100 | 126/0 | 91/28 | 81/36 |

### Example 7

(1) A partial hepatecomy of 7-week old male Wistar rats was carried out by surgical treatment as follows. Specifically, the rats were abdominally incised under ether anesthesia, and about a 30% portion of the liver was excised after the root of blood vessel was knotted with a surgical suture. The abdominally incised section was sutured with a suture needle.

The fucoidan derived from *Kjellmaniella crassifolia* described in item (1) of Reference Example 1 was intraperitoneally administered at a 12-hour interval, the administration immediately after the excision counted as a first. The control group was intraperitoneally administered with physiological saline.

Blood was collected from abdominal aorta of the rats under anesthesia at 24 hours or 72 hours after hepatecomy, and plasma with 0.1% disodium ethylenediaminetetraacetate was separated by centrifugation. The amount of HGF in the plasma was determined by using HGF ELISA Kit (manufactured by Institute of Immunology Co. Ltd.).

The results are shown in Table 56. The numerical figures in the table are expressed by an average value ± standard error, and inside parentheses ( ) show the number of rats per one group. Also, the asterisks * in the table mean that there is a significant difference at a significance level of 5% or less, as compared to the control group.

**Table 56**

| Administered Sample | Dosage (mg/kg) | HGF Concentration in Plasma (ng/ml) | |
|---|---|---|---|
| | | 24 hours | 72 hours |
| Physiological Saline (Control Group) | | 0.28 ± 0.04 (4) | 0.30 ± 0.02 (3) |
| Fucoidan Derived | 0.1 | 0.40 ± 0.12 (4) | 0.52 ± 0.06 (5)* |
| from *Kjellmaniella crassifolia* | 1 | 0.65 ± 0.18 (4) | 0.64 ± 0.09 (2)* |

The group administered with the fucoidan had a tendency of elevating the amount of HGF in the plasma 24 hours after hepatecomy, and showed a significant elevation after 72 hours, as compared to the control group.

As described above, since the fucoidan induces HGF production, the fucoidan is useful for a rapid regeneration after surgery in hepatitic diseases requiring surgical operation, and for a functional recovery of the remaining liver.

(2) A partial hepatecomy of 7-week old male Wistar rats was carried out by surgical treatment. The rats were abdominally incised under ether anesthesia, and about a 30% portion of the liver was excised after the root of blood vessel was knotted with a surgical suture. The abdominally incised section was sutured with a suture needle. The enzymatically treated F-fucoidan prepared in item (4) of Reference Example 2 was orally administered in two separate portions, one in the morning and other in the evening, the administration immediately after the excision counted as a first. The control group was administered with physiological saline. Blood was collected from abdominal aorta of the rats under anesthesia at 24 hours after hepatecomy, and plasma with 0.1% disodium ethylenediaminetetraacetate was separated by centrifugation. The amount of HGF in the plasma was determined by using HGF ELISA Kit (manufactured by Institute of Immunology Co. Ltd.).

The results are shown in Table 57. The numerical figures in the table are expressed by an average value ± standard error, and inside parentheses () show the number of rats per one group. Also, the asterisks * in the table mean the group having a significant difference at a significance level of 1% or less, as compared to the control group.

**Table 57**

| | HGF Concentration in Plasma (ng/ml) |
|---|---|
| Group | 24 hours |
| Physiological Saline | 0.184 ± 0.33 (6) |
| Enzymatically | |
| Treated F-Fucoidan (1g/kg/day) | 0.505 ± 0.97* (5) |

The group administered with the enzymatically treated F-fucoidan showed a significant elevation 24 hours after hepatecom, as compared to the control group.

As described above, since F-fucoidan having a high 7-12SFd-F content, the fucoidan, induces HGF production, the fucoidan is useful for a rapid regeneration after surgery in hepatitic diseases requiring surgical operation, and for a functional recovery of the remaining liver.

### Example 8

Hs68 cells (ATCC CRL-1635) of a cell line of human newborn foreskin fibroblast expressing h-IGF-I, a kind of insulin-like growth factor, at a high level were cultured in a DMEM medium (manufactured by Gibco BRL) containing 10% fetal bovine serum (FBS: manufactured by Bio Whittaker) at 37°C in the presence of 5% CO₂, until the cells reached confluence in the culture vessel. The cells were suspended in the above-mentioned medium with trypsin-EDTA solution (manufactured by Bio Whittaker) so as to have a concentration of 3 × 10³ cells/well, and added into each well of a 96-well microtiter plate in a volume of 200 µl each. After culturing the cells for 5 to 7 days, the medium was discarded at a point where the cells reached almost confluence in the culture vessel. The above-mentioned medium containing Fraction I, Fraction II, or Fraction III described in item (2) of Reference Example 1 or the fucoidan derived from *Kjellmaniella crassifolia* described in item (1) of Reference Example 1 at a concentration of 0, 12.3, 37, 111, 333, 1000 or 3000 µg/ml was added in an amount of 200 µl/well. A 24-hour time course was taken, and culture supernatant was collected with the passage of time at 1, 4, 12 or 24 hours, and the induction activity for h-IGF production for the Hs68 cells was determined by using h-IGF-1 ELISA Kit (manufactured by Diagnostique). The results are shown in Tables 58 to 61. Here, the control was the case where no sample was added.

**Table 58**

| Fucoidan Derived from *Kjellmaniella crassifolia* (µg/ml) | h-IGF-1 Concentration in Medium (ng/ml) | | | |
|---|---|---|---|---|
| | 1 hour | 4 hours | 12 hours | 24 hours |
| Control | 4.3 | 2.9 | 4.1 | 4.5 |
| 12.3 | 22.9 | 14.7 | 10.5 | 11.8 |
| 37 | 17.5 | 13.9 | 10.8 | 11.4 |
| 111 | 14.6 | 13.7 | 10.3 | 7.8 |
| 333 | 13.8 | 17.6 | 9.4 | 7.7 |
| 1000 | 13.9 | 14.7 | 14.6 | 7.5 |
| 3000 | 15.9 | 14.0 | 14.0 | 7.2 |

**Table 59**

| Fraction I (µg/ml) | h-IGF-1 Concentration in Medium (ng/ml) | | | |
|---|---|---|---|---|
| | 1 hour | 4 hours | 12 hours | 24 hours |
| Control | 6.8 | 5.4 | 5.0 | 5.0 |
| 12.3 | 13.9 | 10.5 | 7.6 | 7.3 |
| 37 | 19.0 | 11.7 | 8.3 | 10.1 |
| 111 | 20.4 | 11.0 | 9.5 | 9.9 |
| 333 | 18.7 | 12.2 | 10.9 | 10.3 |
| 1000 | 17.7 | 13.2 | 12.7 | 11.3 |
| 3000 | 19.0 | 12.1 | 13.1 | 11.7 |

**Table 60**

| Fraction II (µg/ml) | h-IGF-1 Concentration in Medium (ng/ml) | | | |
|---|---|---|---|---|
| | 1 hour | 4 hours | 12 hours | 24 hours |
| Control | 4.3 | 2.9 | 4.1 | 4.5 |
| 12.3 | 18.5 | 10.2 | 6.9 | 7.0 |
| 37 | 20.1 | 9.9 | 9.2 | 9.2 |
| 111 | 20.0 | 11.1 | 9.8 | 8.9 |
| 333 | 16.3 | 12.7 | 9.7 | 9.2 |
| 1000 | 17.0 | 12.2 | 10.0 | 8.8 |
| 3000 | 17.9 | 11.3 | 10.0 | 7.6 |

**Table 61**

| Fraction III (µg/ml) | h-IGF-1 Concentration in Medium (ng/ml) | | | |
|---|---|---|---|---|
| | 1 hour | 4 hours | 12 hours | 24 hours |
| Control | 4.3 | 2.9 | 4.1 | 4.5 |
| 12.3 | 16.5 | 10.9 | 8.5 | 8.6 |
| 37 | 16.7 | 10.2 | 11.1 | 8.4 |
| 111 | 11.8 | 11.5 | 8.6 | 6.9 |
| 333 | 9.6 | 11.2 | 8.0 | 5.9 |
| 1000 | 7.9 | 10.4 | 10.6 | 6.6 |
| 3000 | 7.1 | 9.4 | 9.7 | 6.2 |

As shown in Tables 58 to 61, each of the fucoidan derived from *Kjellmaniella crassifolia*, Fraction I, Fraction II and Fraction III showed induction activity for h-IGF-1 production. The induction activity for h-IGF-1 production was a maximal value 1 hour after the addition of 12 to 100 µg/ml of the sample. Here, there was found no toxicity or growth-suppressing activity for the Hs68 cells in each sample. The same level of the induction activity for h-IGF-1 production was also found for other acidic polysaccharides, decomposed products thereof, acidic oligosaccharides, acidic monosaccharides, and salts thereof described in Reference Examples.

### Example 9

Rat fibroblasts L-M (ATCC CCL-1.2) were suspended in an M199 medium (manufactured by ICN) containing 0.5% bactopepton (manufactured by Difco) so as to have a concentration of 1.5 × 10⁵ cells/ml, and the suspension was added into each well of a 96-well plate in an amount of 0.1 ml and the cells were aseptically cultured.

After culturing the cells for 3 days, the medium was removed therefrom, and substituted with an M199 medium containing 0.5% bovine serum albumin (manufactured by Sigma). The fucoidan derived from *Kjellmaniella crassifolia* described in item (1) of Reference Example 1 was added thereto so as to have a final concentration of 0, 62.5, 250 or 1000 µg/ml, and the cells were cultured for 24 hours. As a control, distilled water-added one was used. After the termination of the culture, the concentration of NGF in the culture medium was determined by enzyme immunoassay method (NGF Emax Immuno Assay System: manufactured by Promega). As to the amount of NGF produced, the amount of NGF produced of the control was regarded as 100%. Each experiment was carried out twice, and an average value was taken. The results are shown in Table 62. As shown in Table 62, the fucoidan derived from *Kjellmaniella crassifolia* promoted the production of nerve growth factor of L-M cells in a concentration-dependent manner. Further, its fractions also showed similar activity. In addition, the same level of the induction action for NGF production was also found for other acidic polysaccharides, decomposed products thereof, acidic oligosaccharides, acidic monosaccharides, and salts thereof described in Reference Examples.

**Table 62**

| Sample | Concentration (µg/ml) | Increase in Amount of NGF Produced (%) |
|---|---|---|
| Fucoidan | | |
| Derived from *Kjellmaniella crassifolia* | 62.5 | 117 |
| | 250 | 166 |
| | 1000 | 179 |
| (The amount of NGF produced of control was 155 pg/ml.) | | |

Similarly, the promoting activity of the production of nerve growth factor for Fraction I, Fraction II and Fraction III described in item (2) of Reference Example 1 was determined, and the activity was found in each fraction. The results are shown in Table 63. In addition, the same level of the induction action for NGF production was also found for other acidic polysaccharides, decomposed products thereof, acidic oligosaccharides, acidic monosaccharides, and salts thereof described in Reference Examples.

**Table 63**

| Sample | Concentration (µg/ml) | Increase in Amount of NGF Produced (%) |
|---|---|---|
| Fraction I | 250 | 505.6 |
| | 500 | 619.6 |
| | 1000 | 806.5 |
| Fraction II | 250 | 664.5 |
| | 500 | 864.5 |
| | 1000 | 1137.4 |
| Fraction III | 250 | 1021.1 |
| | 500 | 1187.0 |
| | 1000 | 1265.0 |
| (The amount of NGF produced of control was 50.03 pg/ml.) | | |

### Example 10

(1) Male C3H/He mice were purchased from Nippon SLC and used for an experiment from 5 week-old after preliminary rearing. The fucoidan derived from *Kjellmaniella crassifolia* prepared in item (1) of Reference Example 1 was suspended and dissolved in ethanol so as to have a concentration of 3%, and the resulting solution was applied onto the backside of the mice in an amount of 200 µl per mouse. As to the control group, ethanol was similarly applied. The administration was once per day, for 8 consecutive days. Skin was peeled off on ninth day after the initiation of administration, and HGF activity in the skin was determined by ELISA Kit (Institute of Immunology Co. Ltd.).

The results are shown in Table 64. The numerical figures in the table are expressed by an average value ± standard error of 5 cases.

HGF activity extracted from the skin clearly increased in the fucoidan-applied group, as compared to that of the control group, so that the induction action for HGF production was found by the application of the fucoidan.

**Table 64**

| | HGF Activity in Skin (ng/g tissue) |
|---|---|
| Control Group (N=5) | 16.31±2.86 |
| Fucoidan-Applied Group (N=5) | 104.46±4.05 |
| average value ± standard deviation | |

(2) Twenty-five adult women of ages 20 to 35 years old were subjected to a blind functional test where the lotion of the present invention described in item (1) of Example 26 given below was compared with the control lotion containing no fucoidan. As a result, the number of persons who judged "more effective" is shown in Table 65.

**Table 65**

| | Moistness of Skin | Smoothness on Skin | Liveliness of Skin |
|---|---|---|---|
| Lotion of Present Invention | 21 | 19 | 16 |
| Lotion of Control | 5 | 6 | 9 |

As a result, it was shown that the lotion of the present invention formulated with the fucoidan was excellent in all of moistness, smoothness and liveliness of skin, due to the induction action of HGF production by the fucoidan.

### Example 11

(1) Ninety-eight milligrams of F-fucoidan prepared according to the method described in item (2) of Reference Example 1 was dissolved in 5 ml of DMSO, and 980 mg of piperidine sulfate was added thereto at room temperature. Thereafter, the resulting mixture was stirred at 80°C for 2 hours. After cooling the reaction solution, the reaction solution was dialyzed with a dialysis membrane having an excluding molecular weight of 1000 for 2 days. The resulting solution inside the dialysis membrane was applied onto a cation exchange column [Amberlite IRA-120 (Na⁺)], and thereafter dried under reduced pressure, to give 98 mg of highly-sulfated F-fucoidan.
(2) Thirty-four milligrams of 7-12SFd-F prepared according to the method described in Reference Example 2 was dissolved in 4 ml of DMSO, and thereafter the same steps as those in item (1) of Example 11 were carried out, to give 98 mg of highly-sulfated 7-12SFd-F.
(3) The induction activity for HGF production for each of the highly-sulfated F-fucoidan (Sample (1)) prepared in item (1) of Example 11, the highly-sulfated 7-12SFd-F (Sample (2)) prepared in item (2) of Example 11, F-fucoidan (Sample (3)) prepared in item (2) of Reference Example 1, and 7-12SFd-F (Sample (4)) prepared in Reference Example 2 was studied in the same manner as that in Example 1. Each of the samples was added so as to have a final concentration of 1, 10 or 100 µg/ml. As a control, distilled water was added in the same volume as that of the sample.

The results are shown in Table 66. In Table 66, the amount of HGF produced of the control was regarded as 100%. Each experiment was carried out twice, and an average value was taken. As shown in Table 66, each of Samples (1) to (4) induced the production of HGF. Further, in the highly-sulfated compounds, the induction activity for HGF production increased more than those which were not subjected to highly-sulfating treatment. It was clarified from the above that the induction activity for HGF production of naturally occurring sulfated saccharide is enhanced, as compared to the saccharide itself by further subjecting the saccharide to sulfating treatment.

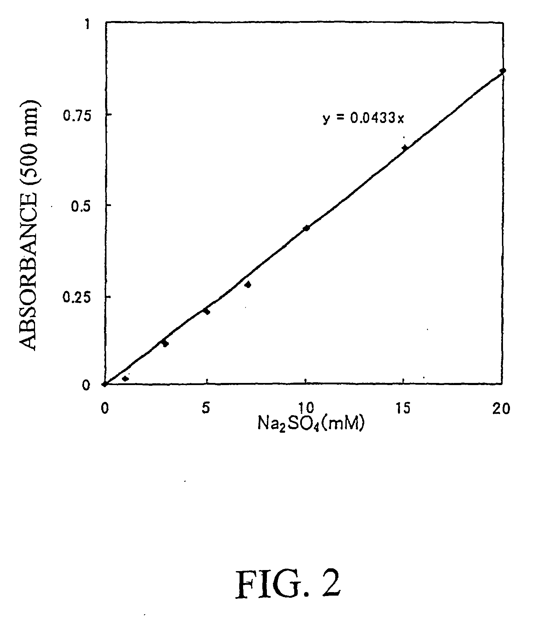

Here, the sulfate content was quantified by the steps of heating 0.2 ml of a 1 N HCl solution of each sample (1 to 10 mg/ml) at 105°C for 4 hours, adding to 0.1 ml of the solution 1.9 ml of 0.1 N HCl solution and 0.25 ml of 1% barium chloride - 0.5% gelatin solution, allowing the mixed solution to stand for 20 minutes, and thereafter determining absorbance at 500 nm. A calibration curve was prepared by using a 1 N HCl solution containing 0, 1, 3, 5, 7, 10, 15, or 20 mM sodium sulfate as a standard sample, and the sulfate content (calculated as SO₃) of each sample was obtained from this calibration curve. The calibration curve is shown in Figure 2, and the sulfate content of each sample is shown in Table 67.

**Table 66**

| Concentration (µg/ml) | Amount of HGF Produced (%) | | | |
|---|---|---|---|---|
| | Sample (1) | Sample (2) | Sample (3) | Sample (4) |
| 1 | 346 | 192 | 312 | 192 |
| 10 | 715 | 214 | 493 | 229 |
| 100 | 794 | 499 | 598 | 355 |
| (The amount of HGF produced of control was 8.15 ng/ml.) | | | | |

**Table 67**

| Sample | Sulfate Content (%, Calculated as SO₃) |
|---|---|
| F-Fucoidan | 40 |
| Highly-sulfated F-Fucoidan | 47 |
| 7-12SFd-F | 40 |
| Highly-sulfated 7-12SFd-F | 48 |

### Example 12

NHDF cells (normal human dermal fibroblasts, manufactured by Bio Whittaker) cultured in a DMEM medium containing 10% fetal bovine serum were suspended in the DMEM medium containing 10% fetal bovine serum so as to have a concentration of 1 × 10⁵ cells/ml, and added into each well of a 48-well cell culture plate in a volume of 500 µl each. The cells were cultured for 24 hours. Thereafter, the medium was exchanged for a DMEM medium containing 1% fetal bovine serum, and thereto were added 10 nM tetradecanoylphorbol 13-acetate (TPA: manufactured by Gibco BRL) and each sample. The cells were cultured for 20 hours after the addition, and thereafter the medium was collected, and the amount of HGF in the medium was determined by using Quantikine Human Hepatocyte Growth Factor (HGF) ELISA Kit (manufactured by Funakoshi, Code. RS-0641-00). The amount of HGF produced of negative control was regarded as 100%. As to the sample, the fucoidan described in item (1) of Reference Example 1 was added so as to each have a final concentration of 1, 10 or 100 µg/ml. Heparin was added so as to have a final concentration of 1 or 10 µg/ml. As a negative control, distilled water was added in the same volume as that of the sample. The culture for the induction experiment was performed by adding 10 nM TPA at the same time with the addition of the sample. Each experiment was carried out three times, and an average value was taken. The results are shown in Table 68. In the all groups of fucoidan added-cells, the amount of HGF produced increased more significantly, as compared to that of the distilled water-added negative control. Further, the amount of HGF increased more significantly, as compared to that of the heparin-added group. It was shown from the above finding that the fucoidan described in item (1) of Reference Example 1 possesses activity of promoting the production of HGF higher than that of heparin of which the induction of HGF had been confirmed so far.

**Table 68**

| Concentration (µg/ml) | Heparin | Fucoidan |
|---|---|---|
| 0 | 100 | 100 |
| 1 | 950 | 1140 |
| 10 | 2170 | 2470 |
| 100 | - | 2770 |
| (The amount of HGF produced of control was 0.20 ng/ml.) | | |

### Example 13

Hs68 cells (human newborn foreskin fibroblasts: manufactured by DAINIPPON PHARMACEUTICAL CO., LTD., ATCC CRL-1635) cultured in a DMEM medium containing 10% fetal bovine serum were suspended in the DMEM medium containing 10% fetal bovine serum so as to have a concentration of 1 × 10⁵ cells/ml, and added into each well of a 48-well cell culture plate in a volume of 500 µl each. The cells were cultured for 24 hours. Thereafter, the medium was exchanged for a DMEM medium containing 1% fetal bovine serum, and thereto were added 10 nM TPA and each sample. Also, the same steps were carried out for the group added with only the sample without adding TPA. The medium was collected, and the amount of HGF in the medium was determined by using Quantikine Human Hepatocyte Growth Factor (HGF) ELISA Kit. Further, the cells were washed with PBS, and thereafter dissolved in 500 µl of a cytolytic buffer (50 mM HEPES, pH 7.4; 10 mM EDTA; 0.1% Triton X100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cell was determined in the same manner as that of the concentration of HGF in the medium. 7-12SFd-F prepared in item (3) of Reference Example 2 was added so as to have a final concentration of 0.1, 1, 10 or 100 µg/ml. As a negative control, distilled water was added in the same volume as that of the sample. Each experiment was carried out three times, and an average value was taken. The results are shown in Tables 69 to 71. In the TPA non-added group, a remarkable production of HGF could not be found. However, in a case where TPA was added, the amount of HGF in the cells decreased in a 7-12SFd-F concentration-dependent manner, and the amount of HGF in the medium and the total amount of HGF increased in a 7-12SFd-F concentration-dependent manner, and further the total amount of HGF significantly increased as compared to the non-added control. In addition, the increase in the amount of HGF when mRNA is increased as described above was very remarkable, as compared to the case where the amount of mRNA is small with no TPA treatment. It was clarified from the above that 7-12SFd-F remarkably promotes the freeing and production of HGF in a case where transcription of mRNA is promoted, so that a large amount of HGF is required.

**Table 69**

| Amount of HGF in Hs68 / 7-12SFD-F Medium (pg/Well) | | |
|---|---|---|
| 7-12SFd-F (µg/ml) | TPA Non-Added | 10 nM TPA |
| 0 | N.D. | 65.54 |
| 0.1 | N.D. | 71.04 |
| 1 | N.D. | 99.25 |
| 10 | N.D. | 226.14 |
| 100 | N.D. | 260.49 |
| N.D. means the detection limit or less. | | |

**Table 70**

| Amount of HGF in Hs68 / 7-12SFD-F Cells (pg/Well) | | |
|---|---|---|
| 7-12SFd-F (µg/ml) | TPA Non-Added | 10 nM TPA |
| 0 | 98.62 | 155.65 |
| 0.1 | 87.60 | 151.99 |
| 1 | 62.47 | 142.17 |
| 10 | N.D. | 120.70 |
| 100 | N.D. | 95.67 |
| N.D. means the detection limit or less. | | |

**Table 71**

| Total Amount of HGF in Hs68 / 7-12SFD-F Cells (pg/Well) | | |
|---|---|---|
| 7-12SFd-F (µg/ml) | TPA Non-Added | 10 nM TPA |
| 0 | | 221.19 |
| 0.1 | | 223.02 |
| 1 | | 241.42 |
| 10 | | 346.84 |
| 100 | | 356.05 |

### Example 14

MRC-5 cells (CCL 171: manufactured by DAINIPPON PHARMACEUTICAL CO., LTD., code. 02-021) cultured in a DMEM medium containing 10% fetal bovine serum were suspended in the DMEM medium containing 10% fetal bovine serum so as to have a concentration of 2.5 × 10⁵ cells/ml, and added into each well of a 6-well cell culture plate. The cells were cultured at 37°C for 24 hours in the presence of 5% CO₂. Thereafter, the medium was exchanged for a DMEM medium containing 1% fetal bovine serum, and the cells were further cultured for 22 hours. Thereafter, the medium was exchanged for each medium added with 7-12SFd-F prepared in item (3) of Reference Example 2 so as to have a final concentration of 100 µg/ml, LM Heparin (manufactured by Celsus Laboratories) so as to have a final concentration of 1 µg/ml, or prostaglandin E₁ (PGE₁) (manufactured by Wako Pure Chemicals Industries, Ltd.) dissolved in dimethyl sulfoxide (DMSO) so as to have a final concentration of 1 µM. As a medium for control group, the medium added with DMSO was used. Here, the solvent of each additive mentioned above was added so as to have a concentration of 1% in all cases. Further, the cells were cultured, and a whole RNA was extracted on Hours 0, 2, 4, 6, 8, 10, 12, and 24. In the extraction of the whole RNA, RNeasy Mini Kit (manufactured by QIAGEN) was used. RT-PCR was carried out by using RNA PCR Kit Ver. 2.1. (manufactured by Takara Shuzo Co., Ltd., R019A). Reverse transcription reaction was carried out at 30°C for 10 minutes, 42°C for 30 minutes, and 99°C for 5 minutes by using a random primer (N6) (manufactured by Takara Shuzo Co., Ltd., 3801), following subjecting the whole RNA to thermal denaturing treatment. In order to detect mRNA for HGF, the primer of SEQ ID NO: 1 of Sequence Listing was used as a sense primer, and the primer of SEQ ID NO: 2 of Sequence Listing was used as an antisense primer. The product to be amplified by the primers has a size of 415 bp. In addition, in order to carry out a semi-quantitative experiment, â-actin, a housekeeping gene, was also detected. The primer of SEQ ID NO: 3 of Sequence Listing was used as a sense primer, and the primer of SEQ ID NO: 4 of Sequence Listing was used as an antisense primer. The product to be amplified by the primers has a size of 275 bp. PCR was carried out by using PJ9600 (manufactured by Perkin-Elmer). PCR was carried out in 24 cycles of denaturation at 94°C for 30 seconds, annealing at 59°C for 30 seconds, and extension reaction at 72°C for 60 seconds, following denaturation at 94°C for 2 minutes. After the reaction, the reaction mixture was subjected to electrophoresis on a 2% agarose gel and ethidium bromide staining, and the gel was observed under UV irradiation. In all of the samples, mRNA of HGF was detected. As compared to that of the control, the induction of mRNA by PGE₁ was found, but the induction of mRNA by 7-12SFd-F or LM Heparin was not found. It was clarified from the above that the promotion of mRNA transcription of HGF does not take place by the addition of 7-12SFd-F or LM Heparin under the conditions such that mRNA of HGF is always transcribed, and each of 7-12SFd-F and LM Heparin does not cause induction of excessive HGF production.

### Example 15

The same procedures as those in Example 13 were carried out except for using NHDF cells (normal human dermal fibroblasts, manufactured by Bio Wittaker) in place of HS68 cells used in Example 13 to study for the induction activity for HGF production by 7-12SFd-F in NHDF cells. The results are shown in Tables 72 to 74.

In the TPA non-added group, the amount of HGF in the cells decreased in a 7-12SFd-F concentration-dependent manner, each of the amount of HGF in the medium and the total amount of HGF increased in a 7-12SFd-F concentration-dependent manner, and further the total amount of HGF significantly increased, as compared with the 7-12SFd-F non-added control. It was clarified from the above that even in a case where mRNA of HGF is little as in the TPA non-treated case, both the freeing of HGF and the synthesis of HGF on the cell surface were promoted. Further, in a case where TPA was added, the amount of HGF in the cells decreased in a 7-12SFd-F concentration-dependent manner, each of the amount of HGF in the medium and the total amount of HGF increased in a 7-12SFd-F concentration-dependent manner, and further the total amount of HGF significantly increased, as compared with the 7-12SFd-F non-added control. Also, the increase in the amount of HGF in a case where mRNA was increased was very remarkable, as compared to a case where the amount of mRNA was small with TPA non-added. It was clarified from the above that 7-12SFd-F promotes the freeing and the production of HGF to some extent even in a case where the amount of mRNA is small, and remarkably promotes the freeing and the production of HGF in a case where the transcription of mRNA is promoted due to the necessity of a large amount of HGF.

**Table 72**

| Amount of HGF in NHDF / 7-12SFD-F Medium (ng/Well) | | |
|---|---|---|
| 7-12SFd-F (µg/ml) | TPA Non-Added | 10 nM TPA |
| 0 | N.D. | 0.39 |
| 1 | 0.05 | 0.68 |
| 10 | 0.18 | 1.22 |
| 100 | 0.385 | 1.625 |
| N.D. means the detection limit or less. | | |

**Table 73**

| Amount of HGF in NHDF / 7-12SFD-F Cells (ng/Well) | | |
|---|---|---|
| 7-12SFd-F (µg/ml) | TPA Non-Added | 10 nM TPA |
| 0 | 0.185 | 0.24 |
| 1 | 0.145 | 0.19 |
| 10 | 0.075 | 0.12 |
| 100 | 0.025 | 0.055 |

**Table 74**

| Total Amount of HGF in NHDF / 7-12SFD-F Cells (ng/Well) | | |
|---|---|---|
| 7-12SFd-F (µg/ml) | TPA Non-Added | 10 nM TPA |
| 0 | | 0.63 |
| 1 | 0.195 | 0.87 |
| 10 | 0.255 | 1.335 |
| 100 | 0.41 | 1.68 |

### Example 16

NHDF cells (normal human dermal fibroblasts) cultured in a DMEM medium containing 10% fetal bovine serum were suspended in the DMEM medium containing 10% fetal bovine serum so as to have a concentration of 2.5 × 10⁵ cells/ml, and added into each well of a 6-well cell culture plate in a volume of 2 ml each. The cells were cultured at 37°C for 24 hours in the presence of 5% CO₂. Thereafter, the medium was exchanged for a DMEM medium containing 1% fetal bovine serum, and thereto were added 10 nM tetradecanoylphorbol 13-acetate (TPA: manufactured by Gibco BRL) and 7-12SFd-F prepared in item (3) of Reference Example 2 each so as to have a final concentration of 100 µg/ml. Also, the same steps were carried out for the group added with only 7-12SFd-F without adding TPA. The cells were further cultured, and a whole RNA was extracted on Hours 4, 6, 8 and 10. In the extraction of the whole RNA, RNeasy Mini Kit (QIAGEN) was used. RT-PCR was carried out entirely in the same manner as that in Example 14, except for changing the cycles for PCR to 28 cycles. After the reaction, the reaction mixture was subjected to electrophoresis on a 2% agarose gel and ethidium bromide staining, and the gel was observed under UV irradiation.

As a result, although the amount of transcription of HGF mRNA is very small in the TPA non-added group, a slight increase in the amount of mRNA transcription was seen by the addition of 7-12SFd-F, after 4 hours from the addition. On the other hand, the amount of HGF mRNA remarkably increased at any time periods by the addition of TPA. In a case where TPA and 7-12SFd-F were added, as compared to a case where only TPA was added, the amount of HGF mRNA was found to increase after 4 hours from the addition, but after 6 hours from the addition, there was no difference by the addition of 7-12SFd-F. In other words, it was clarified that 7-12SFd-F remarkably promotes its mRNA transcription at the initial period where mRNA transcription is started to actively carry out due to the necessity of HGF, but thereafter its promoting effect is lost. It was clarified from the above that 7-12SFd-F induces HGF production at the instance where HGF is required, but there is no excessive induction of HGF production thereafter.

### Example 17

HL60 cells (human promyelocytic leukemia cell line) cultured in an RPMI 1640 medium containing 10% fetal bovine serum were suspended in an RPMI 1640 medium containing 1% fetal bovine serum so as to have a concentration of 5 × 10⁵ cells/ml, and added into each well of a 48-well cell culture plate in a volume of 500 µl each. There after, 10 nM TPA was added thereto, and each sample was further added, and the cells were then cultured for 24 hours. Also, the same steps were carried out for the group added with only the sample without adding TPA. The medium was collected, and the amount of HGF in the medium was determined by using the Quantikine Human Hepatocyte Growth Factor (HGF) ELISA Kit. Further, the cells were washed with PBS, and thereafter dissolved in 500 µl of a cytolytic buffer (50 mM HEPES, pH 7.4; 10 mM EDTA; 0.1% Triton X100; 1 mM PMSF; 1 µg/ml pepstatin A and 1 µg/ml leupeptin). Further, in order to completely dissolve the cells, the mixture was subjected to sonication treatment, and then centrifuged, to give a supernatant (cell extract). The amount of HGF in the cells was determined in the same manner as that of the concentration of HGF in the medium. 7-12SFd-F prepared in item (3) of Reference Example 2 was added so as to have a final concentration of 1, 10 or 100 µg/ml. As a negative control, distilled water was added in the same volume as that of the sample. Each experiment was carried out three times, and an average value was taken. The results are shown in Tables 75 to 77.

In the TPA non-added group, there was no change in the amount of HGF in the cells according to the concentration of 7-12SFd-F. There was also no remarkable change in the amount of HGF in the medium according to the concentration of 7-12SFd-F, even though some increase was seen at 100 µg/ml. In addition, in a case where TPA was added, although there was no change in the amount of HGF in the cells according to the concentration of 7-12SFd-F, the entire values were low. On the other hand, the amount of HGF in the medium and the total amount of HGF remarkably increased in a 7-12SFd-F concentration-dependent manner, and further the total amount of HGF significantly increased as compared to the 7-12SFd-F non-added control. In addition, the increase in the amount of HGF when mRNA was increased as described above was very remarkable, as compared to the case where the amount of mRNA was small with no TPA treatment. It was clarified from the above that 7-12SFd-F remarkably promotes the freeing and the production of HGF in a case where the transcription of mRNA is promoted due to the necessity of a large amount of HGF.

**Table 75**

| Amount of HGF in HL60 / 7-12SFD-F Medium (pg/Well) | | |
|---|---|---|
| 7-12SFd-F (µg/ml) | TPA Non-Added | 10 nM TPA |
| 0 | 76.74 | 261.60 |
| 1 | 67.10 | 295.94 |
| 10 | 78.54 | 464.55 |
| 100 | 162.85 | 843.84 |

**Table 76**

| Amount of HGF in HL60 / 7-12SFD-F Cells (pg/Well) | | |
|---|---|---|
| 7-12SFd-F (µg/ml) | TPA Non-Added | 10 nM TPA |
| 0 | 294.12 | 83.37 |
| 1 | 276.67 | 89.39 |
| 10 | 236.30 | 67.09 |
| 100 | 257.04 | 85.77 |

**Table 77**

| Total Amount of HGF in HL60 / 7-12SFD-F Cells (pg/Well) | | |
|---|---|---|
| 7-12SFd-F (µg/ml) | TPA Non-Added | 10 nM TPA |
| 0 | 370.85 | 344.97 |
| 1 | 343.77 | 385.32 |
| 10 | 314.84 | 531.64 |
| 100 | 420.26 | 929.60 |

### Example 18

HL60 cells (human promyelocytic leukemia cell line) cultured in an RPMI 1640 medium containing 10% fetal bovine serum were suspended in an RPMI 1640 medium containing 1% fetal bovine serum so as to have a concentration of 5 × 10⁵ cells/ml, and added into each well of a 6-well cell culture plate in a volume of 2 ml each. Thereafter, 10 nM TPA and 7-12SFd-F prepared in item (3) of Reference Example 2 were added each so as to have a final concentration of 100 µg/ml. Also, the same steps were carried out for the group added with only 7-12SFd-F without adding TPA. The cells were further cultured, and a whole RNA was extracted on Hours 4, 6, 8, and 10. In the extraction of the whole RNA, RNeasy Mini Kit (manufactured by QIAGEN) was used. RT-PCR was carried out entirely in the same manner as that in Example 14, except for changing the cycles for PCR to 32 cycles. After the reaction, the reaction mixture was subjected to electrophoresis on a 2% agarose gel and ethidium bromide staining, and the gel was observed under UV irradiation.

As a result, although the amount of transcription of HGF mRNA was very small in the TPA non-added group, a slight increase in the amount of HGF mRNA was seen by the addition of 7-12SFd-F, after 4 hours from the addition. On the other hand, the amount of HGF mRNA remarkably increased at any time periods by the addition of TPA. In a case where TPA and 7-12SFd-F were added, as compared to a case where only TPA was added, it was clarified that the amount of HGF mRNA increased after 4 hours from the addition, but after 6 hours from the addition, there was no difference by the addition of 7-12SFd-F. In other words, it was clarified that 7-12SFd-F remarkably promotes its mRNA transcription at the initial period where the mRNA transcription was started to actively carry out due to the necessity of HGF, but its promoting effect is lost thereafter. It was clarified from the above that 7-12SFd-F induces HGF production at the instance where HGF is required, but there is no excessive induction of HGF production thereafter.

### Example 19

(1) Commercially available *Chrysanthemum coronarium* was lyophilized, to give a lyophilized product of *Chrysanthemum coronarium.* Ten grams of a powder prepared by powdering this lyophilized product of *Chrysanthemum coronarium* was suspended in 100 ml of chloroform, and a step of filtering to collect an insoluble fraction was repeated three times. Thereafter, the insoluble fraction was suspended in 100 ml of ethanol, and a step of filtering to collect an insoluble fraction was repeated three times. Ethanol was completely removed from the insoluble fraction obtained by the above step, and the residue was suspended in 100 ml of distilled water. This suspension was kept at 60°C for 1 hour, and thereafter filtered. Ethanol was added to the filtrate in an amount of 2.5 times by volume of the filtrate, and the mixture was cooled to -20°C. Thereafter, the mixture was centrifuged at a low temperature, to give a precipitate. This precipitate was dissolved in distill water, and the solution was lyophilized, to give *a Chrysanthemum coronarium* extract, a powdery fraction containing sugar.
(2) The induction activity for HGF production for the *Chrysanthemum coronarium* extract prepared in item (1) of Example 19 was studied in the same manner as that in item (1) of Example 1. The sample was added so as to have a final concentration of 1, 10 or 100 µg/ml. As a negative control, distilled water was added in the same volume as that of the sample. As to the amount of HGF produced, negative control was regarded as 100%. The results are shown in Table 78. Each experiment was carried out twice, and an average value was taken. As shown in Table 78, the *Chrysanthemum coronarium* extract induced HGF production.

**Table 78**

| *Chrysanthemum coronarium* extract (µg/ml) | Amount of HGF Produced (%) |
|---|---|
| 1 | 125 |
| 10 | 148 |
| 100 | 314 |
| (The amount of HGF produced of control was 8.21 ng/ml.) | |

### Example 20

The induction activity for HGF production for the supernatant fraction of *Altemisia princeps pampan* prepared in item (5) of Reference Example 13 was studied in the same manner as that in item (1) of Example 1, except that the supernatant fraction of *Altemisia princeps pampan* was added in an amount of one thousandth by volume of the medium. The results are shown in Table 79. As a result, it was clarified that the *Altemisia princeps pampan* extract has induction activity for HGF production even in the fraction which is not precipitated by the ethanol precipitation. It was deduced that the low molecular fraction which is not precipitated by the ethanol precipitation also has the activity.

**Table 79**

| Amount of Supernatant Fraction of *Altemisia princeps pampan* Added | Amount of HGF Produced (%) |
|---|---|
| 0 | 100 |
| One Thousandth Added | 463 |
| (The amount of HGF produced of control was 4.31 ng/ml) | |

### Example 21

(1) Fifty grams of dried leaves of *Altemisia princeps pampan* (sold by Sakamoto Kanpodo) were placed in a homogenizer (manufactured by NIPPON SEIKI CO., LTD.), and 500 ml of acetone was added thereto. The resulting mixture was homogenized at 8000 rpm for 10 minutes, and the homogenate was filtered with a filter paper, to give a residue. The above-described steps were carried out twice, and 100 g of the resulting residue of the leaves of *Altemisia* *princeps pampan* was placed in a homogenizer, and 500 ml of acetone was added thereto. The resulting mixture was homogenized at 8000 rpm for 10 minutes, and the homogenate was filtered with a filter paper, to give a residue. The steps were repeated 4 times, to give an acetone-washed residue. The acetone-washed residue was washed 4 times with 90% ethanol, and 4 times with 80% ethanol, in the same manner as that in the acetone-washing, to give an ethanol-washed residue. All of the above steps were carried out from the beginning one more time, to altogether give 200 g of an ethanol-washed residue of the leaves of *Altemisia princeps pampan.*

(2) Ten liters of a 30 mM phosphate buffer (pH 8.0) containing 100 mM sodium chloride and 10% ethanol was added to the ethanol-washed residue, and the mixture was stirred at room temperature for 19 hours. This mixture was filtered with a filter paper, to give a crude extract (filtrate). The crude extract obtained was concentrated to a volume of 2 liters with an ultrafilter equipped with holofiber having an excluding molecular weight of 10000. Thereafter, the concentrate was ultrafiltered with 20 liters of 100 mM sodium chloride containing 10% ethanol added. Subsequently, the filtered solution was concentrated to a volume of 668 ml, and 1 g of activated carbon was placed therein, and the resulting mixture was stirred at room temperature for 30 minutes, and then centrifuged at 10000 rpm for 40 minutes to remove activated carbon. A trace amount of the activated carbon remaining in the supernatant was filtered out with a filter No. 5c. The activated carbon-treated solution was taken in an amount of 66.8 ml, and sufficiently dialyzed with distilled water, and thereafter lyophilized, to give 670 mg of a dried product. This dried product was named a macromolecular fraction of *Altemisia princeps pampan* (YPS). The remaining 601.2 ml of the activated carbon-treated solution was placed in the ultrafilter, and the solvent was substituted with a 10 mM imidazole-hydrochloric acid buffer (pH 7.0) containing 10% ethanol and 50 mM sodium chloride, to give a solvent-substituted macromolecular fraction of *Altemisia princeps pampan.*

(3) The solvent-substituted macromolecular fraction of *Altemisia princeps pampan* was applied onto a DEAE-Cellulofine A-800 column (Ö 4.05 x 37.8 cm) equilibrated with a 10 mM imidazole-hydrochloric acid buffer (pH 7.0) containing 10% ethanol and 50 mM sodium chloride, and the column was washed with 1200 ml of the same buffer. Thereafter, the elution was carried out on a gradient of from 0.1 M (1000 ml) to 2 M (1000 ml) sodium chloride. The eluate was fractionated at 30 ml per fraction. Of the eluted fractions, Fraction Nos. 13 to 33 were named a macromolecular fraction-I of *Altemisia princeps pampan* leaf (YPS-I), Fraction Nos. 69 to 78 were named a macromolecular fraction-II of *Altemisia princeps pampan* leaf (YPS-II), and Fraction Nos. 79 to 137 were named a macromolecular fraction-I of *Altemisia princeps pampan* leaf (YPS-III). Each of YPS-I, YPS-II, and YPS-III was sufficiently dialyzed against distilled water and lyophilized. Each of the lyophilized products was obtained in an amount of 530 mg, 420 mg, and 380 mg.

(4) In order to further fractionate YPS-III, 200 mg of YPS-III was dissolved in 50 ml of a 5 mM imidazole-hydrochloric acid buffer (pH 8.0) containing 4 M sodium chloride, and the resulting solution was applied onto a Phenyl-Cellulofine column (Ö 3.1 x 14.3 cm) equilibrated with the same buffer. The column was washed with 200 ml of the same buffer, and thereafter the elution was carried out with 200 ml each of a 5 mM imidazole-hydrochloric acid buffer (pH 8.0) containing 1 M sodium chloride, distilled water, and ethanol.

The eluate was fractionated at 10 ml per fraction. Of the eluted fractions, Fraction Nos. 1 to 32 were named a macromolecular fraction-III-1 of *Altemisia princeps pampan* leaf (YPS-III-1), Fraction Nos. 33 to 53 were named a macromolecular fraction-III-2 of *Altemisia princeps pampan* leaf (YPS-III-2), and Fraction Nos. 54 to 66 were named a macromolecular fraction-III-3 of *Altemisia princeps pampan* leaf (YPS-III-3). Each of YPS-III-1, YPS-III-2, and YPS-III-3 was sufficiently dialyzed against distilled water and lyophilized. Each of the lyophilized products was obtained in an amount of 20.11 mg, 32.59 mg, and 113.75 mg.

(5) The induction activity for HGF production for each of the fractions of *Altemisia princeps pampan* extracts, YPS (Sample (1)), YPS-I (Sample (2)), YPS-II (Sample (3)) and YPS-III (Sample (4)) each prepared in item (2) and item (3) of Example 21 was studied in the same manner as that in item (1) of Example 1. The results are shown in Table 80. As a result, these fractions of *Altemisia princeps pampan* extracts exhibited induction activity for HGF production.

**Table 80**

| Sample | Concentration (µg/ml) | Amount of HGF Produced (%) |
|---|---|---|
| Sample (1) | 0 | 100 |
| | 1 | 214 |
| | 10 | 267 |
| | 100 | 215 |
| Sample (2) | 0 | 100 |
| | 1 | 121 |
| | 10 | 113 |
| | 100 | 260 |
| Sample (3) | 0 | 100 |
| | 10 | 129 |
| | 100 | 243 |
| Sample (4) | 0 | 100 |
| | 1 | 232 |
| | 10 | 323 |
| | 100 | 272 |
| (The amount of HGF produced of control was 8.43 ng/ml.) | | |

(6) The induction activity for HGF production for each of the fractions of *Altemisia princeps pampan* extracts, YPS-III-1 (Sample (1)), YPS-III-2 (Sample (2)), and YPS-III-3 (Sample (3)) each prepared in item (4) of Example 21 was studied in the same manner as that in item (1) of Example 1. The results are shown in Table 81. As a result, these fractions of *Altemisia princeps pampan* extracts exhibited induction activity for HGF production.

**Table 81**

| Added Amount (µg/ml) | Amount of HGF Produced (%) | | |
|---|---|---|---|
| | Sample (1) | Sample (2) | Sample (3) |
| 0 | 100 | 100 | 100 |
| 1 | 188 | 290 | 247 |
| 10 | 181 | 304 | 217 |
| 100 | 348 | 295 | 243 |
| (The amount of HGF produced of control was 8.26 ng/ml.) | | | |

### Example 22

Thirty grams of the fucoidan derived from *Kjellmaniella crassifolia* described in item (1) of Reference Example 1 was suspended in 12 L of distilled water at room temperature with stirring for 30 minutes. This suspension was centrifuged at 10000 x g for 40 minutes, and its supernatant was collected. This supernatant was subjected to sterile filtration with a membrane filter (0.22 µm) (manufactured by Millipore Corporation), to give 21.4 g of a lyophilized product. This lyophilized product was referred to as Takara Kombu Fucoidan Bf (hereinafter referred to "Fucoidan Bf").

The induction activity for HGF production for Fucoidan Bf (Sample (1)) was studied in the same manner as that in item (1) of Example 1. The results are shown in Table 82. Each experiment was carried out twice, and an average value was taken. As a result, Fucoidan Bf exhibited induction activity for HGF production.

**Table 82**

| Added Amount (µg/ml) | Amount of HGF Produced (%) Sample (1) |
|---|---|
| 0 | 100 |
| 1 | 209 |
| 10 | 333 |
| 100 | 377 |
| (The amount of HGF produced of control was 9.17 ng/ml.) | |

### Example 23

(1) Five-hundred grams of dried *Kjellmaniella crassifolia* was cut into thin pieces, and washed with 10 L of 80% ethanol. Thereafter, the resulting product was stirred at 25°C for 3 days in 50 L of 10% ethanol containing 1 mM potassium chloride, and filtered with a stainless screen having a screen diameter of 32 µm, to give about 45 L of a filtrate. Thirty-four liters of this filtrate was heated at 80°C for 3 hours, and thereafter cooled to 50°C. The resulting solution was concentrated with an ultrafilter OMEGA Cassette (manufactured by Filtron) having an excluding molecular weight of 10000 with keeping the liquid temperature at 50°C. Further, the concentrate was desalted with 5 L of distilled water heated to 50°C, and the flow path was washed twice by adding 200 mL of the same distilled water, and washings were collected, to give 1.5 L of a concentrate. This concentrate was lyophilized, to give 8.2 g of F-rich fucoidan.
(2) The induction activity for HGF production for F-rich fucoidan (Sample (1)) prepared in item (1) of Reference Example 23 was studied in the same manner as that in item (1) of Example 1. The results are shown in Table 83. As a result, F-rich exhibited induction activity for HGF production.

**Table 83**

| Added Amount (µg/ml) | Amount of HGF Produced (%) Sample (1) |
|---|---|
| 0 | 100 |
| 1 | 117 |
| 10 | 186 |
| 100 | 244 |
| (The amount of HGF produced of control was 9.60 ng/ml.) | |

### Example 24

NHDF cells (normal human dermal fibroblasts) cultured in a DMEM medium containing 10% fetal bovine serum were suspended in the DMEM medium containing 10% fetal bovine serum so as to have a concentration of 1 × 10⁵ cells/ml, and added into each well of a 48-well cell culture plate in a volume of 500 µl each. The cells were cultured for 24 hours. Thereafter, the medium was exchanged for a DMEM medium containing 1% fetal bovine serum, and thereto were added 10 µg/ml or 100 µg/ml minoxidil (manufactured by Wako Pure Chemicals Industries, Ltd.) and each sample. Here, in the case where F-rich Fucoidan was added, a test was also conducted for 1 µg/ml minoxidil added. Also, the same steps were carried out for the group added with only the sample without adding minoxidil. The medium was collected, and the amount of HGF in the medium was determined by using Quantikine Human Hepatocyte Growth Factor (HGF) ELISA Kit. As to the samples, fucoidan Bf prepared in Example 22, F-rich fucoidan prepared in Example 23, and 7-12SFd-F prepared in item (3) of Reference Example 2 was added so as to each have a final concentration of 1, 10 or 100 µg/ml. As to fucoidan Bf, a test was also conducted for the case where the final concentration of fucoidan Bf was 0.1 µg/ml. As a negative control, distilled water was added in the same volume as that of the sample. Each experiment was carried out three times, and an average value was taken. The results are shown in Tables 84 to 86.

In the minoxidil non-added group, the amount of HGF in the medium increased in a concentration-dependent manner with fucoidan Bf, F-rich fucoidan, or 7-12SFd-F, significantly increasing as compared to the non-added control. It was clarified from the above that both the freeing of HGF and the synthesis of HGF on the cell surface are promoted even in a case where the amount of HGF mRNA is small as the case of no minoxidil treatment. Further, in a case where minoxidil was added, the amount of HGF in the medium increased in a concentration-dependent manner with fucoidan Bf, F-rich fucoidan, or 7-12SFd-F, significantly increasing as compared with the non-added control. In addition, the increase in the amount of HGF when mRNA was increased as described above was very remarkable, as compared to the case where the amount of mRNA was small with no minoxidil treatment. It was clarified from the above that each of fucoidan Bf, F-rich fucoidan, and 7-12SFd-F promotes the freeing and the production of HGF to some extent even in a case where the amount of mRNA is small, and remarkably promotes the freeing and the production of HGF in a case where a large amount of mRNA exists due to the necessity of a large amount of HGF.

**Table 84**

| Concentration of HGF in NHDF / Fucoidan Bf Medium (pg/ml) | | | |
|---|---|---|---|
| Fucoidan Bf (µg/ml) | Minoxidil Non-Added | Minoxidil 10 µg/ml | Minoxidil 100 µg/ml |
| 0 | N.D. | 126.04 | 148.83 |
| 0.1 | 300.20 | 307.00 | 478.90 |
| 1 | 428.83 | 448.17 | 624.60 |
| 10 | 791.93 | 799.90 | 794.20 |
| 100 | 729.33 | 709.97 | 860.23 |
| N.D. means the detection limit or less. | | | |

**Table 85**

| Amount of HGF in NHDF / F-rich Fucoidan Medium (pg/ml) | | | | |
|---|---|---|---|---|
| F-rich Fucoidan (µg/ml) | Minoxidil Non-Added | Minoxidil 1 µg/ml | Minoxidil 10 µg/ml | Minoxidil 100 µg/ml |
| 0 | N.D. | N.D. | N.D. | N.D. |
| 1 | 132.75 | N.T. | N.T. | 203.03 |
| 10 | 316.90 | 239.90 | 310.20 | 434.10 |
| 100 | 369.33 | 372.70 | 372.70 | 537.87 |
| N.D. means the detection limit or less. Also, N.T. means "not tested." | | | | |

**Table 86**

| Concentration of HGF in NHDF / 7-12SFd-F Medium (pg/ml) | | | |
|---|---|---|---|
| 7-12SFd-F (µg/ml) | Minoxidil Non-Added | Minoxidil 10 µg/ml | Minoxidil 100 µg/ml |
| 0 | N.D. | N.D. | 137.20 |
| 1 | 228.73 | 164.00 | 378.30 |
| 10 | 345.93 | 376.07 | 450.83 |
| 100 | 439.67 | 483.20 | 820.10 |
| N.D. means the detection limit or less. | | | |

### Example 25

Mice (CDF1 female, 7-weeks old, body weight: about 20 kg) were simultaneously intraperitoneally administered with galactosamine (20 mg/mouse) and LPS (lipopolysaccharide: 0.03 µg/mouse) to prepare a lethal model by fulminant hepatitis, and the macrobiotic effect by the fucoidan described in item (1) of Reference Example 1 was studied. The fucoidan was adjusted to have a concentration of 10% with distilled water, and forcibly orally administered twice, one hour before and one hour after the simultaneous administration of galactosamine and LPS, at a dosage of 10 ml/kg body weight (1 g/kg as the fucoidan). The control group was similarly administered with distilled water.

The viability after 72 hours from the beginning of the experiment was one case out of 8 cases for the control group, and 7 cases out of 8 cases for the fucoidan-administered group, so that a remarkable macrobiotic effect could be found by the fucoidan administration. Further, an improved effect was also found in the serum biochemical value in the viable cases. The results are shown in Table 87.

**Table 87**

| Group | GPT (U/1) | GOT (U/l) | Total Bilirubin (mg/dl) |
|---|---|---|---|
| Control | 385 | 613 | 1.0 |
| Fucoidan Administered Group | 94±22 | 233±53 | 0.3±0.02 |

### Example 26

(1) Five-hundred grams of *Kjellmaniella crassifolia* was cut into thin pieces, and washed with 10 L of 80% ethanol. Thereafter, the resulting product was stirred in a container having an inner diameter of 40 cm containing 50 liters of 10% ethanol containing 1 mM potassium chloride at 25°C for 2 days, at a speed of 120 rotations per minute to extract fucoidan. The resulting extract was filtered with a stainless screen having a screen diameter of 32 µm, to give a fucoidan solution.

To 46 liters of the fucoidan solution was added one liter of a palm oil solution; prepared by dissolving 1 g of palm oil (manufactured by Kao Corporation, for cosmetic use) in 1 liter of ethanol with stirring, and one liter of glycerol was further added thereto, to give a lotion.

(2) To the fucoidan solution prepared in item (1) of Example 26, gelatin and perfume were each added so as to have a final concentration of 0.02%, to give a gelatin-used lotion. Also, collagen was similarly added, to give a collagen-used lotion. Deposited Biological Materials
(1) Name and Addressee of Depository Authority
   the Ministry of International Trade and Industry, National Institute of Bioscience and Human-Technology, Agency of Industrial Science and Technology
   1-3, Higashi 1 chome, Tsukuba-shi, Ibaraki-ken, Japan (Zip code 305)
(2) Deposited Microorganisms
   (i) *Alteromonas* sp. SN-1009
      Original Date of Deposit: February 13, 1996
      Date of Request for Transfer to International Deposit:
         November 15, 1996
      Accession Number: FERM BP-5747
   (ii) *Flavobacterium* sp. SA-0082
      Original Date of Deposit: March 29, 1995
      Date of Request for Transfer to International Deposit:
         February 15, 1996
      Accession Number: FERM BP-5402

### INDUSTRIAL APPLICABILITY

According to the present invention, a pharmaceutical effective for a disease requiring growth factor production, comprising a substance exhibiting induction activity for growth factor production as an effective ingredient is provided. The pharmaceutical possesses induction activity for HGF production, induction activity for h-IGF production, induction activity for NGF and neurotrophic factor production, and the like, and is useful as a therapeutic agent or prophylactic agent for a disease requiring the production of these growth factors, such as hepatitis, diabetes, cancers and neurotic diseases.

Further, foods and beverages can be produced by using a compound having induction action for growth factor production, selected from acidic polysaccharides, sulfated polysaccharides such as fucoidan, sodium dextran sulfate and high-chondroitin sulfate content shark cartilage extracts, degradation products thereof, acidic oligosaccharides, acidic monosaccharides, and salts thereof, so that the symptom of a disease requiring growth factor production can be ameliorated by its daily intake as foods and beverages. In addition, a feed having similar physiological functions is provided.

Therefore, the functional foods and beverages or the functional feed each comprising a compound having induction action for growth factor production, selected from acidic polysaccharides, sulfated fucose-containing polysaccharides such as fucoidan, degradation products thereof, acidic oligosaccharides, acidic monosaccharides, and salts thereof as an effective ingredient is functional foods and beverages or feed which is useful for maintaining the homeostatis of a living body, owing to its induction action for growth factor production.

According to the present invention, a bio-cosmetic for induction of HGF production are also provided, and such a bio-cosmetic is very useful for skin health management or the like. Further, a suppressor for metastasis of a cancer is provided.

In addition, an inducer for growth factor production is provided, and such an inducer is useful for functional studies of growth factors, and screening of pharmaceuticals for a disease associated with growth factor.

## Claims

1. A therapeutic agent or prophylactic agent for a disease requiring induction of growth factor production, **characterized in that** the therapeutic agent or prophylactic agent comprises as an effective ingredient a compound selected from the group consisting of acidic polysaccharides, degradation products thereof, acidic oligosaccharides, acidic monosaccharides, acidic sugar alcohols and salts thereof, excluding heparin and heparan sulfate, wherein the compound has induction action for growth factor production.

2. The therapeutic agent or prophylactic agent according to claim 1, wherein the acidic polysaccharide is a sulfated polysaccharide.

3. The therapeutic agent or prophylactic agent according to claim 2, wherein the sulfated polysaccharide is a sulfated polysaccharide derived from an algae, a sulfated polysaccharide derived from an animal, a sulfated polysaccharide derived from a plant, a sulfated polysaccharide derived from a microorganism, or a sulfated polysaccharide derived from fish.

4. The therapeutic agent or prophylactic agent according to claim 2, wherein the sulfated polysaccharide is a synthetic sulfated polysaccharide.

5. The therapeutic agent or prophylactic agent according to claim 2, wherein the sulfated polysaccharide is a fucoidan.

6. The therapeutic agent or prophylactic agent according to claim 1, wherein the acidic monosaccharide is a sulfated glucose, a sulfated galactose, a sulfated xylose, a sulfated 2-deoxy-glucose, a sulfated talose or a sulfated mannose.

7. The therapeutic agent or prophylactic agent according to claim 1, wherein the acidic oligosaccharide is a sulfated oligosaccharide selected from the group consisting of sulfated maltoses, sulfated lactoses, sulfated sucroses, sulfated trehaloses, sulfated lactuloses, sulfated melibioses, sulfated cellobioses, sulfated isomaltoses, sulfated turanoses, sulfated palatinoses, sulfated maltotrioses, sulfated maltohexaoses, sulfated maltoheptaoses, sulfated dodecyl-maltohexaoses, a compound represented by the formula (I): 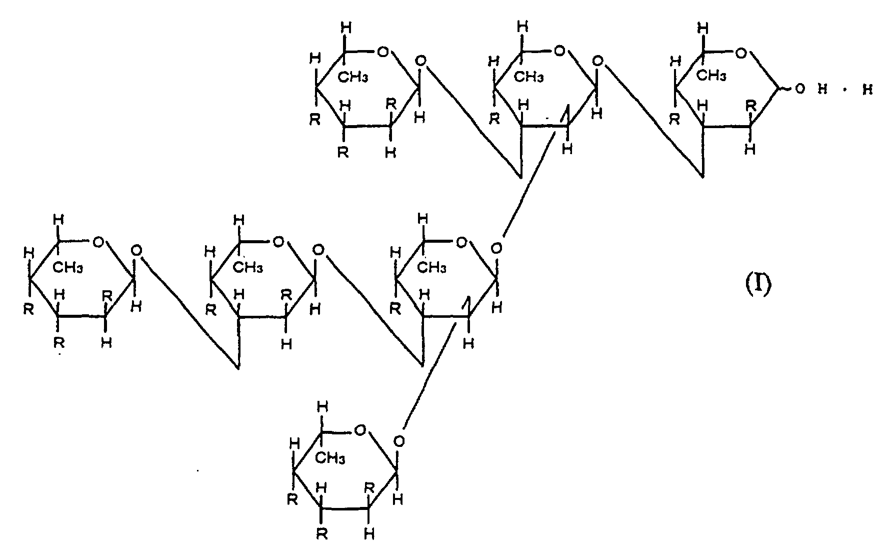 wherein R is OH or OSO₃H, and
a compound represented by the formula (II): 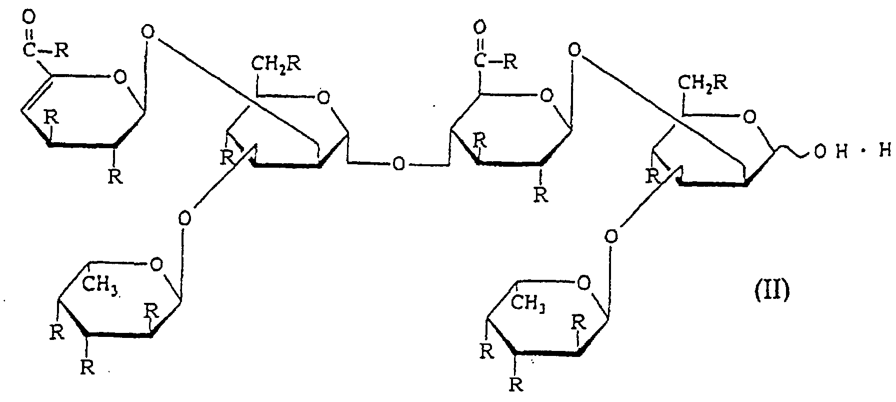 wherein R is OH or OSO₃H.

8. The therapeutic agent or prophylactic agent according to any one of claims 1 to 7, wherein the growth factor is a hepatocyte growth factor, an insulin-like growth factor or a nerve growth factor.

9. The therapeutic agent or prophylactic agent according to any one of claims 1 to 8, **characterized in that** the therapeutic agent or prophylactic agent further comprises a substance capable of synergistically increasing induction action for growth factor production of an acidic polysaccharide, a degradation product thereof, an acidic oligosaccharide, an acidic monosaccharide, an acidic sugar alcohol or a salt thereof.

10. The therapeutic agent or prophylactic agent according to claim 9, wherein the substance capable of synergistically increasing induction action for growth factor production of an acidic polysaccharide, a degradation product thereof, an acidic oligosaccharide, an acidic monosaccharide, an acidic sugar alcohol or a salt thereof is a substance selected from the group consisting of cytokines, prostaglandins, and compounds having a cyclopentene ring.

11. A food, beverage or feed for induction of growth factor production comprising a compound selected from the group consisting of acidic polysaccharides, degradation products thereof, acidic oligosaccharides, acidic monosaccharides, acidic sugar alcohols and salts thereof, wherein the compound has induction action for growth factor production.

12. The food, beverage or feed according to claim 11, wherein the acidic polysaccharide is a sulfated polysaccharide.

13. The food, beverage or feed according to claim 12, wherein the sulfated polysaccharide is a sulfated polysaccharide derived from an algae, a sulfated polysaccharide derived from an animal, a sulfated polysaccharide derived from a plant, a sulfated polysaccharide derived from a microorganism, or a sulfated polysaccharide derived from fish.

14. The food, beverage or feed according to claim 12, wherein the sulfated polysaccharide is a synthetic sulfated polysaccharide.

15. The food, beverage or feed according to claim 12, wherein the sulfated polysaccharide is a fucoidan.

16. The food, beverage or feed according to claim 11, wherein the acidic monosaccharide is a sulfated glucose, a sulfated galactose, a sulfated xylose, a sulfated 2-deoxy-glucose, a sulfated talose or a sulfated mannose.

17. The food, beverage or feed according to claim 11, wherein the acidic oligosaccharide is a sulfated oligosaccharide selected from the group consisting of sulfated maltoses, sulfated lactoses, sulfated sucroses, sulfated trehaloses, sulfated lactuloses, sulfated melibioses, sulfated cellobioses, sulfated isomaltoses, sulfated turanoses, sulfated palatinoses, sulfated maltotrioses, sulfated maltohexaoses, sulfated maltoheptaoses, sulfated dodecyl-maltohexaoses, a compound represented by the formula (I): 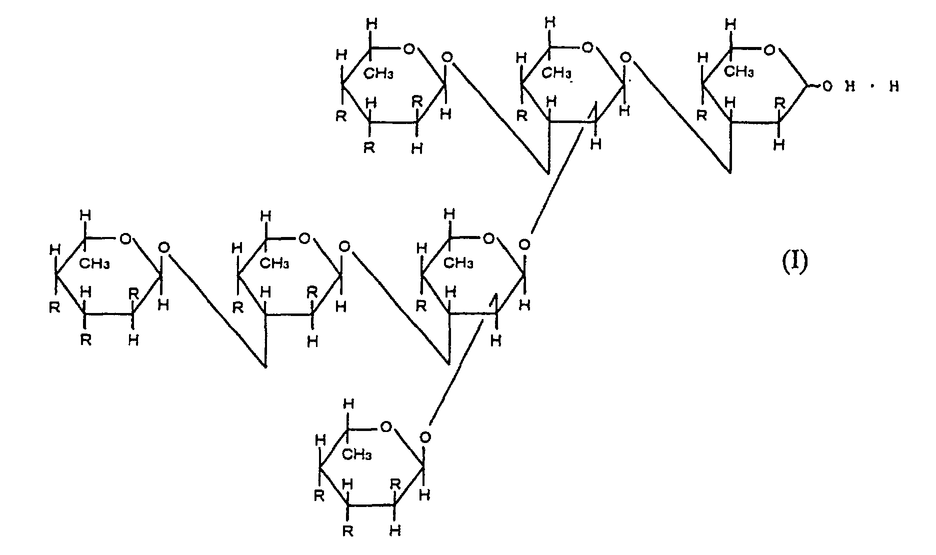 wherein R is OH or OSO₃H, and
a compound represented by the formula (II): 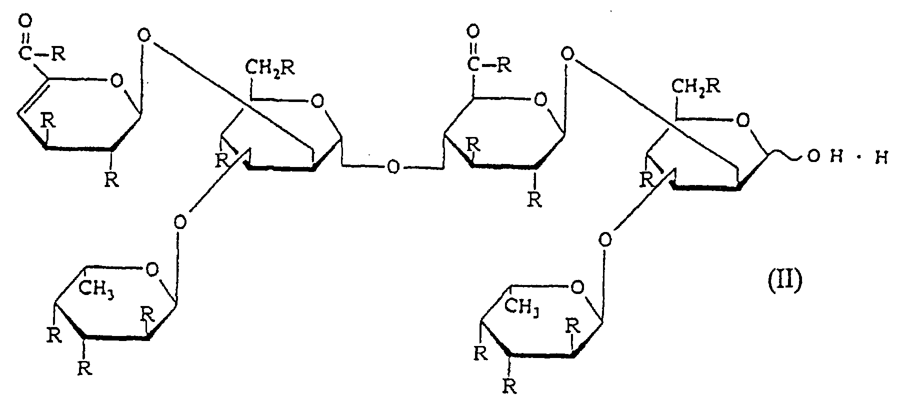 wherein R is OH or OSO₃H.

18. The food, beverage or feed according to any one of claims 11 to 17, wherein the food, beverage or feed is used for induction of a hepatocyte growth factor production, induction of an insulin-like growth factor production or induction of a nerve growth factor production.

19. The food, beverage or feed according to any one of claims 11 to 18, **characterized in that** the food, beverage or feed further comprises a substance capable of synergistically increasing induction action for growth factor production of an acidic polysaccharide, a degradation product thereof, an acidic oligosaccharide, an acidic monosaccharide, an acidic sugar alcohol or a salt thereof.

20. The food, beverage or feed according to claim 19, wherein the substance capable of synergistically increasing induction action for growth factor production of an acidic polysaccharide, a degradation product thereof, an acidic oligosaccharide, an acidic monosaccharide, an acidic sugar alcohol or a salt thereof is a substance selected from the group consisting of cytokines, prostaglandins, and compounds having a cyclopentene ring.

21. A cosmetic for induction of growth factor production comprising a compound selected from the group consisting of acidic polysaccharides, degradation products thereof, acidic oligosaccharides, acidic monosaccharides, acidic sugar alcohols and salts thereof, wherein the compound has induction action for growth factor production.

22. The cosmetic according to claim 21, wherein the acidic polysaccharide is a sulfated polysaccharide.

23. The cosmetic according to claim 22, wherein the sulfated polysaccharide is a sulfated polysaccharide derived from an algae, a sulfated polysaccharide derived from an animal, a sulfated polysaccharide derived from a plant, a sulfated polysaccharide derived from a microorganism, or a sulfated polysaccharide derived from fish.

24. The cosmetic according to claim 22, wherein the sulfated polysaccharide is a synthetic sulfated polysaccharide.

25. The cosmetic according to claim 22, wherein the sulfated polysaccharide is a fucoidan.

26. The cosmetic according to claim 21, wherein the acidic monosaccharide is a sulfated glucose, a sulfated galactose, a sulfated xylose, a sulfated 2-deoxyglucose, a sulfated talose or a sulfated mannose.

27. The cosmetic according to claim 21, wherein the acidic oligosaccharide is a sulfated oligosaccharide selected from the group consisting of sulfated maltoses, sulfated lactoses, sulfated sucroses, sulfated trehaloses, sulfated lactuloses, sulfated melibioses, sulfated cellobioses, sulfated isomaltoses, sulfated turanoses, sulfated palatinoses, sulfated maltotrioses, sulfated maltohexaoses, sulfated maltoheptaoses, sulfated dodecyl-maltohexaoses, a compound represented by the formula (I): 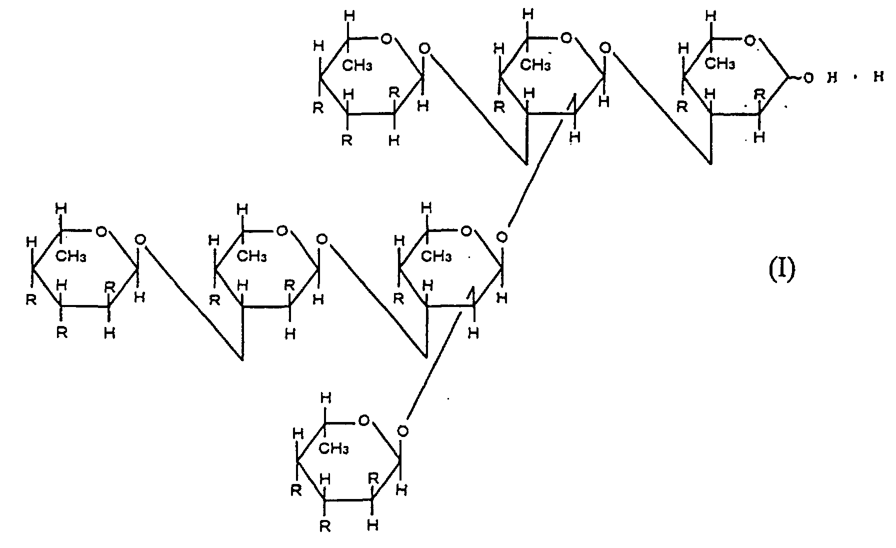 wherein R is OH or OSO₃H, and
a compound represented by the formula (II): 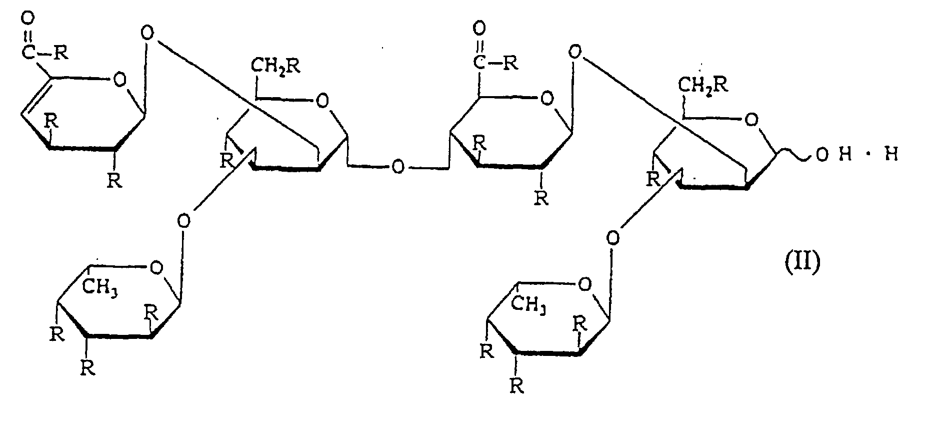 wherein R is OH or OSO₃H.

28. The cosmetic according to any one of claims 21 to 27, wherein the cosmetic is used for induction of a hepatocyte growth factor production, induction of an insulin-like growth factor production or induction of a nerve growth factor production.

29. The cosmetic according to any one of claims 21 to 28, **characterized in that** the cosmetic further comprises a substance capable of synergistically increasing induction action for growth factor production of an acidic polysaccharide, a degradation product thereof, an acidic oligosaccharide, an acidic monosaccharide, an acidic sugar alcohol or a salt thereof.

30. The cosmetic according to claim 29, wherein the substance capable of synergistically increasing induction action for growth factor production of an acidic polysaccharide, a degradation product thereof, an acidic oligosaccharide, an acidic monosaccharide, an acidic sugar alcohol or a salt thereof is a substance selected from the group consisting of cytokines, prostaglandins, and compounds having a cyclopentene ring.

31. The cosmetic according to any one of claims 21 to 30, wherein the cosmetic is used as a lotion, a milky lotion, cream, a facial pack, a bathing agent, a facial cleansing agent, a bathing soap, or a bathing detergent.

32. A controlling agent for growth factor production, **characterized in that** the controlling agent comprises as an effective ingredient a compound selected from the group consisting of acidic polysaccharides, degradation products thereof, acidic oligosaccharides, acidic monosaccharides, acidic sugar alcohols and salts thereof.
